# EUROPEAN PATENT SPECIFICATION

(11) **EP 2 822 588 B1**
(45) Date of publication and mention of the grant of the patent: **11.09.2019**
(21) Application number: 13708156.8
(22) Date of filing: 08.03.2013
(51) Int. Cl.: A61K 39/395, A61K 45/06, C07K 16/28, A61P 35/00

(54) **COMBINATION THERAPY OF ANTIBODIES AGAINST HUMAN CSF-1R AND USES THEREOF**
KOMBINATIONSTHERAPIE VON ANTIKÖRPER GEGEN MENSCHLICHEN CSF-1R UND DEREN VERWENDUNGEN
POLYTHÉRAPIE À BASE D'ANTICORPS DIRIGÉS CONTRE LE CSF-1R HUMAIN ET UTILISATIONS CORRESPONDANTES

(30) Priority: 08.03.2012 EP 12158519
(43) Date of publication of application: 14.01.2015
(73) Proprietor: F. Hoffmann-La Roche AG, 4070 Basel (CH)
(72) Inventor: CANNARILE, Michael, 80337 München (DE); RIES, Carola, 82377 Penzberg (DE); RUETTINGER, Dominik, 82418 Seehausen (DE); WARTHA, Katharina, 4056 Basel (CH)
(74) Representative: Burger, Alexander
(86) International application number: PCT/EP2013/054676
(87) International publication number: WO 2013/132044

(56) References cited:
- EP-A1- 2 423 228
- WO-A1-2009/026303
- WO-A1-2011/070024
- WO-A1-2011/107553
- WO-A1-2011/117329
- WO-A1-2011/123381
- WO-A1-2011/131407
- WO-A2-2007/075326
- T. S. LEWIS ET AL: "Distinct Apoptotic Signaling Characteristics of the Anti-CD40 Monoclonal Antibody Dacetuzumab and Rituximab Produce Enhanced Antitumor Activity in Non-Hodgkin Lymphoma", CLINICAL CANCER RESEARCH, vol. 17, no. 14, 15 July 2011 (2011-07-15) , pages 4672-4681, XP055059121, ISSN: 1078-0432, DOI: 10.1158/1078-0432.CCR-11-0479

## Description

The present invention relates inter alia to the combination of antibodies against human CSF-1R binding to human CSF-1R, characterized in binding to the (dimerization) domains D4 to D5 with a chemotherapeutic agent, radiation, and/or cancer immunotherapy.

### Background of the Invention

The human CSF-1 receptor (CSF-1R; colony stimulating factor 1 receptor; synonyms: M-CSF receptor; Macrophage colony-stimulating factor 1 receptor, Fms proto-oncogene, c-fms, SEQ ID NO: 62) is known since 1986 (Coussens, L., et al., Nature 320 (1986) 277-280). CSF-1R is a growth factor and encoded by the c-fms proto-oncogene (reviewed e.g. in Roth, P., and Stanley, E.R., Curr. Top. Microbiol. Immunol. 181 (1992) 141-167).

CSF-1R is the receptor for CSF-1 (colony stimulating factor 1, also called M-CSF, macrophage colony-stimulating factor) and mediates the biological effects of this cytokine (Sherr, C.J., et al., Cell 41 (1985) 665-676). The cloning of the colony stimulating factor-1 receptor (CSF-1R) (also called c-fms) was described for the first time in Roussel, M.F., et al., Nature 325 (1987) 549-552. In that publication, it was shown that CSF-1R had transforming potential dependent on changes in the C-terminal tail of the protein including the loss of the inhibitory tyrosine 969 phosphorylation which binds Cbl and thereby regulates receptor down regulation (Lee, P.S., et al., Embo J. 18 (1999) 3616-3628). Recently a second ligand for CSF-1R termed interleukin-34 (IL-34) was identified (Lin, H., et al, Science 320 (2008) 807-811).

Currently two CSF-1R ligands that bind to the extracellular domain of CSF-1R are known. The first one is CSF-1 (colony stimulating factor 1, also called M-CSF, macrophage; SEQ ID NO: 86) and is found extracellularly as a disulfide-linked homodimer (Stanley, E.R. et al., Journal of Cellular Biochemistry 21 (1983) 151-159; Stanley, E.R. et al., Stem Cells 12 Suppl. 1 (1995) 15-24). The second one is IL-34 (Human IL-34; SEQ ID NO: 87) (Hume, D. A. , et al, Blood 119 (2012) 1810-1820). The main biological effects of CSF-1R signaling are the differentiation, proliferation, migration, and survival of hematopoietic precursor cells to the macrophage lineage (including osteoclast). Activation of CSF-1R is mediated by its CSF-1R ligands, CSF-1 (M-CSF) and IL-34. Binding of CSF-1 (M-CSF) to CSF-1R induces the formation of homodimers and activation of the kinase by tyrosine phosphorylation (Li, W. et al, EMBO Journal.10 (1991) 277-288; Stanley, E.R., et al., Mol. Reprod. Dev. 46 (1997) 4-10).

The biologically active homodimer CSF-1 binds to the CSF-1R within the subdomains D1 to D3 of the extracellular domain of the CSF-1 receptor (CSF-1R-ECD). The CSF-1R-ECD comprises five immunoglobulin-like subdomains (designated D1 to D5). The subdomains D4 to D5 of the extracellular domain (CSF-1R-ECD) are not involved in the CSF-1 binding (Wang, Z., et al Molecular and Cellular Biology 13 (1993) 5348-5359). The subdomain D4 is involved in dimerization (Yeung, Y-G., et al Molecular & Cellular Proteomics 2 (2003) 1143-1155; Pixley, F. J., et al., Trends Cell Biol 14 (2004) 628-638).

Further signaling is mediated by the p85 subunit of PI3K and Grb2 connecting to the PI3K/AKT and Ras/MAPK pathways, respectively. These two important signaling pathways can regulate proliferation, survival and apoptosis. Other signaling molecules that bind the phosphorylated intracellular domain of CSF-1R include STAT1, STAT3, PLCy, and Cbl (Bourette, R.P. and Rohrschneider, L.R., Growth Factors 17 (2000) 155-166).

CSF-1R signaling has a physiological role in immune responses, in bone remodeling and in the reproductive system. The knockout animals for either CSF-1 (Pollard, J.W., Mol. Reprod. Dev. 46 (1997) 54-61) or CSF-1R (Dai, X.M., et al., Blood 99 (2002) 111-120) have been shown to have osteopetrotic, hematopoietic, tissue macrophage, and reproductive phenotypes consistent with a role for CSF-1R in the respective cell types.

Sherr, C.J., et al., Blood 73 (1989) 1786-1793 relates to some antibodies against CSF-1R that inhibit the CSF-1 activity. Ashmun, R.A., et al., Blood 73 (1989) 827-837 relates to CSF-1R antibodies. Lenda, D., et al., Journal of Immunology 170 (2003) 3254-3262 relates to reduced macrophage recruitment, proliferation, and activation in CSF-1-deficient mice results in decreased tubular apoptosis during renal inflammation. Kitaura, H., et al., Journal of Dental Research 87 (2008) 396-400 refers to an anti-CSF-1 antibody which inhibits orthodontic tooth movement. WO 2001/030381 mentions CSF-1 activity inhibitors including antisense nucleotides and antibodies while disclosing only CSF-1 antisense nucleotides. WO 2004/045532 relates to metastases and bone loss prevention and treatment of metastatic cancer by a CSF-1 antagonist disclosing as antagonist anti-CSF-1-antibodies only. WO 2005/046657 relates to the treatment of inflammatory bowel disease by anti-CSF-1-antibodies. US 2002/0141994 relates to inhibitors of colony stimulating factors. WO 2006/096489 relates to the treatment of rheumatoid arthritis by anti-CSF-1-antibodies. WO 2009/026303 and WO 2009/112245 relate to certain anti-CSF-1R antibodies binding to CSF-1R within the first three subdomains (D1 to D3) of the Extracellular Domain (CSF-1R-ECD) and combination therapies of these antibodies. WO2011/123381(A1) relates to antibodies against CSF-1R and combination therapies of these antibodies..

### Summary of the Invention

The invention is defined in the appended claims and any other aspects set forth herein not falling within the scope of the claims are for information only.The invention comprises an antibody binding to human CSF-1R for use in the treatment of cancer wherein the anti-CSF-1R antibody is administered in combination with an agonistic CD40 antibody.

In one embodiment the antibody binding to human CSF-1R is further caharcterized in i) comprising (a) a heavy chain variable domain amino acid sequence of SEQ ID NO:39 and (b) a light chain variable domain amino acid sequence of SEQ ID NO:40; or ii) comprising (a) a heavy chain variable domain amino acid sequence of SEQ ID NO: 88 and (b) a light chain variable domain amino acid sequence of SEQ ID NO: 89. In one embodiment of the invention the anti-CSF-1R antibody is characterized in that the antibody binds to human CSF-1R Extracellular Domain (SEQ ID NO: 64) (comprising domains D1 to D5) and does not bind to domains D1 to D3 (SEQ ID NO: 66) of the extracellular domain of human CSF-1R.

One preferred embodiment of the invention is the combination therapy of an antibody binding to human CSF-1R (including antibodies binding to domains D1-D3 and antibodies binding to domains D4-D5, preferably antibodies binding to domains D4-D5 as described herein) with a cancer immunotherapy, wherein the cancer immunotherapy is an agonistic CD40 antibody. CSF-1R antibodies binding to domains D1-D3 of human CSF-1R are described e.g. in WO 2009/026303 and WO 2009/112245 relate to certain anti-CSF-1R antibodies binding to CSF-1R within the first three subdomains (D1 to D3) of the Extracellular Domain (CSF-1R-ECD). WO2011/123381(A1) relates to antibodies against CSF-1R. and Sherr, C.J., et al., Blood 73 (1989) 1786-1793 (typically these antibodies are characterized by inhibiting CSF-1R ligand-dependent but not CSF-1R ligand-independent CSF-1R proliferation and /or signaling).

CSF-1R antibodies binding to domains D4-D5 of human CSF-1R are described e.g. within the present invention, in PCT/EP2012/075241 and Sherr, C.J., et al., Blood 73 (1989) 1786-1793 (typically these antibodies are characterized by inhibiting CSF-1R ligand-dependent and CSF-1R ligand-independent CSF-1R proliferation and/or signaling).

Thus in one aspect of the invention also comprises an antibody binding to human CSF-1R, for use in the treatment of cancer wherein the anti-CSF-1R antibody is administered in combination with cancer immunotherapywherein the cancer immunotherapy is an agonistic CD40 antibody (as described e.g. Beatty et al., Science 331 (2011) 1612-1616, R. H. Vonderheide et al., J Clin Oncol 25, 876 (2007); Khalil, M, et al., Update Cancer Ther. 2007 June 1; 2(2): 61-65, examples in clinical trials are e.g CP-870,893 and dacetuzumab (an agonist CD40 antibody, CAS number 880486-59-9, SGN-40; humanized S2C6 antibody) (Khalil, M, et al, Update Cancer Ther. 2007 June 1; 2(2): 61-65; an agonist CD40 rat anti-mouse IgG2a mAb FGK45 as model antibody is described in S. P. Schoenberger, et al, Nature 393, 480 (1998)). CP-870,893 is a fully human IgG2 CD40 agonist antibody developed by Pfizer. It binds CD40 with a KD of 3.48×10-10 M, but does not block binding of CD40L (see e.g., U.S.7,338,660 or EP1476185 wherein CP-870,893 is described as antibody 21.4.1). CP-870,893 (antibody 21.4.1 of U.S.7,338,660) is characterized by comprising (a) a heavy chain variable domain amino acid sequence of QVQLVQSGAEVKKPGASVKVSCKAS GYTFTGYYMHWVRQAPGQGLEWMGWINPDSGGTNYAQKFQGRVTMTR DTSISTAYMELNRLRSDDTAVYYCARDQPLGYCTNGVCSYFDYWGQGTL VTVSS (SEQ ID NO: 88) (which corresponds to SEQ ID NO: 42 of US 7,338,660) (b) a light chain variable domain amino acid sequence of DIQMTQSPSSVSASVGDRVTITCRASQGIYSWLAWYQQKPGKAPNLLIYTA STLQSGVPSRFSGSGSGTDFTLTISSLQPEDFATYYCQQANIFPLTFGGGTKV EIK (SEQ ID NO: 89) (which corresponds to SEQ ID NO: 44 of US 7,338,660); and /or having the heavy chain variable domain and light chain variable domain amino acid sequences of the antibody produced by hybridoma 21.4.1 having American Type Culture Collection (ATCC) accession number PTA-3605. Dacetuzumab and other humanized S2C6 antibodies are described in US6946129 and US8303955. Humanized S2C6 antibodies are e.g. based on the CDR1, 2 and 3 of the heavy and light chain variable domain of murine mAB S2C6 (deposited with the ATCC as PTA-110). The CDR1, 2 and 3 of the heavy and light chain variable domain of murine mAB S2C6 is described and disclosed US6946129. In one embodiment the agonist CD40 antibody is dacetuzumab. In one embodiment the agonist CD40 antibody is characterized by comprising (a) a heavy chain variable domain amino acid sequence of EVQLVESGGGLVQPGGSLRLSCAASGYSFTGYYIHWVRQAPGKGLEWVA RVIPNAGGTSYNQKFKGRFTLSVDNSKNTAYLQMNSLRAEDTAVYYCARE GIYWWGQGTLVTVS (SEQ ID NO: 90) (b) a light chain variable domain amino acid sequence of DIQMTQSPSSLSASVGDRVTITCRSSQSLVHSNGNTFLHW YQQKPGKAPKLLIYTVSNRFSGVPSRFSGSGSGTDFTLTISSLQPEDFAT YFCSQTTHVPWTFGQGTKVEIKR (SEQ ID NO: 91)).

In one embodiment of the invention the anti-CSF-1R antibody is characterized in that the antibody binds to human CSF-1R fragment delD4 (SEQ ID NO: 65) and to human CSF-1R Extracellular Domain (SEQ ID NO: 64) with a ratio of 1:50 or lower.

In one embodiment of the invention the antibody is characterized in that the antibody does not bind to human CSF-1R fragment delD4 (SEQ ID NO: 65).

In one embodiment of the invention the antibody is characterized in that
a) the heavy chain variable domain is SEQ ID NO:7 and the light chain variable domain is SEQ ID NO:8,
b) the heavy chain variable domain is SEQ ID NO:15 and the light chain variable domain is SEQ ID NO:16;
c) the heavy chain variable domain is SEQ ID NO:75 and the light chain variable domain is SEQ ID NO:76;
d) the heavy chain variable domain is SEQ ID NO:83 and the light chain variable domain is SEQ ID NO:84;
or a humanized version thereof.

In one embodiment of the invention the antibody is characterized in that
a) the heavy chain variable domain is SEQ ID NO:7 and the light chain variable domain is SEQ ID NO:8,
b) the heavy chain variable domain is SEQ ID NO:15 and the light chain variable domain is SEQ ID NO:16;
or a humanized version thereof.

In one embodiment of the invention the antibody is characterized in that
a) the heavy chain variable domain is SEQ ID NO:23 and the light chain variable domain is SEQ ID NO:24, or
b) the heavy chain variable domain is SEQ ID NO:31 and the light chain variable domain is SEQ ID NO:32, or
c) the heavy chain variable domain is SEQ ID NO:39 and the light chain variable domain is SEQ ID NO:40, or
d) the heavy chain variable domain is SEQ ID NO:47 and the light chain variable domain is SEQ ID NO:48, or
e) the heavy chain variable domain is SEQ ID NO:55 and the light chain variable domain is SEQ ID NO:56.

In one embodiment of the invention the antibody is characterized in that
a) the heavy chain variable domain comprises a CDR3 region of SEQ ID NO: 1, a CDR2 region of SEQ ID NO: 2, and a CDR1 region of SEQ ID NO:3, and the light chain variable domain comprises a CDR3 region of SEQ ID NO: 4, a CDR2 region of SEQ ID NO:5, and a CDR1 region of SEQ ID NO:6, or
b) the heavy chain variable domain comprises a CDR3 region of SEQ ID NO: 9, a CDR2 region of SEQ ID NO: 10, and a CDR1 region of SEQ ID NO: 11, and the light chain variable domain comprises a CDR3 region of SEQ ID NO:12, a CDR2 region of SEQ ID NO: 13, and a CDR1 region of SEQ ID NO: 14, or
c) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
d) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
e) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
f) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46, or
g) the heavy chain variable domain comprises a CDR3 region of SEQ ID NO: 49, a CDR2 region of SEQ ID NO: 50, and a CDR1 region of SEQ ID NO: 51, and the light chain variable domain comprises a CDR3 region of SEQ ID NO:52, a CDR2 region of SEQ ID NO: 53, and a CDR1 region of SEQ ID NO: 54; or
h) the heavy chain variable domain comprises a CDR3 region of SEQ ID NO:69, a CDR2 region of SEQ ID NO: 70, and a CDR1 region of SEQ ID NO:71, and the light chain variable domain comprises a CDR3 region of SEQ ID NO: 72, a CDR2 region of SEQ ID NO:73, and a CDR1 region of SEQ ID NO:74, or
i) the heavy chain variable domain comprises a CDR3 region of SEQ ID NO: 77, a CDR2 region of SEQ ID NO: 78, and a CDR1 region of SEQ ID NO: 79, and the light chain variable domain comprises a CDR3 region of SEQ ID NO:80, a CDR2 region of SEQ ID NO: 81, and a CDR1 region of SEQ ID NO: 82.

In one embodiment of the invention the antibody is of human IgG1 subclass or of human IgG4 subclass.

The combination therapies of the antibodies described herein show benefits for patients in need of a CSF-1R targeting therapy. The antibodies according to the invention show efficient antiproliferative activity against ligand-independent and ligand-dependent proliferation and are therefore especially useful in the treatment of cancer and metastasis in combination with an CD40 agonistic antibody

Surprisingly it has been found that, using a human CSF-1R fragment delD4 in which the D4 subdomain of human CSF-1R-ECD was deleted (SEQ ID NO:65), the anti-CSF-1R antibodies could be selected. These antibodies show valuable properties like excellent ligand-dependent cell growth inhibition and at the same time ligand independent cell growth inhibition of NIH 3T3 cell, retrovirally infected with either an expression vector for full-length wildtype CSF-1R (SEQ ID NO:62) or mutant CSF-1R L301S Y969F (SEQ ID NO:63) whereby mutant CSF-1R recombinant cells are able to form spheroids independent of the CSF-1 ligand. Furthermore these antibodies inhibit (both) human and cynomolgous macrophage differentiation, as they inhibit survival of human and cynomolgous monocytes.

Further antibodies binding to the (dimerization) domains D4 to D5 can be selected by screening for antibodies that bind to the complete extracellular domain of human CSF-1R (SEQ ID NO: 64) (including domains D1 to D5), and not binding to the domains D1 to D3 (SEQ ID NO: 66) of the extracellular domain of human CSF-1R.

### Description of the Figures

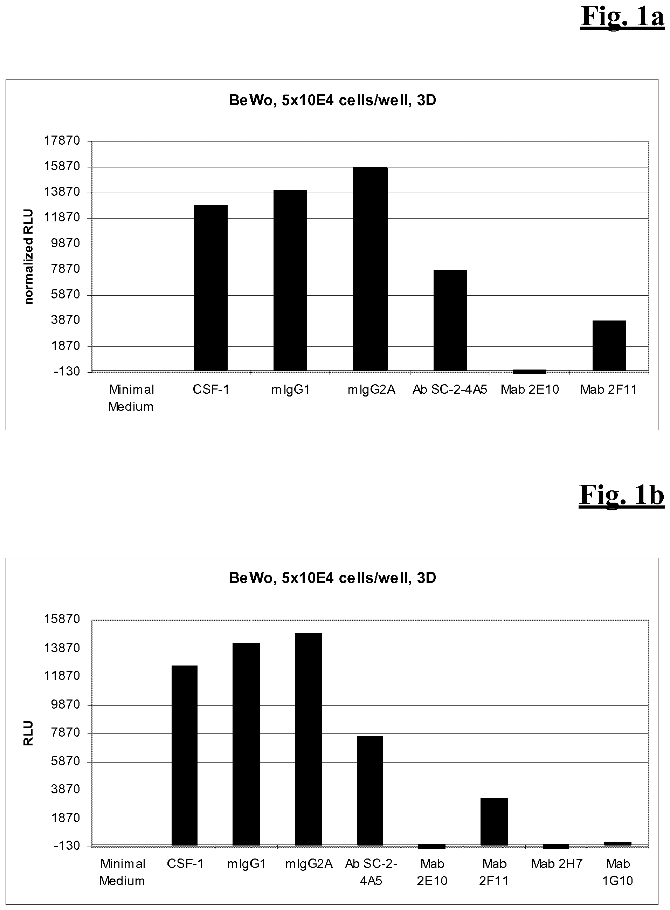

- **Figure 1**: Growth inhibition of BeWo tumor cells in 3D culture under treatment with different anti-CSF-1R monoclonal antibodies at a concentration of 10µg/ml.
X axis: viability normalized mean relative light units (RLU) corresponding to the ATP-content of the cells (CellTiterGlo assay).
Y axis: tested probes: Minimal Medium (0.5% FBS), mouse IgG1 (mIgG1, 10µg/ml), mouse IgG2a (mIgG2a 10µg/ml), CSF-1 only, Mab 2F11, Mab 2E10, Mab2H7, Mab1G10 and SC 2-4A5. Highest inhibition of CSF-1 induced growth was observed with the anti-CSF-1R antibodies according to the invention.

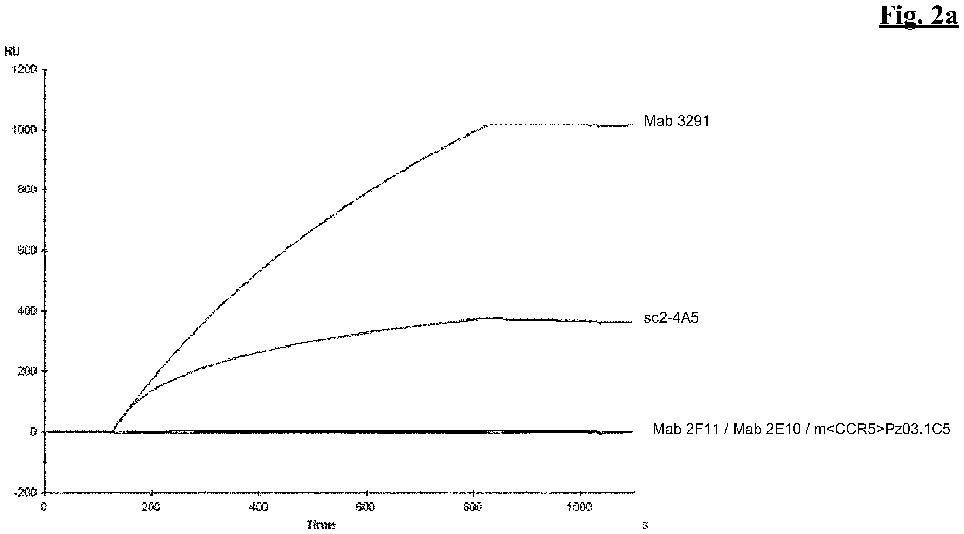

- **Figure 2a**: Biacore sensogram of binding of different anti-CSF-1R antibodies to immobilized human CSF-1R fragment delD4 (comprising the extracellular subdomains D1 -D3 and D5) (SEQ ID NO: 65) (y-axis: binding signal in Response Units (RU), baseline = 0 RU, x-axis: time in seconds (s)): While the antibodies Mab 3291 and sc 2-4A5 clearly show binding to this delD4 fragment, the antibodies according to the invention e.g. Mab 2F11, and Mab 2E10, did not bind to the CSF-1R fragment delD4. The control anti-CCR5 antibody m<CCR5>Pz03.1C5 did also not bind to the CSF-1R fragment delD4.

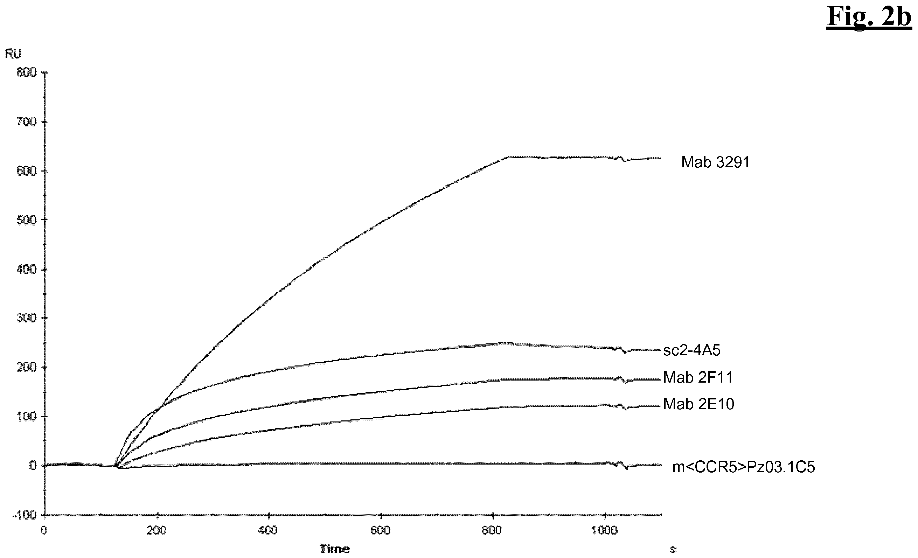

- **Figure 2b**: Biacore sensogram of binding of different anti-CSF-1R antibodies to immobilized human CSF-1R Extracellular Domain (CSF-1R-ECD) (comprising the extracellular subdomains D1-D5) (SEQ ID NO: 64) (y-axis: binding signal in Response Units (RU), baseline = 0 RU, x-axis: time in seconds (s)): All anti-CSF-1R antibodies show binding to CSF-1R-ECD. The control anti-CCR5 antibody m<CCR5>Pz03.1C5 did not bind to the CSF-1R-ECD.

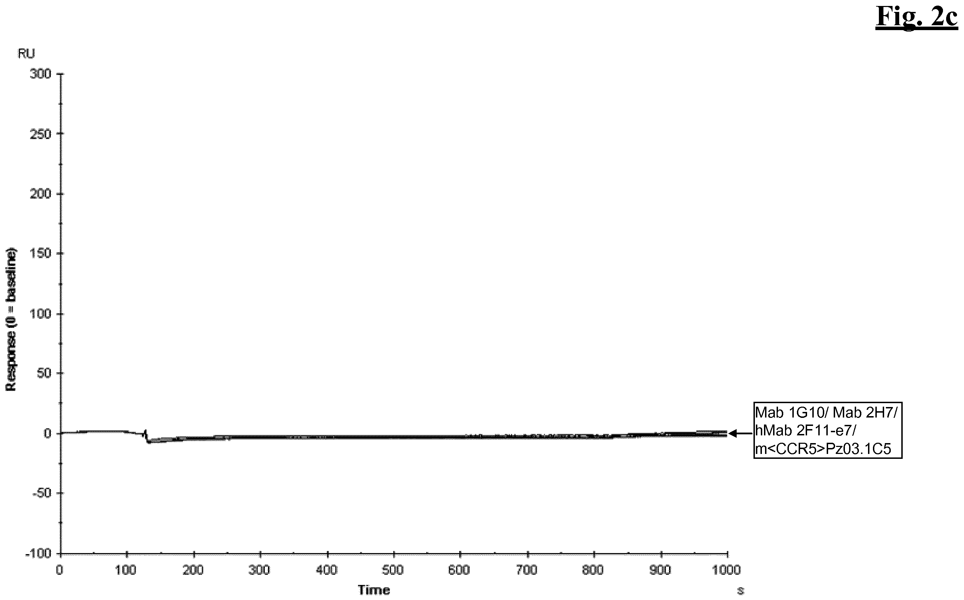

- **Figure 2c**: Biacore sensogram of binding of different anti-CSF-1R antibodies to immobilized human CSF-1R fragment delD4 (comprising the extracellular subdomains D1 -D3 and D5) (SEQ ID NO: 65) (y-axis: binding signal in Response Units (RU), baseline = 0 RU, x-axis: time in seconds (s)): Mab 1G10, Mab 2H7 and humanized hMab 2F11-e7 did not bind to the CSF-1R fragment delD4. The control anti-CCR5 antibody m<CCR5>Pz03.1C5 did also not bind to the CSF-1R fragment delD4.

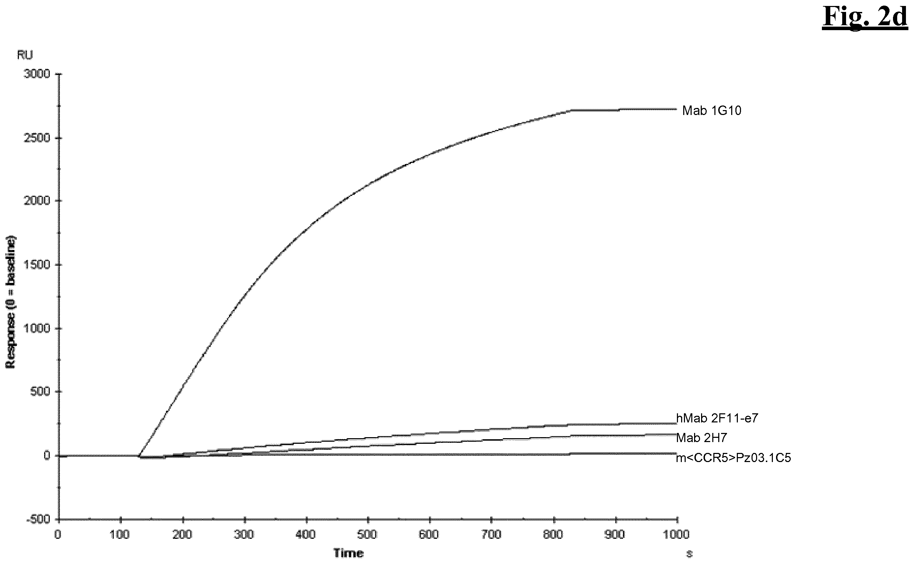

- **Figure 2d**: Biacore sensogram of binding of different anti-CSF-1R antibodies to immobilized human CSF-1R Extracellular Domain (CSF-1R-ECD) (comprising the extracellular subdomains D1-D5) (SEQ ID NO: 64) (y-axis: binding signal in Response Units (RU), baseline = 0 RU, x-axis: time in seconds (s)): All anti-CSF-1R antibodies Mab 1G10, Mab 2H7 and humanized hMab 2F11-e7 showed binding to CSF-1R-ECD. The control anti-CCR5 antibody m<CCR5>Pz03.1C5 did not bind to the CSF-1R-ECD.

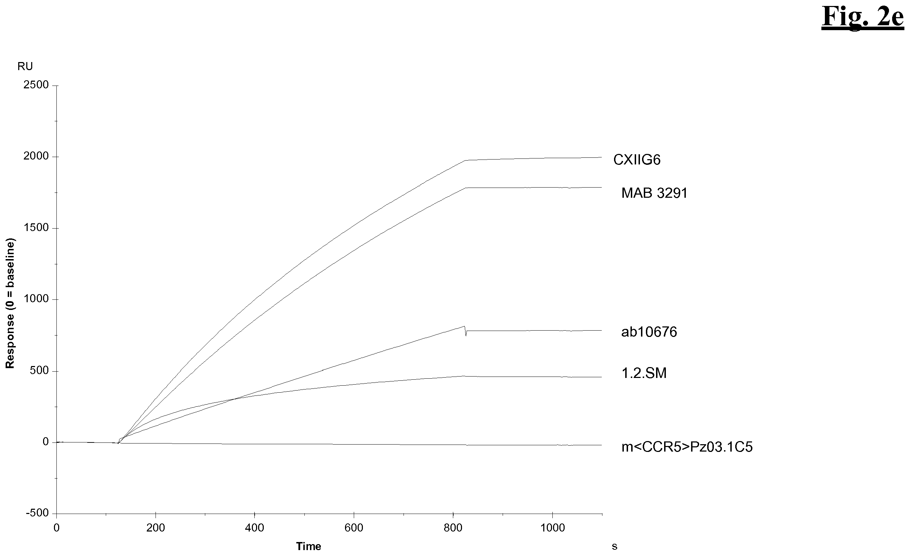

- **Figure 2e**: Biacore sensogram of binding of different anti-CSF-1R antibodies to immobilized human CSF-1R fragment delD4 (comprising the extracellular subdomains D1 -D3 and D5) (SEQ ID NO: 65) (y-axis: binding signal in Response Units (RU), baseline = 0 RU, x-axis: time in seconds (s)): All anti-CSF-1R antibodies 1.2.SM, CXIIG6, ab10676 and MAB3291 show binding to the CSF-1R fragment delD4. The control anti-CCR5 antibody m<CCR5>Pz03.1C5 did also not bind to the CSF-1R fragment delD4.

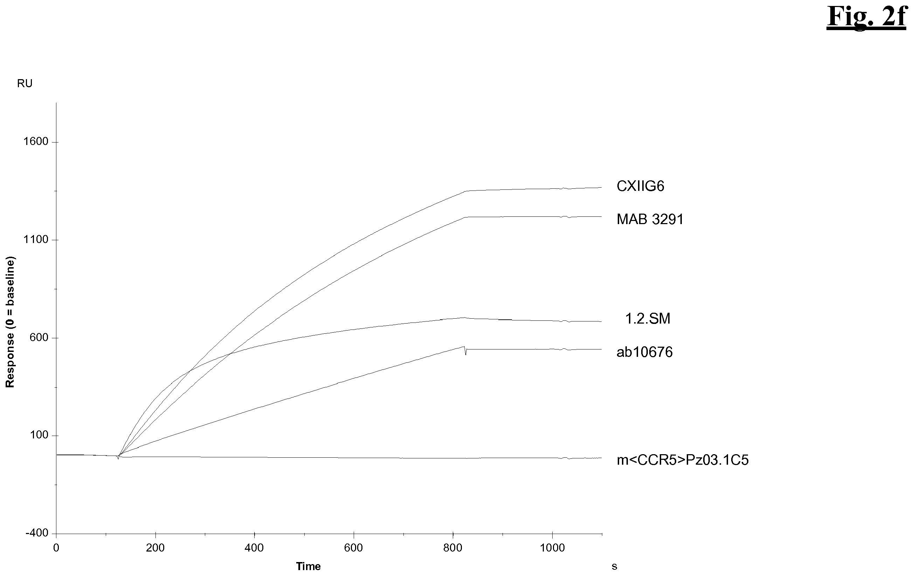

- **Figure 2f**: Biacore sensogram of binding of different anti-CSF-1R antibodies to immobilized human CSF-1R Extracellular Domain (CSF-1R-ECD) (comprising the extracellular subdomains D1-D5) (SEQ ID NO: 64) (y-axis: binding signal in Response Units (RU), baseline = 0 RU, x-axis: time in seconds (s)): All anti-CSF-1R antibodies 1.2.SM, CXIIG6, ab10676 and MAB3291 show binding to CSF-1R-ECD. The control anti-CCR5 antibody m<CCR5>Pz03.1C5 did not bind to the CSF-1R-ECD.

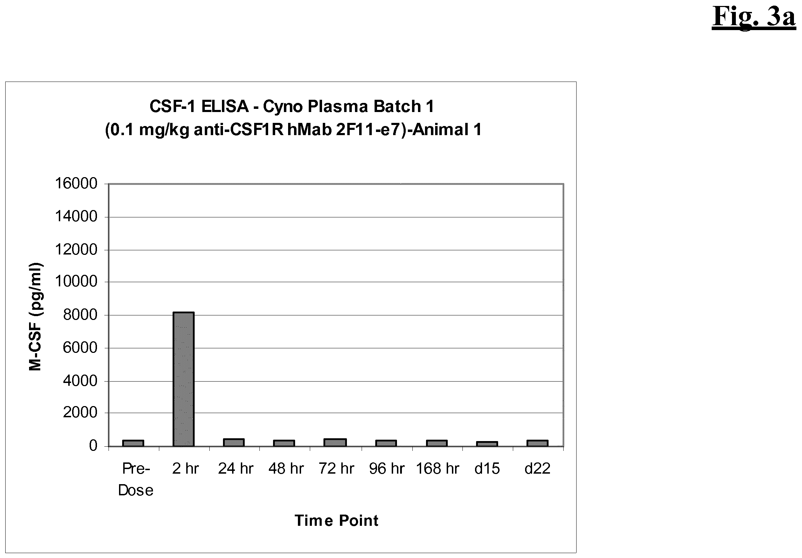

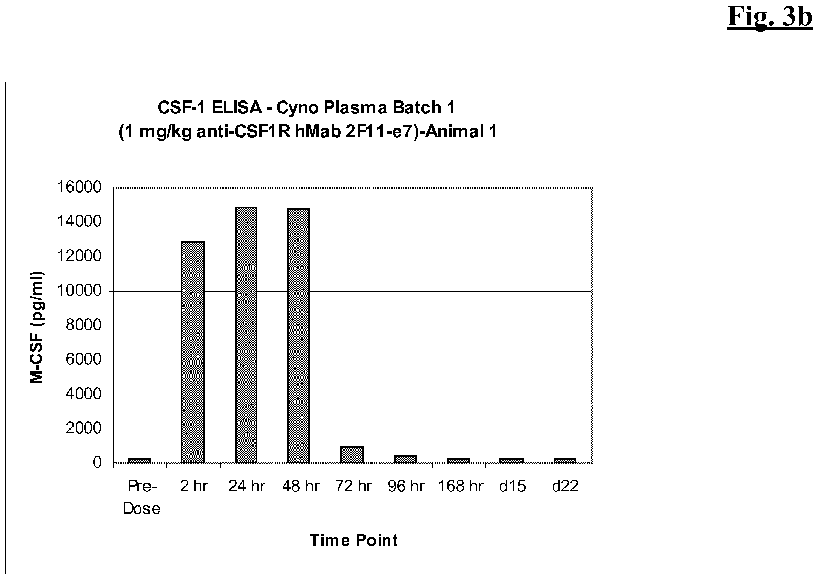

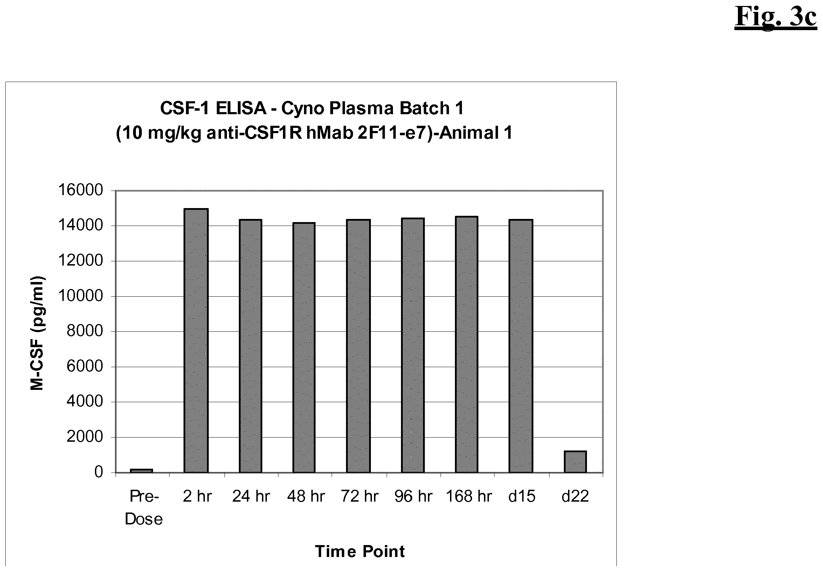

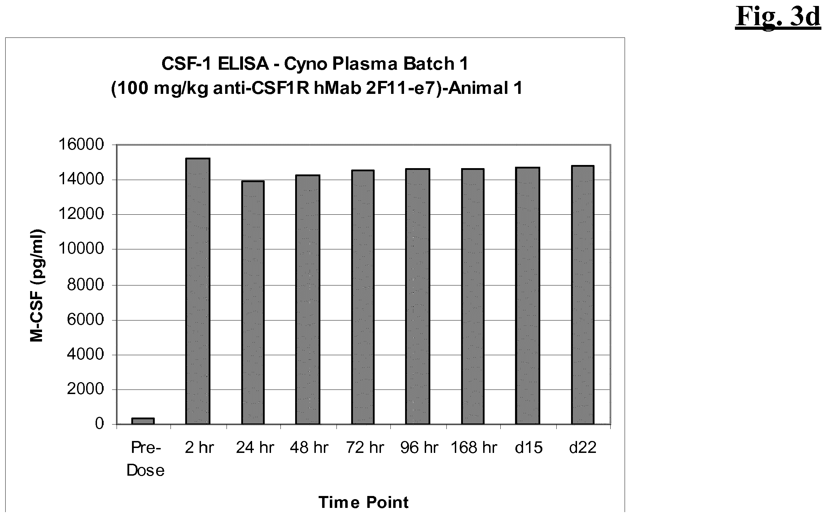

- **Figure 3a-d**: CSF-1 levels in Cynomolgous monkey after application of different dosages of anti-CSF-1R antibody according to the invention.

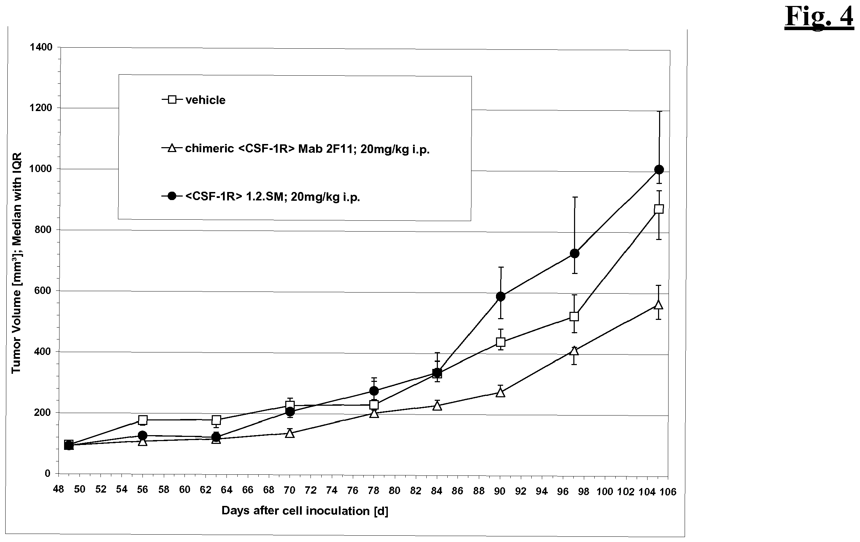

- **Figure 4**: In vivo efficacy - tumor growth inhibition of anti-CSF-1R antibodies according to the invention in breast cancer BT20 xenograft.

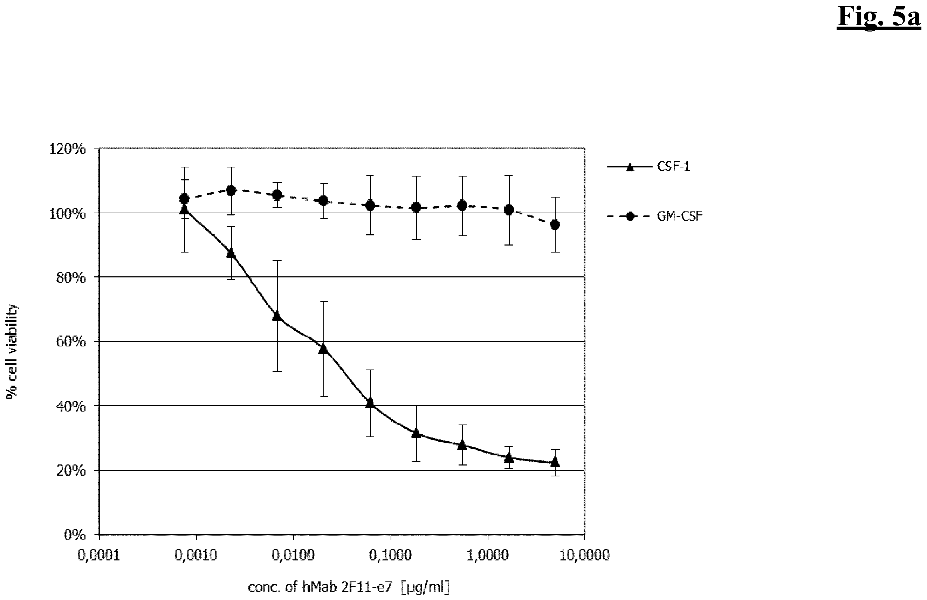

- **Figure 5a-b**: **5a:** Human Monocytes differentiated into macrophages with coculture of GM-CSF or CSF-1 (100ng/ml ligand). After 6 days differentiation addition of RO7155. Cell viability was measured at day 7 of antibody treatment in a CTG Viability Assay (CellTiterGlo® Promega). Calculation of % cell viability: RLU signals from treated cells divided by RLU signal from untreated control without antibody, (n =4)
**5b:** Human Monocytes differentiated into macrophages with GM-CSF (M1) or M-CSF (M2) for 7 days. Phenotype analyzed by indirect fluorescence analysis - staining with anti CD163-PE, anti CD80-PE or anti HLA-DR/DQ/DP-Zenon-Alexa647 labeled. The number in each histogram corresponds to mean ratio fluorescence intensity (MRFI); calculated ratio between mean fluorescence intensity (MFI) of cells stained with the selected antibody (empty histogram) and of corresponding isotyp control (negative control; gray filled histogram) (mean ± SD; n ≥ 5)

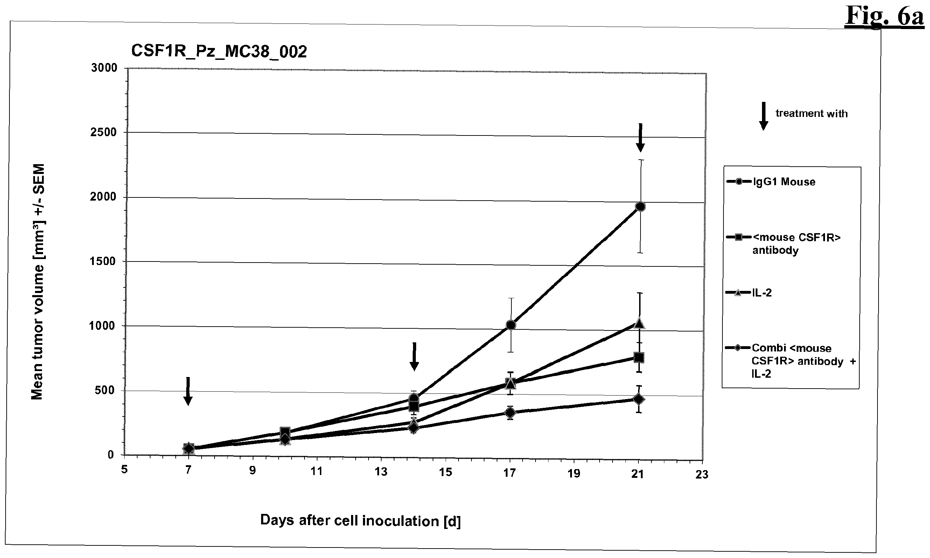

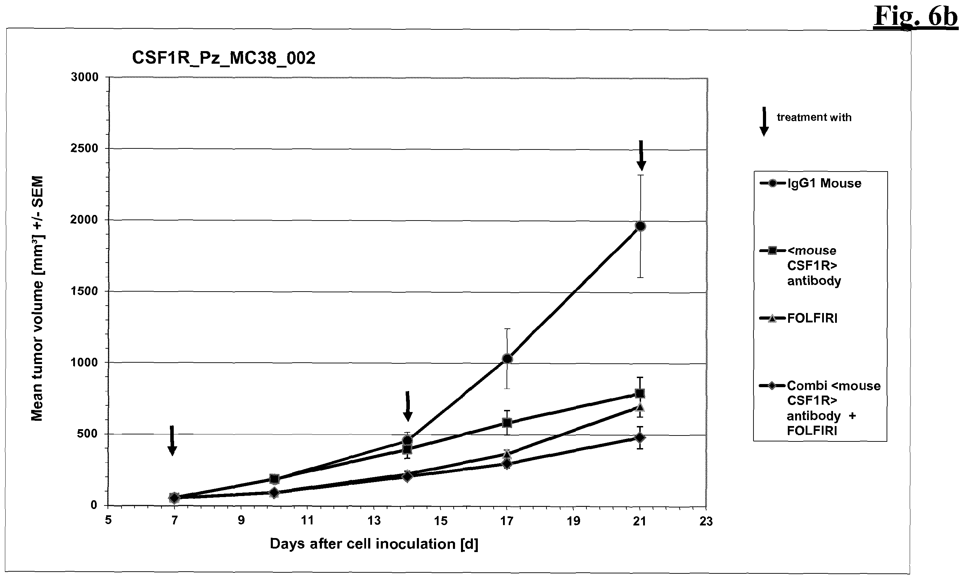

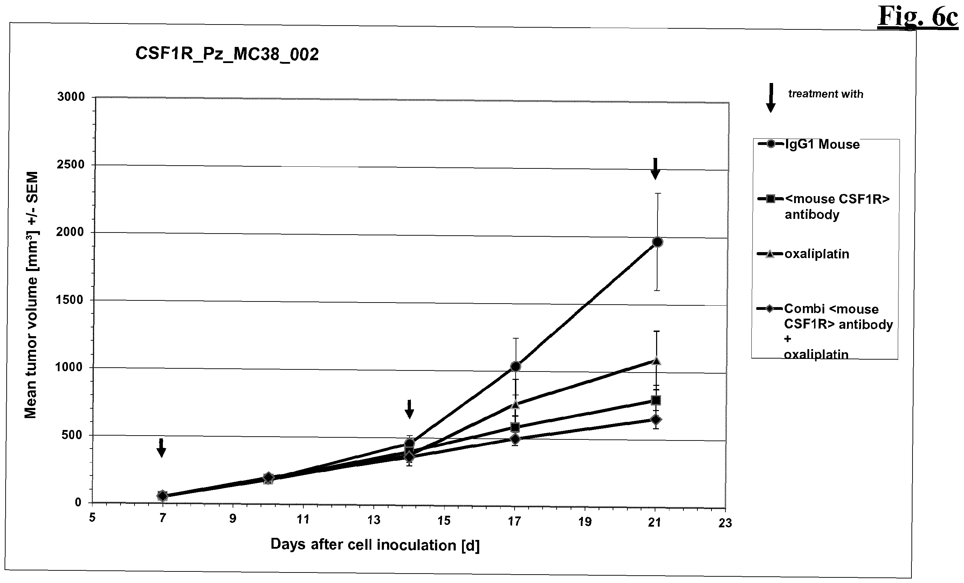

- **Figure 6a -c**: In vivo efficacy of <mouse CSF1R> antibody combinations in the MC38 mouse CRC in vivo model.
- **Figure 7**: In vivo efficacy of <CSF1R> antibody and <CD40> antibody combination: Combination of CSF1R mAb + CD40 mAb FGK45 shows improved anti-tumor efficacy over monotherapies in syngenic MC38 mouse colon cancer model

### Detailed Description of the Invention

The invention is defined in the appended claims and any other aspects set forth herein not falling within the scope of the claims are for information only.

Many tumors are characterized by a prominent immune cell infiltrate, including macrophages. Initially, the immune cells were thought to be part of a defense mechanism against the tumor, but recent data support the notion that several immune cell populations including macrophages may, in fact, promote tumor progression. Macrophages are characterized by their plasticity. Depending on the cytokine microenvironment, macrophages can exhibit so-called M1 or M2-subtypes. M2 macrophages are engaged in the suppression of tumor immunity. They also play an important role in tissue repair functions such as angiogenesis and tissue remodeling which are coopted by the tumor to support growth. In contrast to tumor promoting M2 macrophages, M1 macrophages exhibit antitumor activity via the secretion of inflammatory cytokines and their engagement in antigen presentation and phagocytosis (Mantovani, A. et al., Curr. Opin. Immunol. 2 (2010) 231-237).

By secreting various cytokines such as colony stimulating factor 1 (CSF-1) and IL-10, tumor cells are able to recruit and shape macrophages into the M2- subtype, whereas cytokines such as granulocyte macrophage colony stimulating factor (GM-CSF), IFN-gamma program macrophages towards the M1 subtype. Using immunohistochemistry, it is possible to distinguish between a macrophage subpopulation co-expressing CD68 and CD163, which is likely to be enriched for M2 Macrophages, and a subset showing the CD68+/MHC II+, or CD68+/CD80+ immunophenotype, likely to include M1 macrophages. Cell shape, size, and spatial distribution of CD68 and CD163 positive macrophages is consistent with published hypotheses on a tumor-promoting role of M2 macrophages, for example by their preferential location in tumor intersecting stroma, and vital tumor areas. In contrast, CD68+/MHC class II+ macrophages are ubiquitously found. Their hypothetical role in phagocytosis is reflected by clusters of the CD68+/MHC class II+, but CD163- immunophenotype near apoptotic cells and necrotic tumor areas.

The subtype and marker expression of different macrophage subpopulations is linked with their functional state. M2 macrophages can support tumorigenesis by:
a) enhancing angiogenesis via the secretion of angiogenic factors such as VEGF or bFGF,
b) supporting metastasis formation via secretion of matrix metalloproteinases(MMPs), growth factors and migratory factors guiding the tumor cells to the blood stream and setting up the metastatic niche (Wyckoff, J. et al., Cancer Res. 67 (2007) 2649-2656),
c) playing a role in building an immunosuppressive milieu by secreting immunosuppressive cytokines such as IL-4, Il-13, IL-1ra and IL-10, which in turn regulate T regulatory cell function. Conversely CD4 positive T cells have been shown to enhance the activity of tumor promoting macrophages in preclinical models (Mantovani, A. et al., Eur. J. Cancer 40 (2004) 1660-1667; DeNardo, D. et al., Cancer Cell 16 (2009) 91-102).

Accordingly, in several types of cancer (e.g. breast, ovarian, Hodgkin's lymphoma) the prevalence of M2 subtype tumor associated macrophages (TAMs) has been associated with poor prognosis (Bingle, L. et al., J. Pathol. 3 (2002) 254-265; Orre, M., and Rogers, P.A., Gynecol. Oncol. 1 (1999) 47-50; Steidl, C. et al., N. Engl. J. Med. 10 (2010) 875-885). Recent data show a correlation of CD163 positive macrophage infiltrate in tumors and tumor grade (Kawamura, K. et al., Pathol. Int. 59 (2009) 300-305). TAMs isolated from patient tumors had a tolerant phenotype and were not cytotoxic to tumor cells (Mantovani, A. et al., Eur. J. Cancer 40 (2004) 1660-1667). However, infiltration of TAMs in the presence of cytotoxic T cells correlates with improved survival in non small cell lung cancer and hence reflects a more prominent M1 macrophage infiltrate in this tumor type (Kawai, O. et al., Cancer 6 (2008) 1387-1395).

Recently, a so-called immune signature comprising high numbers of macrophages and CD4 positive T cells, but low numbers of cytotoxic CD8 positive T cells was shown to correlate with reduced overall survival (OS) in breast cancer patients and to represent an independent prognostic factor (DeNardo, D. et al., Cancer Discovery 1 (2011) 54-67).

Consistent with a role for CSF-1 in driving the pro-tumorigenic function of M2 macrophages, high CSF-1 expression in rare sarcomas or locally aggressive connective tissue tumors, such as pigmented villonodular synovitis (PVNS) and tenosynovial giant cell tumor (TGCT) due in part to a translocation of the CSF-1 gene, leads to the accumulation of monocytes and macrophages expressing the receptor for CSF-1, the colony-stimulating factor 1 receptor (CSF-1R) forming the majority of the tumor mass (West, R.B. et al., Proc. Natl. Acad. Sci. USA 3 (2006) 690-695). These tumors were subsequently used to define a CSF-1 dependent macrophage signature by gene expression profiling. In breast cancer and leiomyosarcoma patient tumors this CSF-1 response gene signature predicts poor prognosis (Espinosa, I. et al., Am. J. Pathol. 6 (2009) 2347-2356; Beck, A. et al., Clin. Cancer Res. 3 (2009) 778-787).

CSF-1R belongs to the class III subfamily of receptor tyrosine kinases and is encoded by the c-fms proto-oncogene. Binding of CSF-1 or IL-34 induces receptor dimerization, followed by autophosphorylation and activation of downstream signaling cascades. Activation of CSF-1R regulates the survival, proliferation and differentiation of monocytes and macrophages (Xiong, Y. et al., J. Biol. Chem. 286 (2011) 952-960).

In addition to cells of the monocytic lineage and osteoclasts, which derive from the same hematopoetic precursor as the macrophage, CSF-1R/c-fms has also been found to be expressed by several human epithelial cancers such as ovarian and breast cancer and in leiomyosarcoma and TGCT/PVNS, albeit at lower expression levels compared to macrophages. As with TGCT/PVNS, elevated levels of CSF-1, the ligand for CSF-1R, in serum as well as ascites of ovarian cancer patients have been correlated with poor prognosis (Scholl, S. et al., Br. J. Cancer 62 (1994) 342-346; Price, F. et al., Am. J. Obstet. Gynecol. 168 (1993) 520-527). Furthermore, a constitutively active mutant form of CSF 1R is able to transform NIH3T3 cells, one of the properties of an oncogene (Chambers, S., Future Oncol 5 (2009) 1429-1440).

Preclinical models provide validation of CSF-1R as an oncology target. Blockade of CSF-1 as well as CSF-1R activity results in reduced recruitment of TAMs. Chemotherapy resulted in elevated CSF-1 expression in tumor cells leading to enhanced TAM recruitment. Blockade of CSF-1R in combination with paclitaxel resulted in activation of CD8 positive cytotoxic T cells leading to reduced tumor growth and metastatic burden in a spontaneous transgenic breast cancer model (DeNardo, D. et al., Cancer Discovery 1 (2011) 54-67).

The anti-CSF-1R antibodies described in the invention bind to the membrane proximal extracellular domains D4 and D5 which constitute the receptor dimerization interface. They block CSF-1, IL-34 mediated as well as ligand-independent activation of the receptor resulting in induction of apoptosis of M2-like macrophages differentiated in vitro in the presence of CSF-1 while sparing the M1-like GM-CSF differentiated macrophages. In human breast cancer tissue, M2 (CD68+/CD163+) macrophages and CSF 1R-expressing macrophages are colocalized. In the cynomolgous monkey 13 week treatment with hMab 2F11-e7 reduced CD163 positive macrophages in the liver and colon but not the macrophages of the lung.

Despite the introduction of several new agents, the clinical management of many advanced solid tumors remains challenging. Advances in the understanding of molecular cancer biology have stimulated research into more targeted therapies with the aim of improving the outcome.

CSF-1R is a protein encoded by the CSF-1R gene. It controls the production, differentiation, and function of M2 macrophages, which, in turn, support tumor growth and metastasis formation and secrete immunosuppressive cytokines, leading to a poor prognosis in patients. Furthermore, presence of CSF-1R positive macrophages in several human cancers (such as ovarian and breast carcinoma) has been shown to correlate not only with increased vascular density but also worse clinical outcome. CSF-1R inhibitors, which selectively inhibit M2-like TAMs, have demonstrated activity in preclinical models (DeNardo, D. et al., Cancer Discovery 1 (2011) 54-67; Lin, E. et al., J. Exp. Med. 193 (2001) 727-740). Blockade of CSF-1R activity results in reduced recruitment of TAMs and, in combination with chemotherapy, a synergistic action results in reduced tumor growth and metastatic burden. Recent data have shown that in patients with PVNS and TGCT, overexpression of the CSF-1 is detected and is in part mediated by a translocation of the CSF-1R gene (West, R.B. et al., Proc. Natl. Acad. Sci. USA 3 (2006) 690-695). In breast cancer the presence of a CSF-1 response gene signature predicts risk of recurrence and metastasis (Beck, A. et al., Clin. Cancer Res. 3 (2009) 778-787).

The invention comprises the combination therapy with an antibody binding to human CSF-1R, characterized in that the antibody binds to human CSF-1R Extracellular Domain (SEQ ID NO: 64) (comprising domains D1 to D5) and does not bind to domains D1 to D3 (SEQ ID NO: 66) of the extracellular domain of human CSF-1R.

The invention further comprises the combination therapy with an antibody binding to human CSF-1R, characterized in that the antibody binds to human CSF-1R fragment delD4 (comprising the extracellular subdomains D1-D3 and D5) (SEQ ID NO: 65) and to human CSF-1R Extracellular Domain (CSF-1R-ECD) (comprising the extracellular subdomains D1 -D5) (SEQ ID NO: 64) with a ratio of 1:50 or lower.

The invention further comprises the combination therapy with an antibody binding to human CSF-1R,characterized in comprising as heavy chain variable domain CDR3 region a CDR3 region of SEQ ID NO: 1, SEQ ID NO: 9, SEQ ID NO:23, SEQ ID NO:31, SEQ ID NO:39, SEQ ID NO:47 or SEQ ID NO:55.

The invention further comprises the combination therapy with an antibody binding to human CSF-1R,characterized in that
a) the heavy chain variable domain is SEQ ID NO:7 and the light chain variable domain is SEQ ID NO:8,
b) the heavy chain variable domain is SEQ ID NO:15 and the light chain variable domain is SEQ ID NO:16;
or a humanized version thereof.

The invention further comprises the combination therapy with an antibody binding to human CSF-1R,characterized in that
a) the heavy chain variable domain is SEQ ID NO:7 and the light chain variable domain is SEQ ID NO:8,
b) the heavy chain variable domain is SEQ ID NO:15 and the light chain variable domain is SEQ ID NO:16;
c) the heavy chain variable domain is SEQ ID NO:75 and the light chain variable domain is SEQ ID NO:76;
d) the heavy chain variable domain is SEQ ID NO:83 and the light chain variable domain is SEQ ID NO:84;
or a humanized version thereof.

The invention further comprises the combination therapy with an antibody binding to human CSF-1R,characterized in that
the heavy chain variable domain is SEQ ID NO:7 and the light chain variable domain is SEQ ID NO:8, or a humanized version thereof.

In one embodiment the combination therapy with an antibody binding to human CSF-1R, is characterized in that
a) the heavy chain variable domain is SEQ ID NO:23 and the light chain variable domain is SEQ ID NO:24, or
b) the heavy chain variable domain is SEQ ID NO:31 and the light chain variable domain is SEQ ID NO:32, or
c) the heavy chain variable domain is SEQ ID NO:39 and the light chain variable domain is SEQ ID NO:40, or
d) the heavy chain variable domain is SEQ ID NO:47 and the light chain variable domain is SEQ ID NO:48, or
e) the heavy chain variable domain is SEQ ID NO:55 and the light chain variable domain is SEQ ID NO:56.

In one embodiment the combination therapy with an antibody binding to human CSF-1R, is characterized in that
a) the heavy chain variable domain is SEQ ID NO:23 and the light chain variable domain is SEQ ID NO:24, or
b) the heavy chain variable domain is SEQ ID NO:31 and the light chain variable domain is SEQ ID NO:32, or
c) the heavy chain variable domain is SEQ ID NO:39 and the light chain variable domain is SEQ ID NO:40, or
d) the heavy chain variable domain is SEQ ID NO:47 and the light chain variable domain is SEQ ID NO:48.

In one embodiment the antibody according to the invention is characterized in that
the heavy chain variable domain is SEQ ID NO:23 and the light chain variable domain is SEQ ID NO:24.

In one embodiment the combination therapy with an antibody binding to human CSF-1R, is characterized in that
the heavy chain variable domain is SEQ ID NO:31 and the light chain variable domain is SEQ ID NO:32.

In one embodiment the combination therapy with an antibody binding to human CSF-1R, is characterized in that
the heavy chain variable domain is SEQ ID NO:39 and the light chain variable domain is SEQ ID NO:40.

In one embodiment the combination therapy with an antibody binding to human CSF-1R, is characterized in that
the heavy chain variable domain is SEQ ID NO:47 and the light chain variable domain is SEQ ID NO:48.

The invention further comprises the combination therapy with an antibody binding to human CSF-1R,characterized in that
the heavy chain variable domain is SEQ ID NO:15 and the light chain variable domain is SEQ ID NO:16, or a humanized version thereof.

The invention further comprises the combination therapy with an antibody binding to human CSF-1R,characterized in that
the heavy chain variable domain is SEQ ID NO:75 and the light chain variable domain is SEQ ID NO:76;
or a humanized version thereof.

The invention further the combination therapy with an antibody binding to human CSF-1R, characterized in that
the heavy chain variable domain is SEQ ID NO:83 and the light chain variable domain is SEQ ID NO:84;
or a humanized version thereof.

The invention further the combination therapy with an antibody binding to human CSF-1R, characterized in that
a) the heavy chain variable domain comprises a CDR3 region of SEQ ID NO:1, a CDR2 region of SEQ ID NO: 2, and a CDR1 region of SEQ ID NO:3, and the light chain variable domain comprises a CDR3 region of SEQ ID NO: 4, a CDR2 region of SEQ ID NO:5, and a CDR1 region of SEQ ID NO:6, or,
b) the heavy chain variable domain comprises a CDR3 region of SEQ ID NO: 9, a CDR2 region of SEQ ID NO: 10, and a CDR1 region of SEQ ID NO: 11, and the light chain variable domain comprises a CDR3 region of SEQ ID NO:12, a CDR2 region of SEQ ID NO: 13, and a CDR1 region of SEQ ID NO: 14, or
c) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
d) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
e) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
f) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46, or
g) the heavy chain variable domain comprises a CDR3 region of SEQ ID NO: 49, a CDR2 region of SEQ ID NO: 50, and a CDR1 region of SEQ ID NO: 51, and the light chain variable domain comprises a CDR3 region of SEQ ID NO:52, a CDR2 region of SEQ ID NO: 53, and a CDR1 region of SEQ ID NO: 54.

The invention further comprises the combination therapy with an antibody binding to human CSF-1R, characterized in that
a) the heavy chain variable domain comprises a CDR3 region of SEQ ID NO:1, a CDR2 region of SEQ ID NO: 2, and a CDR1 region of SEQ ID NO:3, and the light chain variable domain comprises a CDR3 region of SEQ ID NO: 4, a CDR2 region of SEQ ID NO:5, and a CDR1 region of SEQ ID NO:6, or
b) the heavy chain variable domain comprises a CDR3 region of SEQ ID NO: 9, a CDR2 region of SEQ ID NO: 10, and a CDR1 region of SEQ ID NO: 11, and the light chain variable domain comprises a CDR3 region of SEQ ID NO:12, a CDR2 region of SEQ ID NO: 13, and a CDR1 region of SEQ ID NO: 14, or
c) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
d) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
e) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
f) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46, or
g) the heavy chain variable domain comprises a CDR3 region of SEQ ID NO: 49, a CDR2 region of SEQ ID NO: 50, and a CDR1 region of SEQ ID NO: 51, and the light chain variable domain comprises a CDR3 region of SEQ ID NO:52, a CDR2 region of SEQ ID NO: 53, and a CDR1 region of SEQ ID NO: 54; or
h) the heavy chain variable domain comprises a CDR3 region of SEQ ID NO:69, a CDR2 region of SEQ ID NO: 70, and a CDR1 region of SEQ ID NO:71, and the light chain variable domain comprises a CDR3 region of SEQ ID NO: 72, a CDR2 region of SEQ ID NO:73, and a CDR1 region of SEQ ID NO:74, or
i) the heavy chain variable domain comprises a CDR3 region of SEQ ID NO: 77, a CDR2 region of SEQ ID NO: 78, and a CDR1 region of SEQ ID NO: 79, and the light chain variable domain comprises a CDR3 region of SEQ ID NO:80, a CDR2 region of SEQ ID NO: 81, and a CDR1 region of SEQ ID NO: 82.

In one embodiment the combination therapy with an antibody binding to human CSF-1R, is characterized in that
a) the heavy chain variable domain comprises a CDR3 region of SEQ ID NO:69, a CDR2 region of SEQ ID NO: 70, and a CDR1 region of SEQ ID NO:71, and the light chain variable domain comprises a CDR3 region of SEQ ID NO: 72, a CDR2 region of SEQ ID NO:73, and a CDR1 region of SEQ ID NO:74, or
b) the heavy chain variable domain comprises a CDR3 region of SEQ ID NO: 77, a CDR2 region of SEQ ID NO: 78, and a CDR1 region of SEQ ID NO: 79, and the light chain variable domain comprises a CDR3 region of SEQ ID NO:80, a CDR2 region of SEQ ID NO: 81, and a CDR1 region of SEQ ID NO: 82.

In one embodiment the combination therapy with an antibody binding to human CSF-1R, is characterized in that
a) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
b) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
c) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
d) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46, or
e) the heavy chain variable domain comprises a CDR3 region of SEQ ID NO: 49, a CDR2 region of SEQ ID NO: 50, and a CDR1 region of SEQ ID NO: 51, and the light chain variable domain comprises a CDR3 region of SEQ ID NO:52, a CDR2 region of SEQ ID NO: 53, and a CDR1 region of SEQ ID NO: 54.

In one embodiment the combination therapy with an antibody binding to human CSF-1R, is characterized in that
a) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
b) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
c) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
d) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46.

In one embodiment the combination therapy with an antibody binding to human CSF-1R, is characterized in that
the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22.

In one embodiment the combination therapy with an antibody binding to human CSF-1R, is characterized in that
the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30.

In one embodiment the combination therapy with an antibody binding to human CSF-1R, is characterized in that
the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38.

In one embodiment the combination therapy with an antibody binding to human CSF-1R, is characterized in that
the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46.

In one embodiment the antibody binding to human CSF-1R, characterized in that the antibody binds to human CSF-1R fragment delD4 (SEQ ID NO: 65) and to human CSF-1R-ECD (SEQ ID NO: 64) with a ratio of 1:50 or lower, is further characterized in not binding to human CSF-1R fragment D1-D3 (SEQ ID NO: 66).

The term "antibody" encompasses the various forms of antibodies including but not being limited to whole antibodies, antibody fragments, human antibodies, humanized antibodies, chimeric antibodies, T cell epitope depleted antibodies, and further genetically engineered antibodies as long as the characteristic properties according to the invention are retained. "Antibody fragments" comprise a portion of a full length antibody, preferably the variable domain thereof, or at least the antigen binding site thereof. Examples of antibody fragments include diabodies, single-chain antibody molecules, and multispecific antibodies formed from antibody fragments. scFv antibodies are, e.g., described in Houston, J.S., Methods in Enzymol. 203 (1991) 46-88). In addition, antibody fragments comprise single chain polypeptides having the characteristics of a V_{H} domain binding to CSF-1R, namely being able to assemble together with a V_{L} domain, or of a V_{L} domain binding to CSF-1R, namely being able to assemble together with a V_{H} domain to a functional antigen binding site and thereby providing the property.

The terms "monoclonal antibody" or "monoclonal antibody composition" as used herein refer to a preparation of antibody molecules of a single amino acid composition.

The term "chimeric antibody" refers to a monoclonal antibody comprising a variable region, i.e., binding region, from mouse and at least a portion of a constant region derived from a different source or species, usually prepared by recombinant DNA techniques. Chimeric antibodies comprising a mouse variable region and a human constant region are especially preferred. Such rat/human chimeric antibodies are the product of expressed immunoglobulin genes comprising DNA segments encoding rat immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See e.g. Riechmann, L., et al., Nature 332 (1988) 323-327; and Neuberger, M.S., et al., Nature 314 (1985) 268-270. Optionally the framework region can be modified by further mutations. Also the CDRs can be modified by one or more mutations to generate antibodies according to the invention e.g. by mutagenesis based upon molecular modeling as described by Riechmann, L., et al., Nature 332 (1988) 323-327 and Queen, C., et al., Proc. Natl. Acad. Sci. USA 86 (1989) 10029-10033, or others. Particularly preferred CDRs correspond to those representing sequences recognizing the antigens noted above for chimeric antibodies. A "humanized version of an antibody according to the invention" (which is e.g. of mouse origin) refers to an antibody, which is based on the mouse antibody sequences in which the V_{H} and V_{L} are humanized by standard techniques (including CDR grafting and optionally subsequent mutagenesis of certain amino acids in the framework region and the CDRs). Preferably such humanized version is chimerized with a human constant region (see e.g. Sequences SEQ ID NO:57-61).

Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

In the following examples the terms "Mab" or "muMab" refer to murine monoclonal antibodies such as Mab 2F11 or Mab 2E10, whereas the term "hMab" refers to humanized monoclonal versions of such murine antibodies such as hMab 2F11-cl 1, hMab 2F11-d8, hMab 2F11-e7, hMab 2F11-f12, etc..

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Brueggemann, M., et al., Year Immunol. 7 (1993) 33-40). Human antibodies can also be produced in phage display libraries (Hoogenboom, H.R., and Winter, G.J. Mol. Biol. 227 (1992) 381-388; Marks, J.D., et al., J. Mol. Biol. 222 (1991) 581-597). The techniques of Cole, et al., and Boerner, et al., are also available for the preparation of human monoclonal antibodies (Cole, S.P.C., et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation).

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The antibodies according to the invention include, in addition, such antibodies having "conservative sequence modifications", nucleotide and amino acid sequence modifications which do not affect or alter the above-mentioned characteristics of the antibody according to the invention. Modifications can be introduced by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g. glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, a predicted nonessential amino acid residue in a human anti-CSF-1R antibody can be preferably replaced with another amino acid residue from the same side chain family.

Amino acid substitutions can be performed by mutagenesis based upon molecular modeling as described by Riechmann, L., et al., Nature 332 (1988) 323-327 and Queen, C., et al., Proc. Natl. Acad. Sci. USA 86 (1989) 10029-10033.

The human CSF-1R (CSF-1 receptor; synonyms: M-CSF receptor; Macrophage colony-stimulating factor 1 receptor, Fms proto-oncogene, c-fms, SEQ ID NO: 22)) is known since 1986 (Coussens, L., et al., Nature 320 (1986) 277-280). CSF-1R is a growth factor and encoded by the c-fms proto-oncogene (reviewed e.g. in Roth, P. and Stanley, E.R., Curr. Top. Microbiol. Immunol. 181 (1992) 141-167).

CSF-1R is the receptor for the CSF-1R ligands CSF-1 (macrophage colony stimulating factor, also called M-CSF) (SEQ ID No.: 86) and IL-34 (SEQ ID No.: 87) and mediates the biological effects of these cytokines (Sherr, C.J., et al., Cell 41 (1985) 665-676; Lin, H., et al., Science 320 (2008) 807-811). The cloning of the colony stimulating factor-1 receptor (also called c-fms) was described for the first time in Roussel, M.F., et al., Nature 325 (1987) 549-552. In that publication, it was shown that CSF-1R had transforming potential dependent on changes in the C-terminal tail of the protein including the loss of the inhibitory tyrosine 969 phosphorylation which binds Cbl and thereby regulates receptor down regulation (Lee, P.S., et al., Embo J. 18 (1999) 3616-3628).

CSF-1R is a single chain, transmembrane receptor tyrosine kinase (RTK) and a member of the family of immunoglobulin (Ig) motif containing RTKs characterized by 5 repeated Ig-like subdomains D1-D5 in the extracellular domain (ECD) of the receptor (Wang, Z., et al Molecular and Cellular Biology 13 (1993) 5348-5359). The human CSF-1R Extracellular Domain (CSF-1R-ECD) (SEQ ID NO: 64) comprises all five extracellular Ig-like subdomains D1 -D5. The human CSF-1R fragment delD4 (SEQ ID NO: 65) comprises the extracellular Ig-like subdomains D1-D3 and D5, but is missing the D4 subdomain. The human CSF-1R fragment D1-D3 (SEQ ID NO: 66) comprises the respective subdomains D1-D3. The sequences are listed without the signal peptide MGSGPGVLLL LLVATAWHGQ G (SEQ ID NO: 67). The human CSF-1R fragment D4-D3 (SEQ ID NO: 85) comprises the respective subdomains D4-D3.

Currently two CSF-1R ligands that bind to the extracellular domain of CSF-1R are known. The first one is CSF-1 (colony stimulating factor 1, also called M-CSF, macrophage; human CSF-1, SEQ ID NO: 86) and is found extracellularly as a disulfide-linked homodimer (Stanley, E.R. et al., Journal of Cellular Biochemistry 21 (1983) 151-159; Stanley, E.R. et al., Stem Cells 12 Suppl. 1 (1995) 15-24). The second one is IL-34 (human IL-34; SEQ ID NO: 87) (Hume, D. A. , et al, Blood 119 (2012) 1810-1820). Thus in one embodiment the term "CSF-1R ligand" refers to human CSF-1 (SEQ ID NO: 86) and/or human IL-34 (SEQ ID NO: 87).

For experiments often the active 149 amino acid (aa) fragment of human CSF-1 (aa 33-181 of SEQ ID NO: 86) is used. This active 149 aa fragment of human CSF-1 (aa 33-181 of SEQ ID NO: 86) is contained in all 3 major forms of CSF-1 and is sufficient to mediate binding to CSF-1R (Hume, D. A. , et al, Blood 119 (2012) 1810-1820).

The main biological effects of CSF-1R signaling are the differentiation, proliferation, migration, and survival of hematopoietic precursor cells to the macrophage lineage (including osteoclast). Activation of CSF-1R is mediated by its CSF-1R ligands, CSF-1 (M-CSF) and IL-34. Binding of CSF-1 (M-CSF) to CSF-1R induces the formation of homodimers and activation of the kinase by tyrosine phosphorylation (Li, W. et al, EMBO Journal. 10 (1991) 277-288; Stanley, E.R., et al., Mol. Reprod. Dev. 46 (1997) 4-10).

The intracellular protein tyrosine kinase domain is interrupted by a unique insert domain that is also present in the other related RTK class III family members that include the platelet derived growth factor receptors (PDGFR), stem cell growth factor receptor (c-Kit) and fins-like cytokine receptor (FLT3). In spite of the structural homology among this family of growth factor receptors, they have distinct tissue-specific functions.

CSF-1R is mainly expressed on cells of the monocytic lineage and in the female reproductive tract and placenta. In addition expression of CSF-1R has been reported in Langerhans cells in skin, a subset of smooth muscle cells (Inaba, T., et al., J. Biol. Chem. 267 (1992) 5693-5699), B cells (Baker, A.H., et al., Oncogene 8 (1993) 371-378) and microglia (Sawada, M., et al., Brain Res. 509 (1990) 119-124). Cells with mutant human CSF-1R ((SEQ ID NO: 23) are known to proliferate independently of ligand stimulation.

As used herein, "binding to human CSF-1R" or "specifically binding to human CSF-1R" refers to an antibody specifically binding to the human CSF-1R antigen with a binding affinity of KD-value of 1.0 x 10⁻⁸ mol/l or lower at 35°C, in one embodiment of a KD-value of 1.0 x10⁻⁹ mol/l or lower at 35°C. The binding affinity is determined with a standard binding assay at 35°C, such as surface plasmon resonance technique (BIAcore®, GE-Healthcare Uppsala, Sweden) A method for determining the KD-value of the binding affinity is described in Example 9. Thus an "antibody binding to human CSF-1R" as used herein refers to an antibody specifically binding to the human CSF-1R antigen with a binding affinity of KD 1.0 x 10⁻⁸ mol/l or lower (preferably 1.0 x 10⁻⁸ mol/l - 1.0 x 10⁻¹² mol/l) at 35°C, preferably of a KD 1.0 x10⁻⁹ mol/l or lower at 35°C (preferably 1.0 x 10⁻⁹ mol/l - 1.0 x 10⁻¹² mol/l).

The "binding to human CSF-1R fragment delD4 (SEQ ID NO: 65) and to human CSF-1R Extracellular Domain (SEQ ID NO: 64)" as used herein is measured by a Surface Plasmon Resonance assay (Biacore assay) as described in Example 4. The human CSF-1R fragment delD4 (SEQ ID NO: 65) or human CSF-1R Extracellular Domain (SEQ ID NO: 64), respectively, are captured to the surface (each to a separate surface) and the test antibodies were added (each in a separate measurement) and the respective binding signals (Response Units (RU)) were determined. Reference signals (blank surface) were subtracted. If signals of nonbinding test antibodies were slightly below 0 the values were set as 0. Then the ratio of the respective binding signals (binding signal (RU) to human CSF-1R fragment delD4 /binding signal (RU) to human CSF-1R Extracellular Domain (CSF-1R-ECD)) is determined. The antibodies according to the invention have a ratio of the binding signals (RU(delD4) / RU(CSF-IR-ECD) of 1:50 or lower, preferably of 1:100 or lower (the lower included end is 0 (e.g. if the RU is 0, then the ratio is 0:50 or 0:100)).

This means that such anti-CSF-1R antibodies according to the invention do not bind to the human CSF-1R fragment delD4 (like the anti-CCR5 antibody m<CCR5>Pz03.1C5 (deposited as DSM ACC 2683 on 18.08.2004 at DSMZ) and have binding signals for binding to the human CSF-1R fragment delD4 in the range of the anti-CCR5 antibody m<CCR5>Pz03.1C5, which are below 20 RU (Response Units), preferably below 10 RU in a Surface Plasmon Resonance (BIAcore) assay as shown in Example 4.

The term "binding to human CSF-1R fragment D1-D3" refers to a binding affinity determination by a Surface Plasmon Resonance assay (Biacore assay). The test antibody is captured to the surface and the human CSF-1R fragment D1-D3 (SEQ ID NO: 66) was added and the respective binding affinities were determined. The terms "not binding to human CSF-1R fragment D1-D3" or "which do not bind to human CSF-1R fragment D1-D3" denotes that in such an assay the detected signal was in the area of no more than 1.2 fold of background signal and therefore no significant binding could be detected and no binding affinity could be determined (see Example 10).

One embodiment of the invention is a screening method for selecting antibodies useful in a combination therapy according to the invention comprising the following steps:
a) measuring of the binding of anti-CSF-1R antibodies to human CSF-1R Extracellular Domain (CSF-1R-ECD) (SEQ ID NO: 64) by a Surface Plasmon Resonance assay (Biacore assay),
b) measuring of the binding of anti-CSF-1R antibodies to human CSF-1R fragment D1-D3 (SEQ ID NO: 66) (D1-D3),
c) selecting antibodies which specifically bind to human CSF-1R Extracellular Domain (CSF-1R-ECD) and which do not bind to to human CSF-1R fragment D1-D3 (SEQ ID NO: 66) (D1-D3).

One embodiment of the invention is a screening method for selecting antibodies according to the invention comprising the following steps:
a) determining the binding signal (Response Units (RU)) of anti-CSF-1R antibodies to human CSF-1R fragment delD4 (SEQ ID NO: 65) and to human CSF-1R Extracellular Domain (CSF-1R-ECD) (SEQ ID NO: 64) by a Surface Plasmon Resonance assay (Biacore assay),
b) selecting antibodies with ratio of the binding signals (human CSF-1R fragment delD4/ human CSF-1R Extracellular Domain (CSF-1R-ECD)) of 50:1 or lower.

In one embodiment the determination is performed at 25°C.

In one embodiment the screening method comprises as further steps the measuring of the binding of anti-CSF-1R antibodies to human CSF-1R fragment D1-D3 (SEQ ID NO: 66) (D1-D3) and the selecting of antibodies which show no binding to said fragment.

The term "epitope" denotes a protein determinant of human CSF-1R capable of specifically binding to an antibody. Epitopes usually consist of chemically active surface groupings of molecules such as amino acids or sugar side chains and usually epitopes have specific three dimensional structural characteristics, as well as specific charge characteristics. Conformational and nonconformational epitopes are distinguished in that the binding to the former but not the latter is lost in the presence of denaturing solvents. Preferably an antibody according to the invention binds specifically to native and to denatured CSF-1R.

The "variable domain" (variable domain of a light chain (V_{L}), variable domain of a heavy chain (V_{H})) as used herein denotes each of the pair of light and heavy chain domains which are involved directly in binding the antibody to the antigen. The variable light and heavy chain domains have the same general structure and each domain comprises four framework (FR) regions whose sequences are widely conserved, connected by three "hypervariable regions" (or complementary determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site. The antibody's heavy and light chain CDR3 regions play a particularly important role in the binding specificity/affinity of the antibodies according to the invention and therefore provide a further object of the invention.

The term "antigen-binding portion of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

The terms "nucleic acid" or "nucleic acid molecule", as used herein, are intended to include DNA molecules and RNA molecules. A nucleic acid molecule may be single-stranded or double-stranded, but preferably is double-stranded DNA.

The term "amino acid" as used within this application denotes the group of naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

In one embodiment the antibodies according to the invention inhibit CSF-1 binding to CSF-1R. In one embodiment with an IC50 of 200 ng/ml or lower, in one embodiment with an IC50 of 50 ng/ml or lower. The IC50 of inhibition of CSF-1 binding to CSF-1R can be determined as shown in Example 2.

In one embodiment the antibodies according to the invention inhibit CSF-1-induced CSF-1R phosphorylation (in NIH3T3-CSF-1R recombinant cells).

In one embodiment with an IC50 of 800 ng/ml or lower, in one embodiment with an IC50 of 600 ng/ml or lower, in one embodiment with an IC50 of 250 ng/ml or lower. The IC50 of CSF-1-induced CSF-1R phosphorylation can be determined as shown in Example 3.

In one embodiment the antibodies according to the invention inhibit the growth of recombinant NIH3T3 cells expressing human CSF-1R (SEQ ID No: 62). In one embodiment with an IC50 of 10 µg/ml or lower, in one embodiment with an IC50 of 5 µg/ml or lower, in one embodiment with an IC50 of 2 µg/ml or lower. In one embodiment with an IC30 of 10 µg/ml or lower, in one embodiment with an IC30 of 5 µg/ml or lower, in one embodiment with an IC30 of 2 µg/ml or lower. The IC50 value, the IC30 value or the % growth inhibition is determined as shown in Example 5.

In one embodiment the antibodies according to the invention inhibit the growth of recombinant NIH3T3 cells expressing human mutant CSF-1R L301S Y969F (SEQ ID No: 63). In one embodiment with an IC50 of 15 µg/ml or lower, in one embodiment with an IC50 of 10 µg/ml or lower. In one embodiment with an IC30 of 10 µg/ml or lower, in one embodiment with an IC50 of 5 µg/ml ng/ml or lower; in one embodiment with an IC50 of 2 µg/ml or lower. The IC50 value, the IC30 value or the % growth inhibition is determined as shown in Example 5.

In one embodiment the antibodies according to the invention inhibit the growth of BeWo tumor cells (ATCC CCL-98) by 65 % or more (at an antibody concentration of 10µg/ml; and as compared to the absence of antibody). The % growth inhibition is determined as shown in Example 8. E.g. Mab 2F11 shows a growth inhibition of BeWo tumor cells of 70 %.

In one embodiment the antibodies according to the invention inhibit (both) human and cynomolgous macrophage differentiation (which is indicated by the inhibition of the survival of human and cynomolgous monocytes as shown in Examples 7 and 8). In one embodiment the antibodies according to the invention inhibit the survival of human monocytes with an IC50 of 0.15 µg/ml or lower, in on embodiment with an IC50 of 0.10 µg/ml or lower. The inhibition of the survival of human monocytes is determined as shown in Example 7. In one embodiment the antibodies according to the invention inhibit the survival of cynomolgous monocytes by 80 % or more, in one embodiment by 90 % or more (at an antibody concentration of 5 µg/ml ;and as compared to the absence of antibody). The inhibition of the survival of human monocytes is determined as shown in Example 8.

A further embodiment of the invention is a method for the production of an antibody against CSF-1R characterized in that the sequence of a nucleic acid encoding the heavy chain of a human IgG1 class antibody binding to human CSF-1R according to the invention said modified nucleic acid and the nucleic acid encoding the light chain of said antibody are inserted into an expression vector, said vector is inserted in a eukaryotic host cell, the encoded protein is expressed and recovered from the host cell or the supernatant.

The antibodies according to the invention are preferably produced by recombinant means. Therefore the antibody is preferably an isolated monoclonal antibody. Such recombinant methods are widely known in the state of the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate prokaryotic or eukaryotic host cells like CHO cells, NS0 cells, SP2/0 cells, HEK293 cells, COS cells, yeast, or E.coli cells, and the antibody is recovered from the cells (supernatant or cells after lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; and Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., in J. Immunol. Methods 194 (1996) 191-199.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies are readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

As used herein, the expressions "cell", "cell line", and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included.

The "Fc part" of an antibody is not involved directly in binding of an antibody to an antigen, but exhibit various effector functions. A "Fc part of an antibody" is a term well known to the skilled artisan and defined on the basis of papain cleavage of antibodies. Depending on the amino acid sequence of the constant region of their heavy chains, antibodies or immunoglobulins are divided in the classes: IgA, IgD, IgE, IgG and IgM, and several of these may be further divided into subclasses (isotypes), e.g. IgG1, IgG2, IgG3, and IgG4, IgA1, and IgA2. According to the heavy chain constant regions the different classes of immunoglobulins are called α, δ, ε, γ, and µ, respectively. The Fc part of an antibody is directly involved in ADCC (antibody-dependent cell-mediated cytotoxicity) and CDC (complement-dependent cytotoxicity) based on complement activation, C1q binding and Fc receptor binding. Complement activation (CDC) is initiated by binding of complement factor C1q to the Fc part of most IgG antibody subclasses. While the influence of an antibody on the complement system is dependent on certain conditions, binding to C1q is caused by defined binding sites in the Fc part. Such binding sites are known in the state of the art and described e.g. by Boackle, R.J., et al., Nature 282 (1979) 742-743; Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol.164 (2000) 4178-4184; Hezareh, M., et al., J. Virology 75 (2001) 12161-12168; Morgan, A., et al., Immunology 86 (1995) 319-324; EP 0 307 434. Such binding sites are e.g. L234, L235, D270, N297, E318, K320, K322, P331 and P329 (numbering according to EU index of Kabat, E.A., see below). Antibodies of subclass IgG1, IgG2 and IgG3 usually show complement activation and C1q and C3 binding, whereas IgG4 do not activate the complement system and do not bind C1q and C3.

In one embodiment the antibody according to the invention comprises a Fc part derived from human origin and preferably all other parts of the human constant regions. As used herein the term "Fc part derived from human origin" denotes a Fc part which is either a Fc part of a human antibody of the subclass IgG1, IgG2, IgG3 or IgG4, preferably a Fc part from human IgG1 subclass, a mutated Fc part from human IgG1 subclass (preferably with a mutation on L234A + L235A), a Fc part from human IgG4 subclass or a mutated Fc part from human IgG4 subclass (preferably with a mutation on S228P). Mostly preferred are the human heavy chain constant regions of SEQ ID NO: 58 (human IgG1 subclass), SEQ ID NO: 59 (human IgG1 subclass with mutations L234A and L235A), SEQ ID NO: 60 human IgG4 subclass), or SEQ ID NO: 61 (human IgG4 subclass with mutation S228P).

Preferably the antibody according to the invention is of human IgG1 subclass or of human IgG4 subclass. In one embodiment the antibody according to the invention is of human IgG1 subclass. In one embodiment the antibody according to the invention is of human IgG4 subclass.

In one embodiment the antibody according to the invention is characterized in that the constant chains are of human origin. Such constant chains are well known in the state of the art and e.g. described by Kabat, E.A., (see e.g. Johnson, G. and Wu, T.T., Nucleic Acids Res. 28 (2000) 214-218). For example, a useful human heavy chain constant region comprises an amino acid sequence of SEQ ID NO: 58. For example, a useful human light chain constant region comprises an amino acid sequence of a kappa-light chain constant region of SEQ ID NO: 57.

Another aspect of the invention is the combination therapy with an antibody binding to human CSF-1R, characterized in that
a) the heavy chain variable domain is SEQ ID NO:7 and the light chain variable domain is SEQ ID NO:8,
b) the heavy chain variable domain is SEQ ID NO:15 and the light chain variable domain is SEQ ID NO:16;
or a humanized version thereof.

Another aspect of the invention is the combination therapy with an antibody binding to human CSF-1R, characterized in that
a) the heavy chain variable domain is SEQ ID NO:7 and the light chain variable domain is SEQ ID NO:8,
b) the heavy chain variable domain is SEQ ID NO:15 and the light chain variable domain is SEQ ID NO:16;
c) the heavy chain variable domain is SEQ ID NO:75 and the light chain variable domain is SEQ ID NO:76;
d) the heavy chain variable domain is SEQ ID NO:83 and the light chain variable domain is SEQ ID NO:84;
or a humanized version thereof.

Another aspect of the invention is the combination therapy with an antibody binding to human CSF-1R, characterized in that
the heavy chain variable domain is SEQ ID NO:7 and the light chain variable domain is SEQ ID NO:8, or a humanized version thereof.

Another aspect of the invention is the combination therapy with an antibody binding to human CSF-1R, characterized in that
a) the heavy chain variable domain is SEQ ID NO:23 and the light chain variable domain is SEQ ID NO:24, or
b) the heavy chain variable domain is SEQ ID NO:31 and the light chain variable domain is SEQ ID NO:32, or
c) the heavy chain variable domain is SEQ ID NO:39 and the light chain variable domain is SEQ ID NO:40, or
d) the heavy chain variable domain is SEQ ID NO:47 and the light chain variable domain is SEQ ID NO:48, or
e) the heavy chain variable domain is SEQ ID NO:55 and the light chain variable domain is SEQ ID NO:56.

Another aspect of the invention is the combination therapy with an antibody binding to human CSF-1R, characterized in that
a) the heavy chain variable domain is SEQ ID NO:23 and the light chain variable domain is SEQ ID NO:24, or
b) the heavy chain variable domain is SEQ ID NO:31 and the light chain variable domain is SEQ ID NO:32, or
c) the heavy chain variable domain is SEQ ID NO:39 and the light chain variable domain is SEQ ID NO:40, or
d) the heavy chain variable domain is SEQ ID NO:47 and the light chain variable domain is SEQ ID NO:48.

Another aspect of the invention is the combination therapy with an antibody binding to human CSF-1R, characterized in that
the heavy chain variable domain is SEQ ID NO:23 and the light chain variable domain is SEQ ID NO:24.

Another aspect of the invention is the combination therapy with an antibody binding to human CSF-1R, characterized in that
the heavy chain variable domain is SEQ ID NO:31 and the light chain variable domain is SEQ ID NO:32.

Another aspect of the invention is the combination therapy with an antibody binding to human CSF-1R, characterized in that
the heavy chain variable domain is SEQ ID NO:39 and the light chain variable domain is SEQ ID NO:40.

Another aspect of the invention is the combination therapy with an antibody binding to human CSF-1R, characterized in that
the heavy chain variable domain is SEQ ID NO:47 and the light chain variable domain is SEQ ID NO:48.

Another aspect of the invention is the combination therapy with an antibody binding to human CSF-1R, characterized in that
the heavy chain variable domain is SEQ ID NO:15 and the light chain variable domain is SEQ ID NO:16, or a humanized version thereof.

Another aspect of the invention is the combination therapy with an antibody binding to human CSF-1R, characterized in that
the heavy chain variable domain is SEQ ID NO:75 and the light chain variable domain is SEQ ID NO:76;
or a humanized version thereof.

Another aspect of the invention is the combination therapy with an antibody binding to human CSF-1R, characterized in that
the heavy chain variable domain is SEQ ID NO:83 and the light chain variable domain is SEQ ID NO:84;
or a humanized version thereof.

Another aspect of the invention is the combination therapy with an antibody binding to human CSF-1R, characterized in that
a) the heavy chain variable domain comprises a CDR3 region of SEQ ID NO:1, a CDR2 region of SEQ ID NO: 2, and a CDR1 region of SEQ ID NO:3, and the light chain variable domain comprises a CDR3 region of SEQ ID NO: 4, a CDR2 region of SEQ ID NO:5, and a CDR1 region of SEQ ID NO:6, or,
b) the heavy chain variable domain comprises a CDR3 region of SEQ ID NO: 9, a CDR2 region of SEQ ID NO: 10, and a CDR1 region of SEQ ID NO: 11, and the light chain variable domain comprises a CDR3 region of SEQ ID NO:12, a CDR2 region of SEQ ID NO: 13, and a CDR1 region of SEQ ID NO: 14, or
c) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
d) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
e) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
f) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46, or
g) the heavy chain variable domain comprises a CDR3 region of SEQ ID NO: 49, a CDR2 region of SEQ ID NO: 50, and a CDR1 region of SEQ ID NO: 51, and the light chain variable domain comprises a CDR3 region of SEQ ID NO:52, a CDR2 region of SEQ ID NO: 53, and a CDR1 region of SEQ ID NO: 54; or
h) the heavy chain variable domain comprises a CDR3 region of SEQ ID NO:69, a CDR2 region of SEQ ID NO: 70, and a CDR1 region of SEQ ID NO:71, and the light chain variable domain comprises a CDR3 region of SEQ ID NO: 72, a CDR2 region of SEQ ID NO:73, and a CDR1 region of SEQ ID NO:74, or
i) the heavy chain variable domain comprises a CDR3 region of SEQ ID NO: 77, a CDR2 region of SEQ ID NO: 78, and a CDR1 region of SEQ ID NO: 79, and the light chain variable domain comprises a CDR3 region of SEQ ID NO:80, a CDR2 region of SEQ ID NO: 81, and a CDR1 region of SEQ ID NO: 82.

Another aspect of the invention is the combination therapy with an antibody binding to human CSF-1R, characterized in that
a) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
b) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
c) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
d) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46, or
e) the heavy chain variable domain comprises a CDR3 region of SEQ ID NO: 49, a CDR2 region of SEQ ID NO: 50, and a CDR1 region of SEQ ID NO: 51, and the light chain variable domain comprises a CDR3 region of SEQ ID NO:52, a CDR2 region of SEQ ID NO: 53, and a CDR1 region of SEQ ID NO: 54.

Another aspect of the invention is the combination therapy with an antibody binding to human CSF-1R, characterized in that
a) the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22, or
b) the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30, or
c) the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38, or
d) the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46.

Another aspect of the invention is the combination therapy with an antibody binding to human CSF-1R, characterized in that
the heavy chain variable domain comprises a CDR3 region of SEQ ID NO: 17, a CDR2 region of SEQ ID NO: 18, and a CDR1 region of SEQ ID NO:19, and the light chain variable domain comprises a CDR3 region of SEQ ID NO: 20, a CDR2 region of SEQ ID NO:21, and a CDR1 region of SEQ ID NO:22.

Another aspect of the invention is the combination therapy with an antibody binding to human CSF-1R, characterized in that
the heavy chain variable domain comprises a CDR3 region of SEQ ID NO: 25, a CDR2 region of SEQ ID NO: 26, and a CDR1 region of SEQ ID NO: 27, and the light chain variable domain comprises a CDR3 region of SEQ ID NO:28, a CDR2 region of SEQ ID NO: 29, and a CDR1 region of SEQ ID NO: 30.

Another aspect of the invention is the combination therapy with an antibody binding to human CSF-1R, characterized in that
the heavy chain variable domain comprises a CDR3 region of SEQ ID NO: 33, a CDR2 region of SEQ ID NO: 34, and a CDR1 region of SEQ ID NO: 35, and the light chain variable domain comprises a CDR3 region of SEQ ID NO:36, a CDR2 region of SEQ ID NO: 37, and a CDR1 region of SEQ ID NO: 38.

Another aspect of the invention is the combination therapy with an antibody binding to human CSF-1R, characterized in that
the heavy chain variable domain comprises a CDR3 region of SEQ ID NO:41, a CDR2 region of SEQ ID NO: 42, and a CDR1 region of SEQ ID NO:43, and the light chain variable domain comprises a CDR3 region of SEQ ID NO: 44, a CDR2 region of SEQ ID NO:45, and a CDR1 region of SEQ ID NO:46.

The invention comprises a method for the treatment of a patient in need of therapy, characterized by administering to the patient a therapeutically effective amount of an antibody according to the invention.

There are 3 distinct mechanisms by which CSF-1R signaling is likely involved in tumor growth and metastasis. The first is that expression of CSF-ligand and receptor has been found in tumor cells originating in the female reproductive system (breast, ovarian, endometrium, cervical) (Scholl, S.M., et al., J. Natl. Cancer Inst. 86 (1994) 120-126; Kacinski, B.M., Mol. Reprod. Dev. 46 (1997) 71-74; Ngan, H.Y., et al., Eur. J. Cancer 35 (1999) 1546-1550; Kirma, N., et al., Cancer Res 67 (2007) 1918-1926) and the expression has been associated with breast cancer xenograft growth as well as poor prognosis in breast cancer patients. Two point mutations were seen in CSF-1R in about 10-20% of acute myelocytic leukemia, chronic myelocytic leukemia and myelodysplasia patients tested in one study, and one of the mutations was found to disrupt receptor turnover (Ridge, S.A., et al., Proc. Natl. Acad. Sci USA 87 (1990) 1377-1380). However the incidence of the mutations could not be confirmed in later studies (Abu-Duhier, F.M., et al., Br. J. Haematol. 120 (2003) 464-470). Mutations were also found in some cases of hepatocellular cancer (Yang, D.H., et al., Hepatobiliary Pancreat. Dis. Int. 3 (2004) 86-89) and idiopathic myelofibrosis (Abu-Duhier, F.M., et al., Br. J. Haematol. 120 (2003) 464-470). Recently, in the GDM-1 cell line derived from a patient with myelomonoblastic leukemia the Y571D mutation in CSF-1R was identified (Chase, A., et al., Leukemia 23 (2009) 358-364).

Pigmented villonodular synovitis (PVNS) and Tenosynovial Giant cell tumors (TGCT) can occur as a result of a translocation that fuses the M-CSF gene to a collagen gene COL6A3 and results in overexpression of M-CSF (West, R.B., et al., Proc. Natl. Acad. Sci. USA 103 (2006) 690-695). A landscape effect is proposed to be responsible for the resulting tumor mass that consists of monocytic cells attracted by cells that express M-CSF. TGCTs are smaller tumors that can be relatively easily removed from fingers where they mostly occur. PVNS is more aggressive as it can recur in large joints and is not as easily controlled surgically.

The second mechanism is based on blocking signaling through M-CSF/CSF-1R at metastatic sites in bone which induces osteoclastogenesis, bone resorption and osteolytic bone lesions. Breast, multiple myeloma and lung cancers are examples of cancers that have been found to metastasize to the bone and cause osteolytic bone disease resulting in skeletal complications. M-CSF released by tumor cells and stroma induces the differentiation of hematopoietic myeloid monocyte progenitors to mature osteoclasts in collaboration with the receptor activator of nuclear factor kappa-B ligand-RANKL. During this process, M-CSF acts as a permissive factor by giving the survival signal to osteoclasts (Tanaka, S., et al., J. Clin. Invest. 91 (1993) 257-263). Inhibition of CSF-1R activity during osteoclast differentiation and maturation with an anti-CSF-1R antibody is likely to prevent unbalanced activity of osteoclasts that cause osteolytic disease and the associated skeletal related events in metastatic disease. Whereas breast, lung cancer and multiple myeloma typically result in osteolytic lesions, metastasis to the bone in prostate cancer initially has an osteoblastic appearance in which increased bone forming activity results in 'woven bone' which is different from typical lamellar structure of normal bone. During disease progression bone lesions display a significant osteolytic component as well as high serum levels of bone resorption and suggests that anti-resorptive therapy may be useful. Bisphosphonates have been shown to inhibit the formation of osteolytic lesions and reduced the number of skeletal-related events only in men with hormone-refractory metastatic prostate cancer but at this point their effect on osteoblastic lesions is controversial and bisphosphonates have not been beneficial in preventing bone metastasis or hormone responsive prostate cancer to date. The effect of anti-resorptive agents in mixed osteolytic/osteoblastic prostate cancer is still being studied in the clinic (Choueiri, M.B., et al., Cancer Metastasis Rev. 25 (2006) 601-609; Vessella, R.L. and Corey, E., Clin. Cancer Res. 12 (20 Pt 2) (2006) 6285s-6290s).

The third mechanism is based on the recent observation that tumor associated macrophages (TAM) found in solid tumors of the breast, prostate, ovarian and cervical cancers correlated with poor prognosis (Bingle, L., et al., J. Pathol. 196 (2002) 254-265; Pollard, J.W., Nat. Rev. Cancer 4 (2004) 71-78). Macrophages are recruited to the tumor by M-CSF and other chemokines. The macrophages can then contribute to tumor progression through the secretion of angiogenic factors, proteases and other growth factors and cytokines and may be blocked by inhibition of CSF-1R signaling. Recently it was shown by Zins et al (Zins, K., et al., Cancer Res. 67 (2007) 1038-1045) that expression of siRNA of Tumor necrosis factor alpha (TNF alpha), M-CSF or the combination of both would reduce tumor growth in a mouse xenograft model between 34% and 50% after intratumoral injection of the respective siRNA. SiRNA targeting the TNF alpha secreted by the human SW620 cells reduced mouse M-CSF levels and led to reduction of macrophages in the tumor. In addition treatment of MCF7 tumor xenografts with an antigen binding fragment directed against M-CSF did result in 40% tumor growth inhibition, reversed the resistance to chemotherapeutics and improved survival of the mice when given in combination with chemotherapeutics (Paulus, P., et al., Cancer Res. 66 (2006) 4349-4356).

TAMs are only one example of an emerging link between chronic inflammation and cancer. There is additional evidence for a link between inflammation and cancer as many chronic diseases are associated with an increased risk of cancer, cancers arise at sites of chronic inflammation, chemical mediators of inflammation are found in many cancers; deletion of the cellular or chemical mediators of inflammation inhibits development of experimental cancers and long-term use of anti-inflammatory agents reduce the risk of some cancers. A link to cancer exists for a number of inflammatory conditions among- those H.pylori induced gastritis for gastric cancer, Schistosomiasis for bladder cancer, HHVX for Kaposi's sarcoma, endometriosis for ovarian cancer and prostatitis for prostate cancer (Balkwill, F., et al., Cancer Cell 7 (2005) 211-217). Macrophages are key cells in chronic inflammation and respond differentially to their microenvironment. There are two types of macrophages that are considered extremes in a continuum of functional states: M1 macrophages are involved in Type 1 reactions. These reactions involve the activation by microbial products and consequent killing of pathogenic microorganisms that result in reactive oxygen intermediates. On the other end of the extreme are M2 macrophages involved in Type 2 reactions that promote cell proliferation, tune inflammation and adaptive immunity and promote tissue remodeling, angiogenesis and repair (Mantovani, A., et al., Trends Immunol. 25 (2004) 677-686). Chronic inflammation resulting in established neoplasia is usually associated with M2 macrophages. A pivotal cytokine that mediates inflammatory reactions is TNF alpha that true to its name can stimulate anti-tumor immunity and hemorrhagic necrosis at high doses but has also recently been found to be expressed by tumor cells and acting as a tumor promoter (Zins, K., et al., Cancer Res. 67 (2007) 1038-1045; Balkwill, F., Cancer Metastasis Rev. 25 (2006) 409-416). The specific role of macrophages with respect to the tumor still needs to be better understood including the potential spatial and temporal dependence on their function and the relevance to specific tumor types.

Thus one embodiment of the invention are the CSF-1R antibodies of the present invention for use in the treatment of cancer. The term "cancer" as used herein may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. Preferably such cancer is a breast cancer, ovarian cancer , cervical cancer, lung cancer or prostate cancer. Preferably such cancers are further characterized by CSF-1 or CSF-1R expression or overexpression. One further embodiment the invention are the CSF-1R antibodies of the present invention for use in the simultaneous treatment of primary tumors and new metastases.

Thus another embodiment of the invention are the CSF-1R antibodies of the present invention for use in the treatment of periodontitis, histiocytosis X, osteoporosis, Paget's disease of bone (PDB), bone loss due to cancer therapy, periprosthetic osteolysis, glucocorticoid-induced osteoporosis, rheumatoid arthritis, psiratic arthritis, osteoarthritis, inflammatory arthridities, and inflammation.

Rabello, D., et al., Biochem. Biophys. Res. Commun. 347 (2006) 791-796 has demonstrated that SNPs in the CSF1 gene exhibited a positive association with aggressive periodontitis: an inflammatory disease of the periodontal tissues that causes tooth loss due to resorption of the alveolar bone.

Histiocytosis X (also called Langerhans cell histiocytosis, LCH) is a proliferative disease of Langerhans dendritic cells that appear to differentiate into osteoclasts in bone and extra osseous LCH lesions. Langerhans cells are derived from circulating monocytes. Increased levels of M-CSF that have been measured in sera and lesions where found to correlate with disease severity (da Costa, C.E., et al., J. Exp. Med. 201 (2005) 687-693). The disease occurs primarily in a pediatric patient population and has to be treated with chemotherapy when the disease becomes systemic or is recurrent.

The pathophysiology of osteoporosis is mediated by loss of bone forming osteoblasts and increased osteoclast dependent bone resorption. Supporting data has been described by Cenci et al showing that an anti-M-CSF antibody injection preserves bone density and inhibits bone resorption in ovariectomized mice (Cenci, S., et al., J. Clin. Invest. 105 (2000) 1279-1287). Recently a potential link between postmenopausal bone loss due to estrogen deficiency was identified and found that the presence of TNF alpha producing T-cell affected bone metabolism (Roggia, C., et al., Minerva Med. 95 (2004) 125-132). A possible mechanism could be the induction of M-CSF by TNF alpha in vivo. An important role for M-CSF in TNF-alpha-induced osteoclastogenesis was confirmed by the effect of an antibody directed against M-CSF that blocked the TNF alpha induced osteolysis in mice and thereby making inhibitors of CSF-1R signaling potential targets for inflammatory arthritis (Kitaura, H., et al., J. Clin. Invest. 115 (2005) 3418-3427).

Paget's disease of bone (PDB) is the second most common bone metabolism disorder after osteoporosis in which focal abnormalities of increased bone turnover lead to complications such as bone pain, deformity, pathological fractures and deafness. Mutations in four genes have been identified that regulate normal osteoclast function and predispose individuals to PDB and related disorders: insertion mutations in TNFRSF11A, which encodes receptor activator of nuclear factor (NF) kappaB (RANK)-a critical regulator of osteoclast function, inactivating mutations of TNFRSF11B which encodes osteoprotegerin (a decoy receptor for RANK ligand), mutations of the sequestosome 1 gene (SQSTM1), which encodes an important scaffold protein in the NFkappaB pathway and mutations in the valosin-containing protein (VCP) gene. This gene encodes VCP, which has a role in targeting the inhibitor of NFkappaB for degradation by the proteasome (Daroszewska, A. and Ralston, S.H., Nat. Clin. Pract. Rheumatol. 2 (2006) 270-277). Targeted CSF-1R inhibitors provide an opportunity to block the deregulation of the RANKL signaling indirectly and add an additional treatment option to the currently used bisphosphonates.

Cancer therapy induced bone loss especially in breast and prostate cancer patients is an additional indication where a targeted CSF-1R inhibitor could prevent bone loss (Lester, J.E., et al., Br. J. Cancer 94 (2006) 30-35). With the improved prognosis for early breast cancer the long-term consequences of the adjuvant therapies become more important as some of the therapies including chemotherapy, irradiation, aromatase inhibitors and ovary ablation affect bone metabolism by decreasing the bone mineral density, resulting in increased risk for osteoporosis and associated fractures (Lester, J.E., et al., Br. J. Cancer 94 (2006) 30-35). The equivalent to adjuvant aromatase inhibitor therapy in breast cancer is androgen ablation therapy in prostate cancer which leads to loss of bone mineral density and significantly increases the risk of osteoporosis-related fractures (Stoch, S.A., et al., J. Clin. Endocrinol. Metab. 86 (2001) 2787-2791).

Targeted inhibition of CSF-1R signaling is likely to be beneficial in other indications as well when targeted cell types include osteoclasts and macrophages e.g. treatment of specific complications in response to joint replacement as a consequence of rheumatoid arthritis. Implant failure due to periprosthetic bone loss and consequent loosing of prostheses is a major complication of joint replacement and requires repeated surgery with high socioeconomic burdens for the individual patient and the health-care system. To date, there is no approved drug therapy to prevent or inhibit periprosthetic osteolysis (Drees, P., et al., Nat. Clin. Pract. Rheumatol. 3 (2007) 165-171).

Glucocorticoid-induced osteoporosis (GIOP) is another indication in which a CSF-1R inhibitor could prevent bone loss after longterm glucocorticocosteroid use that is given as a result of various conditions among those chronic obstructive pulmonary disease, asthma and rheumatoid arthritis (Guzman-Clark, J.R., et al., Arthritis Rheum. 57 (2007) 140-146; Feldstein, A.C., et al., Osteoporos. Int. 16 (2005) 2168-2174).

Rheumatoid arthritis, psioratic arthritis and inflammatory arthridities are in itself potential indications for CSF-1R signaling inhibitors in that they consist of a macrophage component and to a varying degree bone destruction (Ritchlin, C.T., et al., J. Clin. Invest. 111 (2003) 821-831). Osteoarthritis and rheumatoid arthritis are inflammatory autoimmune disease caused by the accumulation of macrophages in the connective tissue and infiltration of macrophages into the synovial fluid, which is at least partially mediated by M-CSF. Campbell, I., K., et al., J. Leukoc. Biol. 68 (2000) 144-150, demonstrated that M-CSF is produced by human-joint tissue cells (chondrocytes, synovial fibroblasts) in vitro and is found in synovial fluid of patients with rheumatoid arthritis, suggesting that it contributes to the synovial tissue proliferation and macrophage infiltration which is associated with the pathogenesis of the disease. Inhibition of CSF-1R signaling is likely to control the number of macrophages in the joint and alleviate the pain from the associated bone destruction. In order to minimize adverse effects and to further understand the impact of the CSF-1R signaling in these indications, one method is to specifically inhibit CSF-1R without targeting a myriad other kinases, such as Raf kinase.

Recent literature reports correlate increased circulating M-CSF with poor prognosis and atherosclerotic progression in chronic coronary artery disease (Saitoh, T., et al., J. Am. Coll. Cardiol. 35 (2000) 655-665; Ikonomidis, I., et al., Eur. Heart. J. 26 (2005) p. 1618-1624); M-CSF influences the atherosclerotic process by aiding the formation of foam cells (macrophages with ingested oxidized LDL) that express CSF-1R and represent the initial plaque (Murayama, T., et al., Circulation 99 (1999) 1740-1746).

Expression and signaling of M-CSF and CSF-1R is found in activated microglia. Microglia, which are resident macrophages of the central nervous system, can be activated by various insults, including infection and traumatic injury. M-CSF is considered a key regulator of inflammatory responses in the brain and M-CSF levels increase in HIV-1, encephalitis, Alzheimer's disease (AD) and brain tumors. Microgliosis as a consequence of autocrine signaling by M-CSF/CSF-1R results in induction of inflammatory cytokines and nitric oxides being released as demonstrated by e.g. using an experimental neuronal damage model (Hao, A.J., et al., Neuroscience 112 (2002) 889-900; Murphy, G.M., Jr., et al., J. Biol. Chem. 273 (1998) 20967-20971). Microglia that have increased expression of CSF-1R are found to surround plaques in AD and in the amyloid precursor protein V717F transgenic mouse model of AD (Murphy, G.M., Jr., et al., Am. J. Pathol. 157 (2000) 895-904). On the other hand op/op mice with fewer microglia in the brain resulted in fibrilar deposition of A-beta and neuronal loss compared to normal control suggesting that microglia do have a neuroprotective function in the development of AD lacking in the op/op mice (Kaku, M., et al., Brain Res. Brain Res. Protoc. 12 (2003) 104-108).

Expression and signaling of M-CSF and CSF-1R is associated with inflammatory bowel disease (IBD) (WO 2005/046657). The term "inflammatory bowel disease" refers to serious, chronic disorders of the intestinal tract characterized by chronic inflammation at various sites in the gastrointestinal tract, and specifically includes ulcerative colitis (UC) and Crohn's disease.

The invention comprises the combination therapy with an antibody binding to human CSF-1R being characterized by the above mentioned epitope binding properties or alternatively by the above mentioned amino acid sequences and amino acid sequence fragments for the treatment of cancer.

The invention comprises the use of an antibody characterized in comprising the antibody binding to human CSF-1R being characterized by the above mentioned epitope binding properties or alternatively by the above mentioned amino acid sequences and amino acid sequence fragments for the combination treatment of cancer as described herein or alternatively for the manufacture of a medicament for the combination treatment of cancer as described herein.

The antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains or fragments thereof are inserted into expression vectors by standard methods. Expression is performed in appropriate prokaryotic or eukaryotic host cells, such as CHO cells, NS0 cells, SP2/0 cells, HEK293 cells, COS cells, yeast, or E. coli cells, and the antibody is recovered from the cells (from the supernatant or after cells lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified, or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; Norderhaug, L., et al., J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199.

Nucleic acid molecules encoding amino acid sequence variants of anti-CSF-1R antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of humanized anti-CSF-1R antibody.

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

In another aspect, the present invention provides a composition, e.g. a pharmaceutical composition, containing one or a combination of monoclonal antibodies, or the antigen-binding portion thereof, of the present invention, formulated together with a pharmaceutically acceptable carrier.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion.

A composition of the present invention can be administered by a variety of methods known in the art. As will be appreciated by the skilled artisan, the route and/or mode of administration will vary depending upon the desired results.

Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient (effective amount). The selected dosage level will depend upon a variety of pharmacokinetic factors including the activity of the particular compositions of the present invention employed, or the ester, salt or amide thereof, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "administered in combination with" or "co-administration", "coadministering" refer to the administration of the anti-CSF-1R, and the chemotherapeutic agent, radiotherapy and/ or cancer immunotherapy e.g. as separate formulations/applications (or as one single formulation/application). The co-administration can be simultaneous or sequential in either order, wherein preferably there is a time period while both (or all) active agents simultaneously exert their biological activities. Said antibody and said further agent are co-administered either simultaneously or sequentially (e.g. intravenous (i.v.) through a continuous infusion. When both therapeutic agents are co-administered sequentially the dose is administered either on the same day in two separate administrations, or one of the agents is administered on day 1 and the second is co-administered on day 2 to day 7, preferably on day 2 to 4. Thus in one embodiment the term "sequentially" means within 7 days after the dose of the first component, preferably within 4 days after the dose of the first component; and the term "simultaneously" means at the same time. The terms "co-administration" with respect to the maintenance doses of anti-CSF-1R antibody mean that the maintenance doses can be either co-administered simultaneously, if the treatment cycle is appropriate for both drugs, e.g. every week. Or the further agent is e.g. administered e.g. every first to third day and said antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration and the timing of co-administration will depend on the type (species, gender, age, weight, etc.) and condition of the patient being treated and the severity of the disease or condition being treated. Said anti-CSF-1R antibody and further agent are suitably co-administered to the patient at one time or over a series of treatments e.g. on the same day or on the day after.

Depending on the type and severity of the disease, about 0.1 mg /kg to 50 mg/kg (e.g. 0.1-20 mg/kg) of said anti-CSF-1R antibody; is an initial candidate dosage for co-administration of both drugs to the patient The invention comprises the use of the antibodies according to the invention for the treatment of a patient suffering from cancer, especially from colon, lung or pancreas cancer.

The invention comprises also a method for the treatment of a patient suffering from such disease.

The invention further provides a method for the manufacture of a pharmaceutical composition comprising an effective amount of an antibody according to the invention together with a pharmaceutically acceptable carrier and the use of the antibody according to the invention for such a method.

The invention further provides the use of an antibody according to the invention in an effective amount for the manufacture of a pharmaceutical agent, preferably together with a pharmaceutically acceptable carrier, for the treatment of a patient suffering from cancer.

The invention also provides the use of an antibody according to the invention in an effective amount for the manufacture of a pharmaceutical agent, preferably together with a pharmaceutically acceptable carrier, for the treatment of a patient suffering from cancer.

The following examples, sequence listing and figures are provided to aid the understanding of the present invention, the true scope of which is set forth in the appended claims..

### Description of the Sequences

SEQ ID NO: 1 heavy chain CDR3, Mab 2F11
SEQ ID NO: 2 heavy chain CDR2, Mab 2F11
SEQ ID NO: 3 heavy chain CDR1, Mab 2F11
SEQ ID NO: 4 light chain CDR3, Mab 2F11
SEQ ID NO: 5 light chain CDR2, Mab 2F11
SEQ ID NO: 6 light chain CDR1, Mab 2F11
SEQ ID NO: 7 heavy chain variable domain, Mab 2F11
SEQ ID NO: 8 light chain variable domain, Mab 2F11
SEQ ID NO: 9 heavy chain CDR3, Mab 2E10
SEQ ID NO: 10 heavy chain CDR2, Mab 2E10
SEQ ID NO: 11 heavy chain CDR1, Mab 2E10
SEQ ID NO: 12 light chain CDR3, Mab 2E10
SEQ ID NO: 13 light chain CDR2, Mab 2E10
SEQ ID NO: 14 light chain CDR1, Mab 2E10
SEQ ID NO: 15 heavy chain variable domain, Mab 2E10
SEQ ID NO: 16 light chain variable domain, Mab 2E10
SEQ ID NO: 17 heavy chain CDR3, hMab 2F11-c11
SEQ ID NO: 18 heavy chain CDR2, hMab 2F11-c11
SEQ ID NO: 19 heavy chain CDR1, hMab 2F11-c11
SEQ ID NO: 20 light chain CDR3, hMab 2F11-c11
SEQ ID NO: 21 light chain CDR2, hMab 2F11-c11
SEQ ID NO: 22 light chain CDR1, hMab 2F11-cl 1
SEQ ID NO: 23 heavy chain variable domain, hMab 2F11-c11
SEQ ID NO: 24 light chain variable domain, hMab 2F11-c11
SEQ ID NO: 25 heavy chain CDR3, hMab 2F11-d8
SEQ ID NO: 26 heavy chain CDR2, hMab 2F11-d8
SEQ ID NO: 27 heavy chain CDR1, hMab 2F11-d8
SEQ ID NO: 28 light chain CDR3, hMab 2F11-d8
SEQ ID NO: 29 light chain CDR2, hMab 2F11-d8
SEQ ID NO: 30 light chain CDR1, hMab 2F11-d8
SEQ ID NO: 31 heavy chain variable domain, hMab 2F11-d8
SEQ ID NO: 32 light chain variable domain, hMab 2F11-d8
SEQ ID NO: 33 heavy chain CDR3, hMab 2F11-e7
SEQ ID NO: 34 heavy chain CDR2, hMab 2F11-e7
SEQ ID NO: 35 heavy chain CDR1, hMab 2F11-e7
SEQ ID NO: 36 light chain CDR3, hMab 2F11-e7
SEQ ID NO: 37 light chain CDR2, hMab 2F11-e7
SEQ ID NO: 38 light chain CDR1, hMab 2F11-e7
SEQ ID NO: 39 heavy chain variable domain, hMab 2F11-e7
SEQ ID NO: 40 light chain variable domain, hMab 2F11-e7
SEQ ID NO: 41 heavy chain CDR3, hMab 2F11-f12
SEQ ID NO: 42 heavy chain CDR2, hMab 2F11-f12
SEQ ID NO: 43 heavy chain CDR1, hMab 2F11-f12
SEQ ID NO: 44 light chain CDR3, hMab 2F11-f12
SEQ ID NO: 45 light chain CDR2, hMab 2F11-f12
SEQ ID NO: 46 light chain CDR1, hMab 2F11-f12
SEQ ID NO: 47 heavy chain variable domain, hMab 2F11-f12
SEQ ID NO: 48 light chain variable domain, hMab 2F11-f12
SEQ ID NO: 49 heavy chain CDR3, hMab 2F11-g1
SEQ ID NO: 50 heavy chain CDR2, hMab 2F11-g1
SEQ ID NO: 51 heavy chain CDR1, hMab 2F11-g1
SEQ ID NO: 52 light chain CDR3, hMab 2F11-g1
SEQ ID NO: 53 light chain CDR2, hMab 2F11-g1
SEQ ID NO: 54 light chain CDR1, hMab 2F11-g1
SEQ ID NO: 55 heavy chain variable domain, hMab 2F11-g1
SEQ ID NO: 56 light chain variable domain, hMab 2F11-g1
SEQ ID NO: 57 human kappa light chain constant region
SEQ ID NO: 58 human heavy chain constant region derived from IgG1
SEQ ID NO: 59 human heavy chain constant region derived from IgG1 mutated on L234A and L235A
SEQ ID NO: 60 human heavy chain constant region derived from IgG4
SEQ ID NO: 61 human heavy chain constant region derived from IgG4 mutated on S228P
SEQ ID NO: 62 human wildtype CSF-1R (wt CSF-1R)
SEQ ID NO: 63 human mutant CSF-1R L301S Y969F
SEQ ID NO: 64 human CSF-1R Extracellular Domain (domains D1-D5)
SEQ ID NO: 65 human CSF-1R fragment delD4
SEQ ID NO: 66 human CSF-1R fragment domains D1-D3
SEQ ID NO: 67 signal peptide
SEQ ID NO: 68 Primer
SEQ ID NO: 69 heavy chain CDR3, Mab 1G10
SEQ ID NO: 70 heavy chain CDR2, Mab 1G10
SEQ ID NO: 71 heavy chain CDR1, Mab 1G10
SEQ ID NO: 72 light chain CDR3, Mab 1G10
SEQ ID NO: 73 light chain CDR2, Mab 1G10
SEQ ID NO: 74 light chain CDR1, Mab 1G10
SEQ ID NO: 75 heavy chain variable domain, Mab 1G10
SEQ ID NO: 76 light chain variable domain, Mab 1G10
SEQ ID NO: 77 heavy chain CDR3, Mab 2H7
SEQ ID NO: 78 heavy chain CDR2, Mab 2H7
SEQ ID NO: 79 heavy chain CDR1, Mab 2H7
SEQ ID NO: 80 light chain CDR3, Mab 2H7
SEQ ID NO: 81 light chain CDR2, Mab 2H7
SEQ ID NO: 82 light chain CDR1, Mab 2H7
SEQ ID NO: 83 heavy chain variable domain, Mab 2H7
SEQ ID NO: 84 light chain variable domain, Mab 2H7
SEQ ID NO: 85 human CSF-1R fragment domains D4-D5
SEQ ID NO: 86 human CSF-1
SEQ ID NO: 87 human IL-34
SEQ ID NO: 88 heavy chain variable domain of CP-870,893 (antibody 21.4.1 of U.S.7,338,660)
SEQ ID NO: 89 light chain variable domain of CP-870,893 (antibody 21.4.1 of U.S.7,338,660)
SEQ ID NO: 90 humanized S2C6 heavy chain variabel domain variant
SEQ ID NO: 91 humanized S2C6 light chain variabel domain variant

### Examples

### Example 1

### Generation of a hybridoma cell line producing anti-CSF-1R antibodies

### Immunization procedure of NMRI mice

NMRI mice were immunized with an expression vector pDisplay™ (Invitrogen, USA) encoding the extracellular domain of huCSF-1R by utilizing electroporation. Every mouse was 4 times immunized with 100µg DNA. When serum titers of anti-huCSF-1R were found to be sufficient, mice were additionally boosted once with 50µg of a 1:1 mixture huCSF-1R ECD/huCSF-1R ECDhuFc chimera in 200 µl PBS intravenously (i.v.) 4 and 3 days before fusion.

### Antigen specific ELISA

### Anti-CSF-1R titers in sera of immunized mice were determined by antigen specific ELISA.

0.3 µg/ml huCSF-1R-huFc chimera (soluble extracellular domain) was captured on a streptavidin plate (MaxiSorb; MicroCoat, DE, Cat.No. 11974998/MC1099) with 0.1 mg/ml biotinylated anti Fcγ (Jackson ImmunoResearch., Cat.No. 109-066-098) and horse radish peroxidase (HRP)-conjugated F(ab')₂ anti-mouse IgG (GE Healthcare, UK, Cat.No.NA9310V) diluted 1/800 in PBS/0.05% Tween20/0.5% BSA was added. Sera from all taps were diluted 1/40 in PBS/0.05% Tween20/0.5% BSA and serially diluted up to 1/1638400. Diluted sera were added to the wells. Pre-tap serum was used as negative control. A dilution series of mouse anti-human CSF-1R Mab3291 (R&D Systems, UK) from 500 ng/ml to 0,25 ng/ml was used as positive control. All components were incubated together for 1,5 hours, Wells were washed 6 times with PBST (PBS/0.2% Tween20) and assays were developed with freshly prepared ABTS® solution (1 mg/ml) (ABTS: 2,2'-azino bis (3-ethylbenzthiazoline-6-sulfonic acid) for 10 minutes at RT. Absorbance was measured at 405 nm.

### Hybridoma generation

The mouse lymphocytes can be isolated and fused with a mouse myeloma cell line using PEG based standard protocols to generate hybridomas. The resulting hybridomas are then screened for the production of antigen-specific antibodies. For example, single cell suspensions of splenic derived lymphocytes from immunized mice are fused to Ag8 non-secreting mouse myeloma cells P3X63Ag8.653 (ATCC, CRL-1580) with 50% PEG. Cells are plated at approximately 10⁴ in flat bottom 96 well micro titer plate, followed by about two weeks incubation in selective medium. Individual wells are then screened by ELISA for human anti-CSF-1R monoclonal IgM and IgG antibodies. Once extensive hybridoma growth occurs, the antibody secreting hybridomas are replated, screened again, and if still positive for human IgG, anti-CSF-1R monoclonal antibodies, can be subcloned by FACS. The stable subclones are then cultured in vitro to produce antibody in tissue culture medium for characterization. Antibodies according to the invention could be selected using the determination of the binding of anti-CSF-1R antibodies to human CSF-1R fragment delD4 and to human CSF-1R Extracellular Domain (CSF-1R-ECD) as described in Example 4, as well as the determination of growth inhibition of NIH3T3 cells transfected with wildtype CSF-1R (ligand dependent signalling) or mutant CSF-1R L301S Y969F (ligand independent signalling) under treatment with anti-CSF-1R monoclonal antibodies as described in Example 5.

### Culture of hybridomas

Generated muMAb hybridomas were cultured in RPMI 1640 (PAN - Catalogue No. (Cat. No.) PO4-17500) supplemented with 2 mM L-glutamine (GIBCO - Cat. No.35050-038), 1 mM Na-Pyruvat (GIBCO - Cat. No.11360-039), 1x NEAA (GIBCO - Cat. No. 11140-035), 10% FCS (PAA - Cat. No.A15-649), 1x Pen Strep (Roche - Cat. No.1074440), 1x Nutridoma CS (Roche - Cat. No.1363743), 50 µM Mercaptoethanol (GIBCO - Cat. No.31350-010) and 50 U/ml IL 6 mouse (Roche - Cat. No.1 444 581) at 37°C and 5% CO₂. Some of the resulting mouse antibodies have been humanized (e.g. Mab 2F11) and been expressed recombinantly.

### Example 2

### Inhibition of CSF-1 binding to CSF-1R (ELISA)

By setting-up this assay to first allow for anti-CSF-1R antibody binding to the CSF-1R-ECD followed by detection of ligand not bound to the receptor both-ligand displacing antibodies and dimerization inhibitor anti-CSF-1R antibodies - can be tested. The test was performed on 384 well microtiter plates (MicroCoat, DE, Cat.No. 464718) at RT. After each incubation step plates were washed 3 times with PBST.

At the beginning, plates were coated with 0.5 mg/ml goat F(ab')2 biotinylated anti Fcγ (Jackson ImmunoResearch., Cat.No.109-006-170) for 1 hour (h).

Thereafter the wells were blocked with PBS supplemented with 0.2% Tween®-20 and 2% BSA (Roche Diagnostics GmbH, DE) for 0.5 h. 75 ng/ml of huCSF-1R-huFc chimera (which forms the dimeric soluble extracellular domain of huCSF-1R) was immobilized to plate for 1 h. Then dilutions of purified antibodies in PBS/0.05% Tween20/0.5% BSA were incubated for 1 h. After adding a mixture of 3 ng/ml hu CSF-1 (active 149 aa fragment of human CSF-1 (aa 33-181 of SEQ ID NO: 86) ;Biomol, DE, Cat.No.60530), 50ng/ml biotinylated anti CSF-1 clone BAF216 (R&D Systems,UK) and 1:5000 diluted streptavidin HRP (Roche Diagnostics GmbH, DE, Cat.No.11089153001) for 1 h the plates were washed 6 times with PBST. Anti CSF-1R SC 2-4A5 (Santa Cruz Biotechnology, US), which inhibits the ligand- receptor interaction, was used as positive control. Plates were developed with freshly prepared BM blue® POD substrate solution (BM blue®: 3,3'-5,5'-Tetramethylbenzidine, Roche Diagnostics GmbH, DE, Cat.No. 11484281001) for 30 minutes at RT. Absorbance was measured at 370 nm.A decrease of absorbance is found, if the anti-CSF-1R antibody causes a release of CSF-1 from the dimeric complex. All anti-CSF-1R antibodies showed significant inhibition of the CSF-1 interaction with CSF-1R (see Table 1). Anti CSF-1R SC 2-4A5 (Santa Cruz Biotechnology, US see also Sherr, C.J. et al., Blood 73 (1989) 1786-1793), which inhibits the ligand- receptor interaction, was used as reference control.

**Table 1:**

| **Calculated IC50 values for the inhibition of the CSF-1/CSF-1R interaction** | |
|---|---|
| **CSF-1R Mab** | **IC50 CSF-1/CSF-1R Inhibition [ng/ml]** |
| Mab 2F11 | 19.3 |
| Mab 2E10 | 20.6 |
| Mab 2H7 | 18.2 |
| Mab 1G10 | 11.8 |
| SC-2-4A5 | 35.2 |

### Example 3

### Inhibition of CSF-1-induced CSF-1R phosphorylation in NIH3T3-CSF-1R recombinant cells

4.5x10³ NIH 3T3 cells, retrovirally infected with an expression vector for full-length CSF-1R, were cultured in DMEM (PAA Cat. No.E15-011), 2mM L-glutamine (Sigma, Cat.No.G7513, 2mM Sodium pyruvate, 1x nonessential aminoacids, 10% FKS (PAA, Cat.No.A15-649) and 100µg/ml PenStrep (Sigma, Cat.No. P4333 [10mg/ml]) until they reached confluency. Thereafter cells were washed with serum-free DMEM media (PAA Cat.No.E15-011) supplemented with sodium selenite [5ng/ml] (Sigma, Cat.No. S9133), transferrin [10µg/ml] (Sigma, Cat.No. T8158), BSA [400µg/ml] (Roche Diagnostics GmbH, Cat.No. 10735078), 4mM L-glutamine (Sigma, Cat.No.G7513), 2mM sodium pyruvate (Gibco, Cat.No. 11360), 1x nonessential aminoacids (Gibco, Cat: 11140-035), 2-mercaptoethanol [0,05mM] (Merck, Cat.No. M7522), 100µg/ml and PenStrep (Sigma, Cat. No. P4333) and incubated in 30 µl of the same medium for 16 hours to allow for receptor up-regulation. 10 µl of diluted anti-CSR-1R antibodies were added to the cells for 1.5 h. Then cells were stimulated with 10 µl of 100 ng/ml hu CSF-1 (active 149 aa fragment of human CSF-1 (aa 33-181 of SEQ ID NO: 86) ;Biomol, DE, Cat.No.60530)for 5 min. After the incubation, supernatant was removed, cells were washed twice with 80 µl of ice-cold PBS and 50 µl of freshly prepared ice-cold lysis buffer (150mM NaCl/ 20mM Tris pH 7.5 / 1mM EDTA/ 1mM EGTA/ 1% Triton X-100 /1 protease inhibitor tablet (Roche Diagnostics GmbH Cat.No.1 836 170) per 10 ml buffer/10µl/ml phosphatase inhibitor cocktail 1 (Sigma Cat.No. P-2850, 100x Stock)_{/} 10µl/ml protease inhibitor 1 (Sigma Cat.No.P-5726, 100x Stock) /10µl/ml 1 M NaF) was added. After 30 minutes on ice the plates were shaken vigourously on a plateshaker for 3 minutes and then centrifuged 10 minutes at 2200 rpm (Heraeus Megafuge 10).

The presence of phosphorylated and total CSF-1 receptor in the cell lysate was analyzed with Elisa. For detection of the phosphorylated receptor the kit from R&D Systems (Cat. No. DYC3268-2) was used according to the instructions of the supplier. For detection of total CSF-1R 10 µl of the lysate was immobilized on plate by use of the capture antibody contained in the kit. Thereafter 1:750 diluted biotinylated anti CSF-1R antibody BAF329 (R&D Systems) and 1:1000 diluted streptavidin-HRP conjugate was added. After 60 minutes plates were developed with freshly prepared ABTS® solution and the absorbance was detected. Data were calculated as % of positive control without antibody and the ratio value phospho/total receptor expressed. The negative control was defined without addition of M-CSF-1. Anti CSF-1R SC 2-4A5 (Santa Cruz Biotechnology, US, see also Sherr, C.J. et al., Blood 73 (1989) 1786-1793), which inhibits the ligand-receptor interaction, was used as reference control.

**Table 2:**

| **Calculated IC50 values for the inhibition of CSF-1 receptor phosphorylation.** | |
|---|---|
| **CSF-1R Mab** | **IC50 CSF-1R Phosphorylation [ng/ml]** |
| Mab 2F11 | 219.4 |
| Mab 2E10 | 752.0 |
| Mab 2H7 | 703.4 |
| Mab 1G10 | 56.6 |
| SC-2-4A5 | 1006.6 |

### Example 4

### Determination of the binding of anti-CSF-1R antibodies to human CSF-1R fragment delD4 and to human CSF-1R Extracellular Domain (CSF-1R-ECD)

### Preparation of human CSF-1R Extracellular Domain (CSF-1R-ECD) (comprising the extracellular subdomains D1 -D5, hCSF-1R-ECD) of SEQ ID NO: 64:

pCMV-preS-Fc-hCSF-1R-ECD (7836bp) encodes the complete ECD of human CSF-1R (SEQ ID NO: 64) C-terminally fused to a PreScission protease cleavage site, followed by aa100-330 of human IgG1 and a 6xHis-Tag, under the control of CMV promoter. The natural signal peptide has been varied by insertion of amino acids G and S after the first M, in order to create a BamHI restriction site.

### Preparation of human CSF-1R fragment delD4 (comprising the extracellular subdomains D1 -D3 and D5, hCSF-1R-delD4) of SEQ ID NO: 65:

hCSF1R-delD4-V1-PreSc-hFc-His was cloned from pCMV-preS-Fc-hCSF-1R-ECD by means of the Stratagene QuikChange XL site-directed mutagenesis protocol, using delD4-for with sequence CACCTCCATGTTCTTCCGGTACCCCCCAGAGGTAAG (SEQ ID NO: 68) as the forward primer and delD4-rev with the reverse complement sequence as the reverse primer. A protocol variation published in BioTechniques 26 (1999) 680 was used to extend both primers in separate reactions in three cycles preceeding the regular Stratagene protocol:
Two separate 50 µl reaction mixtures were set up according to the manufacturer's manual, each containing 10 ng plasmid pCMV-preS-Fc-hCSF1R-ECD as the template and 10 pM of one of the primers delD4-for or delD4-rev, and 0,5 µl Pfu DNA polymerase as provided with the kit. Three PCR cycles 95 °C 30 sec / 55 °C 60 sec / 68 °C 8 min were run, then 25 µl each of both reaction mixtures were combined in a new tube and 0,5 µl fresh Pfu DNA polymerase were added. The regular PCR protocol with 18 temperature cycles as specified by Stratagene in the kit manual was carried out, followed by 2 hrs final digestion with the Dpn1 restriction enzyme provided with the kit. Clones bearing the deletion were detected by digestion with Cel II and Not I and verified by sequencing.

Protein was prepared by transient transfection in the Hek293 FreeStyle suspension cell system (Invitrogen) according to the manufacturer's specifications. After 1 week 500 ml supernatant was filtered and loaded onto a 1ml HiTrap MabSelect Xtra (GE healthcare) protein A column (0,2 ml /min). The column was washed first with PBS, then with 50 mM Tris/ 150 mM NaCl/ 1 mM EDTA/ pH 7,3. 75 µl PreScission Protease (GE #27-0843-01) diluted in 375 µl of the same buffer were loaded onto the column and the closed column was incubated over night at 4 °C with rolling. The column was mounted on top of a 1 ml GSTrap FF column (GE helthcare) and the desired protein was eluted (0,2 ml/min, 0,2 ml fractions). Pooled fractions were concentrated from 1,8 ml to 0,4 ml by centrifugal ultrafiltration via a 3k Nanosep and chromatographed over an S200 HR SEC in PBS (0,5 ml/min).

Human CSF-1R fragment delD4 was obtained in two fractions as a dimeric molecule (pool1, V=1,5 ml; c= 0,30 mg/ml; apparent mass on SDS page 83 kDa, reduced 62 kDa) and as the monomer (pool 2, V=1,4 ml; c = 0,25 mg/ml apparent mass on SDS page 62 kDa). The dimeric form was used for all experiments.

### Determination of the binding of anti-CSF-1R antibodies to human CSF-1R fragment delD4 and to human CSF-1R Extracellular Domain (CSF-1R-ECD) (binding signals as Response Units (RU):

| | | |
|---|---|---|
| Instrument: | Biacore T100 (GE Healthcare) | |
| | Software: | T100 Control, Version 2.0.1 |
| | | T100 Evaluation, Version 2.0.2 |
| Assayformat | Chip: CM5 | |
| Temperature: | 25°C | |

CSF-1R fragments were immobilized via amine coupling. To compare the binding of different anti-CSF-1R antibodies according to the invention one concentration of the test antibody was injected. Anti CSF-1R Mab3291 (R&D-Systems) and SC 2-4A5 (Santa Cruz Biotechnology, US- see also Sherr, C.J. et al., Blood 73 (1989) 1786-1793), was used as reference control, anti-CCR5 m<CCR5>Pz03.1C5 (deposited as DSM ACC 2683 on 18.08.2004 at DSMZ) as negative control, all under the same conditions as the anti-CSF-1R antibodies according to the invention.

### Amine coupling of CSF-1R fragments

Standard amine coupling according to the manufacturer's instructions: running buffer: PBS-T (Roche: 11 666 789 + 0.05% Tween20: 11 332 465), activation by mixture of EDC/NHS, injection of human CSF-1R fragment delD4 (comprising the extracellular subdomains D1 -D3 and D5) (SEQ ID NO: 65) and human CSF-1R Extracellular Domain (CSF-1R-ECD) (comprising the extracellular subdomains D1 -D5) (SEQ ID NO: 64) for 600 seconds at flow rate 10µl/min; diluted in coupling buffer NaAc, pH 5.0, c = 10 µg/mL; finally remaining activated carboxyl groups were blocked by injection of 1 M Ethanolamin.

### Binding of <CSF-1R> Mab 2F11, Mab 2E10, Mab 3291 and sc2-4A5 and other anti-CSF-1R antibodies to human CSF-1R fragment delD4 and human CSF-1R Extracellular Domain (CSF-1R-ECD) at 25°C

Running buffer: PBS-T (Roche: 11 666 789 + 0.05% Tween20: 11 332 465)

### Analyte sample:

Binding was measured at a flow rate of 30 µL/min by one injection of the analyte with concentration c = 10 nM. (for Mab 1G10, Mab 2H7 and humanized hMab 2F11-e7 in second experiment) Each injection was 700 seconds long, followed by a dissociation phase of 180 seconds. Final regeneration was performed after each cycle using 50 mM NaOH, contact time 60 seconds, flow rate 30 µL/min.

Signals were measured by a report point 10 seconds after end of injection. Reference signals (signals from a blank reference flow cell (treated with EDC/NHS and ethanolamine, only) were subtracted to give the binding signals (as RU). If binding signals of nonbinding antibodies were slightly below 0 (Mab 2F11 =-3; Mab 2E10 = -2; Mab 1G10 = - 6, Mab 2H7 =-9; and humanized hMab 2F11-e7 = - 7) the values were set as 0.

**Table 3a:**

| **Binding of <CSF-1R> MAbs to human CSF-1R fragment delD4 and CSF-1R-ECD and ratio at 25°C, measured by SPR** | | | |
|---|---|---|---|
| | Bindingto delD4 [RU] | Bindingto CSF-1R-ECD [RU] | Ratioof binding of anti-CSF1Rantibodiesto CSF1R fragmentdelD4/to CSF-1R-ECD |
| Mab 3291 | 1015 | 627 | 1015/627= 1.61 |
| sc2-4A5 | 374 | 249 | 374/249= 1.50 |
| Mab 2F11 | 0 | 176 | 0/176 = 0 |
| hMab 2F11-e7 | 0 | 237 | 0/237= 0 |
| Mab 2E10 | 0 | 120 | 0/120 = 0 |
| Mab 1G10 | 0 | 2708 | 0/2708 = 0 |
| Mab 2H7 | 0 | 147 | 0/147 = 0 |
| m<CCR5>Pz03.1C5 | 2 | 5 | - |

Mab 2F11 and Mab 2E10 showed binding to the human CSF-1R Extracellular Domain (CSF-1R-ECD) (see Fig. 2b); however no binding was detected to CSF-1R fragment delD4. (see Fig. 2a).

Sc2-4A5 and MAB3291 showed binding to CSF-1R-ECD and to del D4 (see Fig. 2b and 2a).

Thus the ratio of binding of anti-CSFIR antibodies Mab 2F11 and Mab 2E10 to CSF1R fragment delD4 / to CSF-1R-ECD was clearly below 1:50 (= 0.02), while the binding ratio of MAB3291 and Sc2-4A5 were 1.61 and 1.50, respectively and were highly above 1:50 (= 0.02). Negative control antibody m<CCR5>Pz03.1C5 did not show any binding (as expected).

Mab 1G10, Mab 2H7 and humanized hMab 2F11-e7 showed binding to the human CSF-1R Extracellular Domain (CSF-1R-ECD) (see Fig. 2d); however no binding was detected to CSF-1R fragment delD4. (see Fig. 2c). Thus the ratio of binding of anti-CSFIR antibodies Mab 1G10, Mab 2H7 and humanized hMab 2F11-e7 to CSF1R fragment delD4 / to CSF-1R-ECD was clearly below 1:50 (= 0.02).

In a further experiment anti-CSF-1R antibodies 1.2.SM (ligand displacing CSF-1R antibody described in WO2009026303), CXIIG6 (ligand displacing CSF-1R antibody described in WO 2009/112245), the goat polyclonal anti-CSF-1R antibody ab10676 (abcam) were investigated. Anti-CSF-1R antibody Mab3291 (R&D-Systems) was used as reference control. Anti-CCR5 m<CCR5>Pz03.1C5 (deposited as DSM ACC 2683 on 18.08.2004 at DSMZ) was used as negative control.

**Table 3b:**

| **Binding of <CSF-1R> MAbs to human CSF-1R fragment delD4 and CSF-1R-ECD and ratio at 25°C, measured by SPR** | | | |
|---|---|---|---|
| | Binding to delD4 [RU] | Bindingto CSF-1R-ECD [RU] | Ratio of binding of anti-CSF1Rantibodiesto CSF1R fragment delD4 /to CSF-1R-ECD |
| MAB3291 | 1790 | 1222 | 1790/1222= 1.47 |
| 1.2.SM | 469 | 704 | 469/704 = 0.67 |
| CXIIG6 | 1983 | 1356 | 1983/1356= 1.46 |
| ab10676 | 787 | 547 | 787/547= 1.44 |
| m<CCR5>Pz03.1C5 | 0 | 0 | - |

1.2.SM, CXIIG6, ab10676 and MAB3291 showed binding to CSF-1R-ECD and to del D4 (see Fig. 2f and 2e).

The binding ratio of 1.2.SM, CXIIG6, ab10676 and MAB3291 was highly above 1:50 (= 0.02). Negative control antibody m<CCR5>Pz03.1C5 did not show any binding (as expected).

### Example 5

### Growth inhibition of NIH3T3-CSF-1R recombinant cells in 3D culture under treatment with anti-CSF-1R monoclonal antibodies (CellTiterGlo-assay)

NIH 3T3 cells, retrovirally infected with either an expression vector for full-length wildtype CSF-1R (SEQ ID NO: 62) or mutant CSF-1R L301S Y969F (SEQ ID NO: 63), were cultured in DMEM high glucose media (PAA, Pasching, Austria) supplemented with 2mM L-glutamine, 2mM sodium pyruvate and non-essential amino acids and 10% fetal bovine serum (Sigma, Taufkirchen, Germany) on poly-HEMA (poly(2-hydroxyethylmethacrylate)) (Polysciences, Warrington, PA, USA)) coated dishes to prevent adherence to the plastic surface. Cells are seeded in medium replacing serum with 5ng/ml sodium selenite, 10mg/ml transferrin, 400µg/ml BSA and 0.05 mM 2-mercaptoethanol. When treated with 100ng/ml hu CSF-1 (active 149 aa fragment of human CSF-1 (aa 33-181 of SEQ ID NO: 86); Biomol, DE, Cat.No.60530) wtCSF-1R (expressing cells form dense spheroids that grow three dimensionally, a property that is called anchorage independence. These spheroids resemble closely the three dimensional architecture and organization of solid tumors in situ. Mutant CSF-1R recombinant cells are able to form spheroids independent of the CSF-1 ligand. Spheroid cultures were incubated for 3 days in the presence of different concentrations of antibody in order to determine an IC50 (concentration with 50 percent inhibition of cell viability). The CellTiterGlo assay was used to detect cell viability by measuring the ATP-content of the cells.

**Table 5a:**

| **CSF-1R Mab** | **wtCSF-1R IC₅₀ [µg/ml]** | **Mutant CSF-1R IC₅₀ [µg/ml]** |
|---|---|---|
| Mab 2F11 | 1.1 | 8.0 |
| Mab 2E10 | 0.49 | 4.9 |
| Mab 2H7 | 0.31 | 5.3 |
| Mab 1G10 | 0.29 | 14.2 |
| SC 2-4A5 | 10.0 | 10.0 |

Reference control Mab R&D-Systems 3291 did not show inhibition of mutant CSF-1R recombinant cell proliferation.

In a further experiment the anti-CSF-1R antibody according to the invention hMab 2F11-e7 and the anti-CSF-1R antibodies 1.2.SM (ligand displacing CSF-1R antibody described in WO 2009/026303), CXIIG6 (ligand displacing CSF-1R antibody described in WO 2009/112245), the goat polyclonal anti-CSF-1R antibody ab10676 (abcam), and SC 2-4A5 (Santa Cruz Biotechnology, US- see also Sherr, C.J. et al., Blood 73 (1989) 1786-1793) were investigated.

Spheroid cultures were incubated for 3 days in the presence of different concentrations of antibody in order to determine an IC30 (concentration with 30 percent inhibition of cell viability). Maximum concentration was 20 µg/ml The CellTiterGlo assay was used to detect cell viability by measuring the ATP-content of the cells.

**Table 5b:**

| **CSF-1R Mab** | **wtCSF-1R IC₃₀ [µg/ml]** | **Mutant CSF-1R IC₃₀ [µg/ml]** |
|---|---|---|
| hMab 2F11-e7 | 4.91 | 0.54 |
| 1.2.SM | 1.19 | > 20 µg/ml (-19% inhibition at 20 µg/ml = 19% stimulation) |
| CXIIG6 | > 20 µg/ml (21% inhibition at 20 µg/ml) | > 20 µg/ml (-36% inhibition at 20 µg/ml = 36% stimulation) |
| ab 10676 | 14.15 | > 20 µg/ml (0% inhibition at 20 µg/ml) |
| SC 2-4A5 | 16.62 | 2.56 |

### Example 6

### Growth inhibition of BeWo tumor cells in 3D culture under treatment with anti-CSF-1R monoclonal antibodies (CellTiterGlo-assay)

BeWo choriocarcinoma cells (ATCC CCL-98) were cultured in F12K media (Sigma, Steinheim, Germany) supplemented with 10% FBS (Sigma) and 2mM L-glutamine. 5x10⁴ cells/well were seeded in 96-well poly-HEMA (poly(2-hydroxyethylmethacrylate)) coated plates containing F12K medium supplemented with 0.5 % FBS and 5% BSA. Concomitantly, 200 ng/ml huCSF-1 (active 149 aa fragment of human CSF-1 (aa 33-181 of SEQ ID NO: 86)) and 10µg/ml of different anti-CSF-1R monoclonal antibodies were added and incubated for 6 days. The CellTiterGlo assay was used to detect cell viability by measuring the ATP-content of the cells in relative light units (RLU). When BeWo spheroid cultures were treated with different anti-CSF-1R antibodies (10 µg/ml) inhibition of CSF-1 induced growth was observed. To calculate antibody-mediated inhibition the mean RLU value of unstimulated BeWo cells was subtracted from all samples. Mean RLU value of CSF-1 stimulated cells was set arbitrarily to 100%. Mean RLU values of cells stimulated with CSF-1 and treated with anti-CSF-1R antibodies were calculated in % of CSF-1 stimulated RLUs. The Table 6 shows the calculated data of growth inhibition of BeWo tumor cells in 3D culture under treatment with anti-CSF-1R monoclonal antibodies; Fig.1a and b depicts normalized mean RLU values.

**Table 6:**

| **CSF-1R Mab** | **%inhibition 10µg/ml antibody concentration** |
|---|---|
| CSF-1 only | 0 |
| Mab 2F11 | 70 |
| Mab 2E10 | 102 |
| Mab 2H7 | 103 |
| Mab 1G10 | 99 |
| SC 2-4A5 | 39 |

### Example 7

### Inhibition of human macrophage differentiation under treatment with anti-CSF-1R monoclonal antibodies (CellTiterGlo-assay)

Human monocytes were isolated from peripheral blood using the RosetteSep™ Human Monocyte Enrichment Cocktail (StemCell Tech. - Cat. No.15028). Enriched monocyte populations were seeded into 96 well microtiterplates (2.5x10⁴ cells/well) in 100 µl RPMI 1640 (Gibco - Cat. No.31870) supplemented with 10% FCS (GIBCO - Cat. No.011-090014M), 4 mM L-glutamine (GIBCO - Cat. No.25030) and 1x PenStrep (Roche Cat. No.1 074 440) at 37°C and 5% CO₂ in a humidified atmosphere. When 150 ng/ml huCSF-1 was added to the medium, a clear differentiation into adherent macrophages could be observed. This differentiation could be inhibited by addition of anti-CSF-1R antibodies. Furthermore, the monocyte survival is affected and could be analyzed by CellTiterGlo (CTG) analysis. From the concentration dependent inhibition of the survival of monocytes by antibody treatment, an IC₅₀ was calculated (see Table 7).

**Table 7:**

| **CSF-1R Mab** | **IC₅₀ [µg/ml]** |
|---|---|
| Mab 2F11 | 0.08 |
| Mab 2E10 | 0.06 |
| Mab 2H7 | 0.03 |
| Mab 1G10 | 0.06 |
| SC 2-4A5 | 0.36 |

In a separate test series humanized versions of Mab 2 F11, e.g. hMab 2F11-c11, hMab 2F11-d8, hMab 2F11-e7, hMab 2F11-f12, showed IC50 values of 0.07 µg/ml (hMab 2F11-c11), 0.07 µg/ml (hMab 2F11-d8), 0.04 µg/ml (hMab 2F11-e7) and 0.09 µg/ml (hMab 2F11-f12).

### Example 8

### Inhibition of cynomolgous macrophage differentiation under treatment with anti-CSF-1R monoclonal antibodies (CellTiterGlo-assay)

Cynomolgous monocytes were isolated from peripheral blood using the CD14 MicroBeads non-human primate kit (Miltenyi Biotec - Cat.No. 130-091-097) according to the manufacturers description. Enriched monocyte populations were seeded into 96 well microtiterplates (1-3x10⁴ cells/well) in 100 µl RPMI 1640 (Gibco - Cat. No.31870) supplemented with 10% FCS (GIBCO - Cat. No.011-090014M), 4 mM L-glutamine (GIBCO - Cat. No.25030) and 1x PenStrep (Roche Cat. No.1 074 440) at 37°C and 5% CO₂ in a humidified atmosphere. When 150 ng/ml huCSF-1 was added to the medium, a clear differentiation into adherent macrophages could be observed. This differentiation could be inhibited by addition of anti-CSF-1R antibodies. Furthermore, the monocyte survival is affected and could be analyzed by CellTiterGlo (CTG) analysis. The viability was analyzed at a concentration of 5 µg/ml antibody treatment (see Table 8).

**Table 8:**

| **CSF-1R Mab** | **% survival** | **% inhibition (of survival) = (100% - %survival)** |
|---|---|---|
| Mab 2F11 | 4 * | 96 |
| Mab 2E10 | 17 ** | 83 |
| Mab 2H7 | 8 | 92 |
| Mab 1G10 | 2 | 98 |
| SC 2-4A5 | 31 | 69 |

| | | |
|---|---|---|
| * mean of four experiments (3 expts. using the murine, 1 expt. using the chimeric mAb) ** mean of two experiments using the murine mAb only | | |

### Example 9

### Inhibition of human M1 and M2 macrophage differentiation under treatment with anti-CSF-1R monoclonal antibodies (CellTiterGlo-assay)

Human monocytes were isolated from peripheral blood using the RosetteSep™ Human Monocyte Enrichment Cocktail (StemCell Tech. - Cat. No.15028). Enriched monocyte populations were seeded into 96 well microtiterplates (2.5x10⁴ cells/well) in 100 µl RPMI 1640 (Gibco - Cat. No.31870) supplemented with 10% FCS (GIBCO - Cat. No.011-090014M), 4 mM L-glutamine (GIBCO - Cat. No.25030) and 1x PenStrep (Roche Cat. No.1 074 440) at 37°C and 5% CO₂ in a humidified atmosphere. When 100 ng/ml huCSF-1 was added for 6 days to the medium, a clear differentiation into adherent, M2 macrophages with elongated morphology could be observed. When 100 ng/ml huGM-CSF was added to the medium for 6 days, a clear differentiation into adherent, M1 macrophages with round morphology could be observed. This differentiation was associated with the expression of certain markers such as CD163 for M2 macrophages and CD80 or high MHC class II for M1 macrophages as assessed by flow cytometry. Cells were washed with PBS and, if adherent, detached using a 5mM EDTA solution in PBS (20min at 37°C). Cells were then well resuspended, washed with staining buffer (5% FCS in PBS) and centrifuged at 300xg for 5min. Pellets were resuspended in 1ml staining buffer and cells counted in a Neubauer chamber. Aproximately 1x10e5 cells were transferred in each FACS tube, centrifuged at 300xg for 5min and resuspended in staining buffer. Fcγ receptors were blocked by incubation with 1µg human IgG/2,5x10e4 cells (JIR Cat.No.009-000-003) in staining buffer for 20 min on ice. Cells were then mixed with 1,5µl antibody/2,5x10e4 cells for CD80 and CD163 detection whereas 5 µl antibody/2,5x10e4 cells for MHC class II detection was used: PE labeled mouse anti human CD163 (BD Bioscience Cat.No.556018), PE labeled mouse anti human CD80 (BD Bioscience Cat.No. 557227) and Alexa 647 labeled mouse anti human MHC class II (Dako-Cat.No. M0775). The Alexa 647 label was conjugated to the antibody by using the Zenon Alexa 647 mouse IgG labeling kit (Invitrogen Cat.No. Z25008) After a 1-hour incubation on ice cells were washed twice with staining buffer, resuspended and measured at a FACS Canto II.

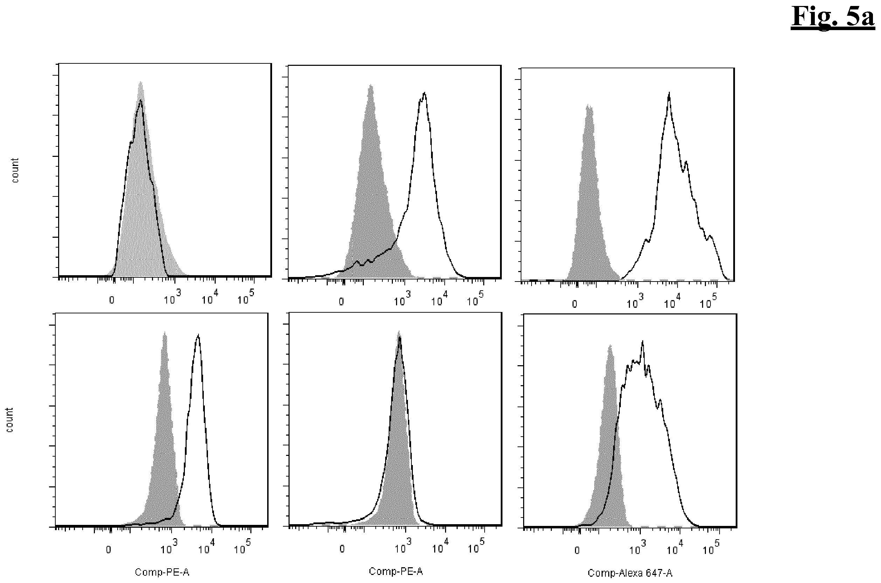

Exclusively M2 macrophage differentiation which is characterized by the expression of CD163, absence of CD80 and low MHC class II expression could be inhibited by addition of humanized anti-CSF-1R antibody hMab 2F11-e7. Furthermore, the M2 but not M1 macrophage survival is affected and could be analyzed by CellTiterGlo (CTG) analysis. Concentration dependent inhibition of the survival of macrophages by antibody treatment for 7 days is depicted in Figure 5a. Expression of M1 and M2 macrophage markers assessed by flow cytometry is shown in Figure 5b.

### Example 10

### Determination of the binding affinity of anti-CSF-1R antibodies to human CSF-1R

| | |
|---|---|
| Instrument: | BIACORE® A100 |
| Chip: | CM5 (Biacore BR-1006-68) |
| Coupling: | amine coupling |
| Buffer: | PBS (Biacore BR-1006-72), pH 7.4, 35°C |

For affinity measurements 36 µg/ml anti mouse Fcγ antibodies (from goat, Jackson Immuno Research JIR115-005-071) have been coupled to the chip surface for capturing the antibodies against CSF-1R. Human CSF-1R Extracellular Domain (CSF-1R-ECD) (comprising the extracellular subdomains D1 -D5) (SEQ ID NO: 64) (R&D-Systems 329-MR or subcloned pCMV-presS-HisAvitag-hCSF-1R-ECD) was added in various concentrations in solution. Association was measured by an CSF-1R-injection of 1.5 minutes at 35 °C; dissociation was measured by washing the chip surface with buffer for 10 minutes at 35 °C. For calculation of kinetic parameters the Langmuir 1:1 model was used.

**Table 9:**

| **Affinity data measured by SPR** | | | | |
|---|---|---|---|---|
| **CSF-1R Mab** | **K_{D} (nM)** | **kₐ (1/Ms)** | **k_{d} (1/s)** | **t_{1/2} (min)** |
| Mab 2F11 | 0.29 | 1.77E⁺⁰⁵ | 5.18E⁻⁰⁵ | 223 |
| Mab 2E10 | 0.2 | 1.52E⁺⁰⁵ | 2.97E⁻⁰⁵ | 389 |
| Mab 2H7 | 0.21 | 1.47E⁺⁰⁵ | 3.12E⁻⁰⁵ | 370 |
| Mab 1G10 | 0.36 | 1.75E⁺⁰⁵ | 6.28E⁻⁰⁵ | 184 |

In a separate biacore binding assay using the CSF-1R ECD (data not shown) some competition of the antibodies Mab 2F11 and Mab 2E10 with the antibody Ab SC-2-4A5 was shown. However Mab 2F11/Mab 2E10 do not bind to the human CSF-1R fragment delD4, whereas Ab SC-2-4A5 binds to this delD4 fragment (see Example 4 and Fig 2a). Thus the binding region of Mab 2F11/Mab 2E10 is clearly distinct from the binding region of Ab SC-2-4A5, but probably located in a vicinity area. In such competition assay both antibodies Mab 2F11 and Mab 2E10 did not compete with Mab3291 from R&D-Systems (data not shown).

### Example 11

### Determination of the binding of anti-CSF-1R antibodies to human CSF-1R fragment D1-D3

| | | |
|---|---|---|
| Instrument: | Biacore T100 (GE Healthcare) | |
| | Software: | T100 Control, Version 1.1.11 |
| | | B3000 Evaluation, Version 4.01 |
| | | Scrubber, Version 2.0a |
| Assayformat | Chip:CM5-Chip | |

Antibodies against CSF-1R were captured via amine coupled capture molecules. Using the single cycle kinetics five increasing concentrations of human CSF-1R fragment D1-D3 (SEQ ID NO: 66) were injected. Human CSF-1R fragment D1-D3 was subcloned into pCMV-presS-HisAvitag expression vector.

Anti CSF-1R SC 2-4A5 (Santa Cruz Biotechnology, US; Sherr, C.J. et al., Blood 73 (1989) 1786-1793) which inhibits the ligand-receptor interaction, and Mab 3291 (R&D-Systems) were used as reference controls.

Capture molecules: Anti mouse Fcγ antibodies (from goat, Jackson Immuno Reasearch JIR115-005-071) for antibodies according to the invention and the R&D-Systems control Mab 3291 and Anti rat Fcγ antibodies (from goat, Jackson Immuno Reasearch JIR112-005-071) for the reference control anti CSF-1R SC 2-4A5.

### Amine coupling of capture molecules

Standard amine coupling according to the manufacturer's instructions: running buffer: HBS-N buffer, activation by mixture of EDC/NHS, aim for ligand density of 2000 RU; the capture-Abs were diluted in coupling buffer NaAc, pH 4.5, c = 10 µg/mL; finally remaining activated carboxyl groups were blocked by injection of 1 M Ethanolamin.

### Kinetic characterization of human CSF-1R fragments D1-D3 binding to MAbs <CSF-1R> at 37°C

Running buffer: PBS (Biacore BR-1006-72)

Capturing of Mabs <CSF-1R> on flow cells 2 to 4: Flow 20 µL/min, contact time 90 seconds, c(Abs<CSF-1R>) = 50 nM, diluted with running buffer + 1 mg/mL BSA;

### Analyte sample:

Single Cycle Kinetics was measured at a flow rate of 30 µL/min by five consecutive injections of the analyte with concentrations, c = 7.8 , 31.25, 125 500 and 2000 nM, without regeneration. Each injection was 30 seconds long and followed by a dissociation phase of 120 Seconds for the first four injections, and finally 1200 seconds for the highest concentration (=last injection).

Final regeneration was performed after each cycle using 10 mM Glycin pH 1.5 (Biacore BR-1003-54), contact time 60 seconds, flow rate 30 µL/min.

Kinetic parameters were calculated by using the usual double referencing (control reference: binding of analyte to capture molecule; Flow Cell: subdomain CSF-1R concentration "0" as Blank) and calculation with model 'titration kinetics 1:1 binding with draft'.

**Table 10:**

| **Affinity data for binding of human CSF-1R fragment D1-D3 measured by SPR** | | | | | |
|---|---|---|---|---|---|
| **CSF-1R Mab** | **Sub domain** | **K_{D} (nM)** | **kₐ (1/Ms)** | **k_{d} (1/s)** | **t_{1/2} (min)** |
| Mab 2F11 | D1-D3 | no binding | | | |
| Mab 2E10 | D1-D3 | no binding | | | |
| Mab 2H7 | D1-D3 | not determined | | | |
| Mab 1G10 | D1-D3 | no binding | | | |
| SC-2-4A5 | D1-D3 | no binding | | | |
| R&D-Systems 3291 | D1-D3 | 5.4 | 2.2E⁺⁵ | 1.2E⁻³ | 9.6 |

The antibodies Mab 2F11, Mab 2E10 and Mab 1G10 showed no binding to human CSF-1R fragment D1-D3.

Also reference control-Ab SC-2-4A5 did not bind to human CSF-1R fragment D1-D3.

The reference control Mab R&D-Systems 3291 showed binding to the human CSF-1R fragment D1-D3.

### Example 12

### CSF-1 level increase during CSF-1R inhibition in Cynomolgus monkey

Serum CSF-1 levels provide a pharmacodynamic marker of CSF-1R neutralizing activity of anti-human CSF-1R dimerization inhibitor hMab 2F11-e7. One male and one female cynomolgus monkey per dosage group (1 and 10 mg/kg) were intravenously administered anti-CSFIR antibody hMab 2F11-e7. Blood samples for analysis of CSF-1 levels were collected 1 week before treatment (pre-dose), 2, 24, 48, 72, 96, 168 hours post-dose and weekly for two additional weeks. CSF-1 levels were determined using a commercially available ELISA kit (Quantikine® human M-CSF) according to the manufacturer's instructions (R&D Systems, UK). Monkey CSF-1 level were determined by comparison with CSF-1 standard curve samples provided in the kit.

Administration of hMab 2F11-e7 induced a dramatic increase in CSF-1 by ∼ 1000-fold, which depending on the dose administered lasted for 48 hr (1mg/kg) or 15 days (10mg/kg). Hence, a dimerization inhibitor for CSF-1R offers the advantage to not directly compete with the dramatically upregulated ligand for binding to the receptor in contrast to a ligand displacing antibody.

### Example 13

### In vivo efficacy - tumor growth inhibition of anti-CSF-1R antibodies in breast cancer BT20 xenograft tumor cells in SCID beige mice

The human breast cancer cell line BT-20 expresses human CSF-1R but lacks CSF-1 expression (Sapi, E. et al Cancer Res 59 (1999) 5578-5585). Since the mouse derived CSF-1 fails to activate human CSF-1R on the tumor cells recombinant human CSF-1 (active 149 aa fragment of human CSF-1 (aa 33-181 of SEQ ID NO: 86) (Biomol, Hamburg, Germany) was supplemented via osmotic minipumps (ALZET, Cupertino, CA) providing a continuous CSF-1 infusion rate of 2µg/day (Martin, T.A., Carcinogenesis 24 (2003) 1317-1323).

To directly compare the efficacy of an antibody interfering with dimerization of CSF-1R with a ligand displacing CSF-1R antibody we tested the chimeric anti-CSF-1R Mab 2F11 (antibody interfering with dimerization of CSF-1R) and 1.2.SM (ligand displacing CSF-1R antibody described in WO 2009/026303) in the BT-20 xenograft model.

SCID beige mice (Charles River, Sulzfeld, Germany) were subcutaneously coinjected with 1x 107 cells BT-20 cells (ATCC HTB-19) and 100µl of Matrigel. Treatment of animals started at day of randomization at a mean tumor volume of 100 mm3. Mice are treated once weekly i.p. with the respective antibodies (see figure 4) in 20mM Histidine, 140 mM NaCl pH 6.0 buffer. The tumor dimensions are measured by caliper beginning on the staging day and subsequently 2 times per week during the whole treatment period. Tumor volume is calculated according to NCI protocol (Tumor weight = 1/2ab2, where "a" and "b" are the long and the short diameters of the tumor, respectively).

Tumor growth analysis is shown in Figure 4. Inhibition of human CSF-1R on tumor cells with the chimeric anti-CSF-1R Mab 2F11 was statistically more efficacious in mediating tumor growth inhibition than anti-CSF-1R antibody 1.2.SM (CSF-1R antibody described in WO 2009/026303).

In a separate experiment 3 mg/kg i.v. docetaxel (Taxotere® Sanofi Aventis, UK) treatment was combined with anti-mouse CSF-1R antibody (30mg/kg i.p/weekly). Docetaxel was administered 3 times weekly as 1 cycle followed by 3 weeks drug holiday. After 2 cycles of docetaxel treatment antibody monotherapy inhibited primary tumor growth (TGI: 83%, npTCR: 0.5, CI: 0.1-1.8) comparable to the 3 mg/kg docetaxol group (TGI: 75%, npTCR: 0.55, CI: 0.2-1.5). Combination of docetaxol and anti-CSF-1R antibody resulted in superior efficacy than the monotherapies (TGI: 94%, npTCR:0.3, CI: 0.1-0.8). At a later time point differences in TGI between combination and monotherapy groups were less pronounced due to the strong inhibition of each of the monotherapy. Nevertheless the analysis of median survival time revealed superiority of the combination (antibody 159d, docetaxel 154d, combination 180d).

### Example 14

### Combination treatment of an anti-CSF-1R antibodies binding to the domains D4 to D5 of the extracellular domain human CSF-1R with paclitaxel.

### 2.1 Primary Objectives

Part I (Arm A: humanized version of anti-CSF-1R Mab 2F11 (hMab 2F11-e7) single agent [SA] dose escalation; Arm B: humanized CSF-1R antibody Mab 2F11 (hMab 2F11-e7) dose escalation in combination [CD] with fixed dose of paclitaxel):
- To evaluate the safety, tolerability and PK of humanized version of Mab 2F11 when administered alone and in combination with paclitaxel
- To determine the maximum tolerated dose (MTD) and/or Optimal Biological Dose (OBD) of humanized Mab 2F11 when administered alone (MTD1/OBD1) and in combination with paclitaxel (MTD2/OBD2) by observing the dose-limiting toxicities (DLTs).

Part II (Expansion Cohorts/ humanized Mab 2F11 single agent only): To extend safety assessment and investigate humanized Mab 2F11 clinical activity in patients with a tumor entity of particular interest based on observations in Part I of the study, all of whom are not amenable to standard treatment.

### 2.2 Secondary Objectives

### Part I (Dose Escalation/Arm A+B)

- To explore the PK and PD effects of humanized Mab 2F11 alone and in combination with paclitaxel in the tumor and surrogate tissue
- To assess the PD and biomarker effects of humanized Mab 2F11 alone and in combination with paclitaxel as measured by changes in 18F Fluoro-Deoxy-Glucose Positron Emission Tomography (FDG-PET) and Dynamic Contrast-Enhanced Ultrasound (DCE-US) (where available)
- To identify the recommended Phase 2 dose (RP2D) and schedules for humanized CSF-1R antibody Mab 2F11 alone and in combination with paclitaxel
- To explore preliminary clinical activity of humanized Mab 2F11 alone and in combination with paclitaxel, using Objective Response Rate (ORR), Clinical Benefit Rate (CBR), Progression-free survival (PFS), Duration of response.

### Part II (Expansion Cohorts/Arm A only)

- To further characterize the PK and PD effects of humanized Mab 2F11 in the tumor and surrogate tissue

### 2.3 Exploratory Objectives

Collected patient Specimens will be analysed to:
- Retrospectively identify TAM dependent tumors
- Explore possible response prediction markers in surrogate tissue like skin and blood
- Study the association of biomarkers with efficacy and/ or adverse events (AEs) associated with medicinal products; and/ or
- Develop biomarker or diagnostic assays;

### 3. STUDY DESIGN

### 3.1 Overview of study design

This is an open-label, multicenter, Phase Ia/b dose escalation study designed to assess the safety, tolerability, PK and PD of every two weeks (Q2W) i.v. dosing of humanized Mab 2F11. humanized Mab 2F11 will be administered alone for patients with solid tumors (which are not amenable to standard treatment and in combination with paclitaxel in locally advanced and/or metastatic carcinoma which are not amenable to standard treatment.

### Part I - dose escalation

All patients enrolled in the dose escalation cohorts will be assessed for DLTs during a DLT assessment period of 28 days following the first administration of humanized Mab 2F11 in Cycle 1. Patients who discontinue for any reason other than DLT during the DLT assessment period will be replaced.

Humanized Mab 2F11 Monotherapy Administration Mode Humanized Mab 2F11 will be administered Q2W as i.v. infusion over 1.5 h, unless the patient experiences an infusion-related reaction (IRR) which would require slowing or temporary halting of the infusion. Treatment will be administered until disease progression, unacceptable toxicity, death or patient refusal, whichever occurs first.

Humanized Mab 2F11 and Paclitaxel Combination Administration Mode (Part I, Arm B only).

Humanized Mab 2F11 will be administered every Q2W as i.v. infusion over 1.5 h, unless the patient experiences an IRR which would require slowing or temporary halting of the infusion. Treatment will be administered until disease progression, unacceptable toxicity, death or patient refusal, whichever occurs first.

Paclitaxel, at a dose of 80 mg/m2 will be administered QW for up to 12 weeks in combination with humanized Mab 2F11. The paclitaxel infusion will be started as soon as the humanized Mab 2F11 infusion has ended and will be administered according to local prescribing information. If a patient experiences toxicity directly attributable to paclitaxel, he/she may stop treatment with paclitaxel but continue to receive humanized Mab 2F11.

### Part I of the Trial Definition of MTD1/OBD1 and MTD2/OBD2

The first 28 days following the first administration of humanized Mab 2F11 in Cycle 1 will be considered the treatment interval for determination of DLT to define MTD1 and MTD2.

The MTD is defined as the highest dose level(s) at which no more than 1 out of 6 patients experiences a DLT.

Safety data and any available PK/PD data will be collected on an ongoing basis and reviewed prior to each dose escalation decision for the next cohort.

### 3.1.1-3.1.3 Rationale for study design

In-house screening of tumor biopsy samples from different patients with different malignancies has shown significant heterogeneity in the density of infiltrating macrophages and co-incident CSF-1R expression (Please see the non-clinical pharmacology section of the IB). Target-mediated drug disposition (TMDD), i.e. distribution and elimination via binding to the pharmacological target, was also clearly evident in monkey and both tumor-bearing and non-tumor bearing mice. Since the pharmacokinetics of humanized Mab 2F11 is affected by its binding to the target, the quantification of the nonlinear PK can be used as a biomarker to approximate target saturation. In order to characterise to what extent baseline patient demographic factors (including tumor mass and TAM density) may influence the non-linear pharmacokinetics of humanized Mab 2F11, blood levels will be measured within the first few days following a single low (100 mg) 'run-in' dose (cycle 0) in all patients from cohort 2 onwards (i.e. 1 week prior to their cycle 1 dose which will be at least 200 mg or higher). At this low dose, nonlinear PK is expected and this will allow quantification of the TMDD in cancer patients. The value of the run-in dose is that it will provide an understanding whether, in the extension phase of the trial (or future studies), different doses may be more effective in the different extension arms (i.e. different malignancies) based on patient demographic and baseline factors (including tumor type, size and inflammatory status). Since CSF-1R blockade has been demonstrated to selectively inhibit TAMs, thus offering the potential to prevent or event reverse TAM-mediated chemo-resistance [10], a concurrent assessment of humanized Mab 2F11 given in combination with paclitaxel will be initiated. Paclitaxel was chosen as a commonly prescribed chemotherapy for these patient groups and is not expected to produce significant overlapping toxicity, since the most commonly reported toxicities with paclitaxel (myelosuppression, neurotoxicity and arthralgia or myalgia) have not been reported in toxicity studies for A fixed dose of paclitaxel, given QW for up to 12 weeks, will be investigated in combination with ascending doses of humanized Mab 2F11 for patients with advanced breast or ovarian cancer. Recent data have shown that in patients with PVNS and TGCT, over-expression of CSF-1 is detected and is in part mediated by a translocation involving the CSF-1R gene in 30-60% of cases. Further, presence of CSF-1R positive macrophages in several other human cancers (such as ovarian and breast carcinoma) has been shown to correlate not only with increased vascular density but also worse clinical outcome. In breast cancer the presence of a CSF-1 response gene signature predicts risk of recurrence and metastasis. On the basis of these findings and our preclinical models, it seems reasonable to test the hypothesis that blockade of tumor associated macrophages and their pro-tumor bioactivity with humanized Mab 2F11 alone or in combination with paclitaxel has the potential to show clinical activity in patients with certain types of solid tumors.

This study contains a number of blood draws for assessment of PK and PD parameters as well as mandatory fresh and archival tumor tissue collection These are important in enabling a full understanding of the PK properties, mechanism of action and potential for predictive response biomarkers.

### 3.1.4 Rationale for biomarker evaluation

Biomarkers have the potential to shape diagnostic strategies and influence therapeutic management. In the future, biomarkers may promote a personalized medicine approach, grouping patients by the molecular signatures of their tumors and of markers in the blood rather than by cancer type. We are concentrating our efforts in identifying predictive biomarkers, which provide information about the likely efficacy and safety of the therapy. To evaluate the PD and mechanistic effect/s of a drug on the tumor a tumor biopsy is often required.

### 3.1.4.1 Rationale for Fresh Pre-and On-Treatment Tumor Biopsy

TAM infiltration and differentiation is dependent on the respective tumor micro-milieu in primary and metastatic lesions. Furthermore the respective immune status and pre-treatment of the patient might influence the patient's tumor microenvironment. Therefore all patients will undergo a mandatory pre-treatment biopsy to define the TAM infiltration and CSF-1R expression levels at baseline but will not be used to determine patient eligibility for the trial. In addition, mandatory on-treatment biopsies will allow the assessment of the PD activity of humanized Mab 2F11 by comparing pre- and post-dose levels. Fine Needle Aspiration (FNA) will not be suitable to substitute for tumor biopsies, as macrophage sub-population distribution needs to be assessed in the tissue.

Archival tumor tissue cannot substitute for the fresh biopsies as macrophage infiltration and differentiation is micro-milieu dependent. The tumor micro-milieu may be variable in the primary tumor due to pre-treatment of the patient and as well be altered in metastatic lesions. However, if archival tumor tissue is available,samples will be used for exploratory retrospective correlation of data with fresh biopsies

### 3.1.4.2 Rationale for Wounded Skin Biopsies

The different phases of wound healing require many processes (e.g. neutrophil recruitment, macrophage infiltration, angiogenesis (Eming, S.A. et al., Prog. Histochem. Cytochem. 42 (2007) 115-170). Skin wounding assays have been used to obtain surrogate tissue to determine PD markers for e.g. anti-angiogenic therapies (Zhang, D. et al., Invest. New Drugs 25 (2006) 49-55; Lockhart, A.C. et al., Clin. Cancer Res. 9 (2003) 586-593). During wound healing macrophages play a substantial role and phenotypic changes of wound associated macrophages (WAM) account for the different roles in the phases of skin repair (e.g. early inflammatory phase=intense phagocytic activity; mid tissue remodelling phase: immunoregulatory state with overexpression of pro-angiogenic factors) (Adamson, R., Journal of Wound Care 18 (2009) 349-351; Rodero, M.P. et al., Int. J. Clin. Exp. Pathol. 25 (2010) 643-653; Brancato, S.K. and Albina, J.E., Wound Macrophages as Key Regulators of Repair, Origin, Phenotype, and Function. AJP (2011), Vol. 178, No.1).

Indeed, the absence of macrophages resulted in delayed wound healing in genetically engineered mice (Rodero, M.P. et al., Int. J. Clin. Exp. Pathol. 25 (2010) 643-653). Preclinical experiments showed a significant (F4/80 positive) macrophage reduction in the skin of an aCSF-1R treated MDA-MB231 xenograft mouse model. However, species specific differences between mouse and human have been reported (Daley, J.M. et al., J. Leukoc. Biol. 87 (2009) 1-9).

As WAMs and TAMs are originating from the same progenitor cells and share similar functions and phenotypes, serial pre-treatment and on-treatment (total of n=4) skin biopsies will be used to analyze the pharmacodynamics effects of humanized Mab 2F11 treatment on WAMs during the wound healing process. Correlation of the skin data with PD effects of humanized Mab 2F11 treatment on TAMs in fresh tumor biopsies can significantly increase knowledge on the molecular basis of how humanized Mab 2F11 works and how the tumor is responding.

In addition, the assessment of wounded skin tissue might potentially substitute for the on-treatment tumor biopsies in later trials and therefore serve as surrogate tissue to assess humanized Mab 2F11 efficacy.

### 3.1.4.3 Rationale for Whole Blood samples to measure PD markers

These surrogate tissue specimens will be used for research purposes to identify biomarkers that are predictive of response to humanized Mab 2F11 treatment (in terms of dose, safety and tolerability) and will help to better understand the pathogenesis, course and outcome of cancer and related diseases. Analysis may include determination of circulating markers associated with the PD activity of humanized Mab 2F11 (e.g. assessment of cytokine levels, circulating immune cells and immune effector cell depletion). Preclinical experiments have shown that changes in e.g. circulating CSF-1, TRAP5b monocyte subpopulations and tissue macrophages are associated with the drug activity. In addition, GLP-Tox data from humanized Mab 2F11 treated cynomolgus monkeys revealed alterations in bone biomarkers of formation (osteocalcin, P1NP), osteoclast activity (TRAP5b) and parathyroid hormone which all correlated with reduced osteoclast numbers.

Therefore, these exploratory PD markers and additional circulating immunostimulatory or immunoinhibitory factors will be assessed during the study.

### Tumor response criteria

Tumor response will be evaluated according to the RECIST 1.1 criteria In this study, tumor response will be measured using spiral CT scans (including a thoracic scan) or CT scan. X-rays and ultrasound are not acceptable for monitoring target lesions. For each subject, the same method of assessment and the same technique must be used to evaluate each lesion throughout the entire study. If more than one method is used, select the most accurate method according to RECIST when recording data.

Tumor response will be confirmed a minimum of 4 weeks after the initial response was noted, or at the next scheduled tumor assessment if it is to occur more than 4 weeks after the initial response.

An assessment of tumor growth kinetics will be made by comparing post-treatment scans with the last available pre-study scan, if available.

### Pharmacokinetic (PK) / Pharmacodynamic (PD) Assessments

Blood samples will be collected to evaluate the pharmacokinetics PK and /or PD as described in the table below

The total volume blood loss for PK assessments, until the end of Cycle 4, will be approximately 58 mL for Part I, Arm A and Part II and approximately 86 mL for Part I, Arm B. At each subsequent cycle a further 6 mL blood will be collected for PK assessments for each treatment group. The total volume blood loss for PD assessments until the end of Cycle 4 (8 weeks post treatment) will be approximately 161 mL. At each subsequent cycle further 9 ml blood samples (1x5 ml, 1x2ml and 2x1ml; see Table 2 for details) will be collected for PD assessments pre-dose.

### PK Assessments

Blood will be collected for analysis of concentrations for humanized Mab 2F11, humanized anti-human antibody (HAHA) to humanized Mab 2F11 and paclitaxel. In addition, a single blood sample will be taken at the time of an infusion-related reaction of significant magnitude and if the infusion is interrupted or the infusion rate is slowed at the discretion of the investigator.

Serum humanized Mab 2F11 and HAHA will be measured using validated assays. All serum samples collected for HAHA determination will also be analyzed for RO5509554. All blood samples for PK assessment will be collected from an i.v. line different to that receiving the infusion. Samples intended for humanized Mab 2F11 exposure and HAHA analysis will be split into two separate aliquots, one each for humanized Mab 2F11 and HAHA determination

Plasma paclitaxel concentrations will be measured using a validated liquid chromatography tandem mass spectrometry (LC/MS/MS) method.

### PD Assessments

Specimens for dynamic (non inherited) and genetic biomarker (inherited) discovery and validation will be collected from all subjects participating in the trial.

### Whole Blood samples for PD and Biomarkers

Blood as source tissue will be collected to determine the PD effects of humanized Mab 2F11. All blood samples for PD assessment will be collected from an i.v. line different to that receiving the infusion. PD assessments of whole blood samples will include but are not limited to:
- Immunophenotyping (monocyte/macrophage and lymphocyte subsets) using flow cytometry. For monocyte/ macrophage subsets these markers include, but are not limited to, CD14, CD16, CD45, MHC class II and for lymphocytes CD3, CD4, CD8, CD16, CD19, CD45, CD56.
- The total volume blood loss for pharmacodynamic assessments of monocytes/macrophages and lymphocyte cell populations will be approximately **17x** 5ml = 85 mL for the first four cycles.
- Three additional blood samples will be used for the preparation of serum to determine PD related changes of soluble markers. These markers include, but are not limited to:

### Cytokine Assessment A:

CSF-1, Trap5b, sCD163, IL-34

The total volume blood loss for PD Cytokine Assessments A will be approximately 25x 2ml = 50 mL for the first four cycles.

### Cytokine Assessment B:

IFNγ, TNFα, IL-1β, IL-4, IL-6 , IL-8 , IL-10, IL-13, GM-CSF, VEGF, MCP-1, CCL18, CCL22, MIP-1 , Galectin 3, ILIRa, TGF alpha

The total volume blood loss for PD Cytokine Assessments B will be approximately 21x 1ml = 21 mL for the first four cycles.

### Bone Biomarkers:

Bone biomarkers such as osteocalcin, P1NP and parathyroid hormone (PTH) will be assessed.

The total volume blood loss will be approximately 5x 1mL = 5 mL for the first four cycles.

The largest amount of total volume blood loss per cycle for PD/biomarker assessments will be approximately 51 mL.

### Wound Healing Skin Tissue Biopsies

Surrogate wound healing skin tissue will be analyzed for exploratory PD biomarker analyses associated with wound healing process including but not limited to neutrophil recruitment, macrophage infiltration and angiogenesis (see also below).

Two skin paired samples will be taken after local anaesthesia from mirror areas of normal skin (preferably located in the back without hair follicles). They will be obtained by using a 2 and a 4 mm diameter punch biopsy device to obtain 2 overlapping samples, which would not require suturing.

The 2 mm biopsy will create the injury and the fully overlapping 4 mm biopsy 7 days later will collect the wound healing material.

The time interval chosen between 2 biopsies is considered to be adequate, based on the understanding of time-course of changes in relevant biomarkers (neutrophil recruitment, macrophage infiltration, angiogenesis) associated with wound healing process (Eming, S.A. et al., Prog. Histochem. Cytochem. 42 (2007) 115-170; Zhang, D. et al., Invest. New Drugs 25 (2006) 49-55; Lockhart, A.C. et al., Clin. Cancer Res. 9 (2003) 586-593).

All skin samples will undergo analysis for:
- Hematoxylin & eosin staining (H&E)
- Immunohistochemistry (IHC) markers will be analyzed for the following parameters: CSF-1R, CD68/CD163, CD68/MHC class II, CD31 (microvessel density) and Ki67.

The specimens will be formalin fixed and paraffin embedded and shipped to a central laboratory for analysis.

### Tumor Biopsies

### Fresh Tumor Biopsies

Fresh pre-treatment and on-treatment tumor biopsies will be collected to assess pharmacodynamics changes of TAM infiltration and additional tumor markers (see also above).

The biopsies should be preferentially taken from the largest metastatic lesion, may be from the primary tumor, or if possible from both primary tumor and a metastatic site and should be biopsied at the tumor-stroma interface if possible.

Collection of tumor biopsies will be guided by ultrasound or CT scan using an 18 gauge needle to provide cores of at least 20 mm in length. At least 2, ideally 4 core biopsies will be obtained at each time point.

One half of the specimen will be formalin fixed and paraffin embedded The second half will be fresh frozen and collected for long term storage for retrospective exploratory analysis of biomarkers (see section 5.5.3.1.2).

Formalin-fixed, paraffin-embedded biopsy samples will be analyzed for:
- Hematoxylin and eosin staining (H&E).
- Immunohistochemistry (IHC) assessments include, but are not limited to the following markers: CSF-1R, CD68/CD163, CD68/MHC class II, CD31 (microvessel density), Ki67 and other exploratory markers.

### Imaging Modalities for Biomarkers

### DCE- Ultrasound

On the basis of preclinical results we expect that treatment with humanized Mab 2F11 may modulate the microvessel density and the vessel lumen in the tumor and hence the angiogenesis and the transcapillary transport of nutrients to the tumor. To monitor these endpoints, we propose to use DCE-Ultrasound as the choice of imaging modality, where possible.

### FDG-PET

FDG-PET can improve patient management by identifying responders early, before tumor size is reduced; non responders could discontinue futile therapy (Weber, W.A., J. Nucl. Med. 50 (2009) 1S-10S). Moreover, a reduction in the FDG-PET signal within days or weeks of initiating therapy (e.g., in breast (Avril, N. et al., J. Nucl. Med. 50 (2009) 55S-63S), ovarian (Schwarz, J.K. et al., J. Nucl. Med. 50 (2009) 64S-73S), and non-small cell lung (Zander, T. et al., J. Clin. Oncol. (2011) 1701-1708)) significantly correlates with prolonged survival and other clinical end points now used. humanized Mab 2F11 treatment-induced changes in tumor metabolism may be assessed with FDG-PET. In addition, humanized Mab 2F11 induced macrophage depletion may result in the decrease of SUVₘₐₓ in FDG-PET scans.

### Example 15

### Inhibition of tumor growth under treatment with anti-CSF-1R monoclonal antibody in combination with chemotherapy or cancer immunotherapy in subcutaneous syngeneic MC38 colon carcinoma models

Cells of the murine colorectal adenocarcinoma cell line MC-38 (obtained from Beckman Research Institute of the City of Hope, California, USA) were cultured in Dulbecco's Modified Eagle Medium (DMEM, PAN Biotech) supplemented with 10% FCS and 2mM L-glutamine at 37°C in a water saturated atmosphere at 5% CO2. At the day of inoculation, MC38 tumor cells were harvested with PBS from culture flasks and transferred into culture medium, centrifuged, washed once and re-suspended in PBS. For injection of cells, the final titer was adjusted to 1×107 cells/ml. Subsequently 100 µl of this suspension (1×106 cells) were inoculated subcutaneously into 7-9 weeks old female C57BL/6N mice (obtained from Charles River, Sulzfeld, Germany). Treatment with control antibody (MOPC-21; Bio X Cell, West Lebanon), anti-murine CSF-1R mAb <mouse CSF1R> antibody at a weekly dose of 30 mg/kg i.p. alone or in combination with IL-2 (Proleukin, Novartis, 100 000 IU/animal i.p. twice daily), or FOLFIRI (5-Fluorouracil, Medac, 100 mg/kg, i.p., 1x / Leucovorin, Pfizer, 40 mg/kg, i.p., 1x / Irinotecan, HEXAL, 20 mg/kg, i.p., 1x) or Oxaliplatin (Eloxatin, Sanofi-Aventis 5 mg/kg, i.p. 1x) started after tumors were established and had reached an average size of 50 mm3. Tumor volume was measured twice a week and animal weights were monitored in parallel. In a separate study with comparable set-up, primary tumors from indicated treatment groups were excised, weighed and subjected to FACS analysis. Primary tumor material was collected between study day 20-25 as indicated. To obtain single cell suspensions amenable for flow cytometry analysis the tumors were minced by using the McIlwain tissue chopper. Subsequently, the tumor pieces were resuspended in RPMI media supplemented with collagenase I, dispase II and DNAse I, incubated at 37°C and cell suspension were passed through a mash. CD45 positive cells were enriched by magnetic cell separation according to the manufacturer's instructions (Miltenyi). Briefly cells were labeled with anti-mouse CD45 conjugated with APC (BD, Cat.No 559864) and separated with anti APC microbeads. To analyse CD8+ T cells these CD45 positive cells were stained with 0,2µg/ml DAPI (Roche, Cat.No10236276001 and PE conjugated CD8 antibody (eBioscience Cat.No.12-0081-83) or PE conjugated CD4 antibody (eBioscience, Cat.No.2-0041-83). Acquisition of data was performed with FACS Canto II and subsequently analysed with FlowJo software. Only viable cells (gated on DAPI-negative cells) were analysed to exclude cell debris and dead cells.

Monotherapy with <mouse CSF1R> antibody inhibited primary tumor growth when compared to control antibody treatment (TGI: 61%, TCR: 0.39 CI: 0.15-0.68). Also IL2 monotherapy had an effect on MC38 primary tumor growth (TGI: 47%, TCR: 0.53 CI: 0.27-0.85). Addition of <mouse CSF1R> antibody to IL-2 therapy led to a superior anti-tumor efficacy compared to IL-2 treatment alone (TGI: 78%, TCR: 0.21 CI: 0.02-0.48) Treatment with the chemotherapeutic regimen FOLFIRI also significantly inhibited tumor growth (TGI: 66%, TCR: 0.34 CI: 0.11-0.61) and addition of <mouse CSF1R> antibody led to a further improved outcome (TGI: 77%, TCR: 0.23 CI: 0.001-0.48). Oxaliplatin also showed some but less pronounced efficacy on MC38 tumor growth (TGI: 46%, TCR: 0.54 CI: 0.29-0.86) that nevertheless could be enhanced by combination with the <mouse CSF1R> antibody (TGI: 69%, TCR: 0.31 CI: 0.07-0.59). When looking at the progression of individual tumors above a size of 700 mm3, the median time to progression of animals treated with the combination of <mouse CSF-1R> antibody with IL-2 was superior to combination with chemotherapies in this model (see table 11).

**Table 11:**

| **Anti tumor Efficacy of <mouse CSF1R> antibody combinations in the MC38 mouse CRC in vivo model** | | | |
|---|---|---|---|
| **Group** | **TGI** (day 21) | **TCR** (day 21) | **Median time to progression** TV > 700 mm3 |
| Control (Mouse IgG1) | -- | | 17 |
| <mouse CSF1R> antibody | 61% | 0.39 | 21 |
| Oxaliplatin | 46% | 0.54 | 21 |
| FOLFIRI | 66% | 0.34 | 22 |
| Proleukin | 47% | 0.53 | 21 |
| <mouse CSF1R> antibody/ Eloxatin | 69% | 0.31 | 21 |
| <mouse CSF1R> antibody /FOLFIRI | 77% | 0.23 | 27,5 |
| <mouse CSF1R> antibody /Proleukin | 78% | 0.22 | 30 |

Flow cytometry analysis of tumors treated with <mouse CSF-1R antibody> revealed a 3-fold increase in the numbers of CD8+ T cells compared to Oxaliplatin monotherapy as well as a slight increase in CD4+ T cells. Tumors treated with the combination of CSF-1R neutralizing antibody and Oxaliplatin showed a comparable increase of T cells when treated with antibody alone. Similar results were obtained for the combination with FOLFIRI. Results are also shown in Figure 5.

### Example 16

### Inhibition of tumor growth under treatment with anti-CSF-1R monoclonal antibody in combination with anti-CD40 monoclonal antibody in subcutaneous syngeneic MC38 colon carcinoma model

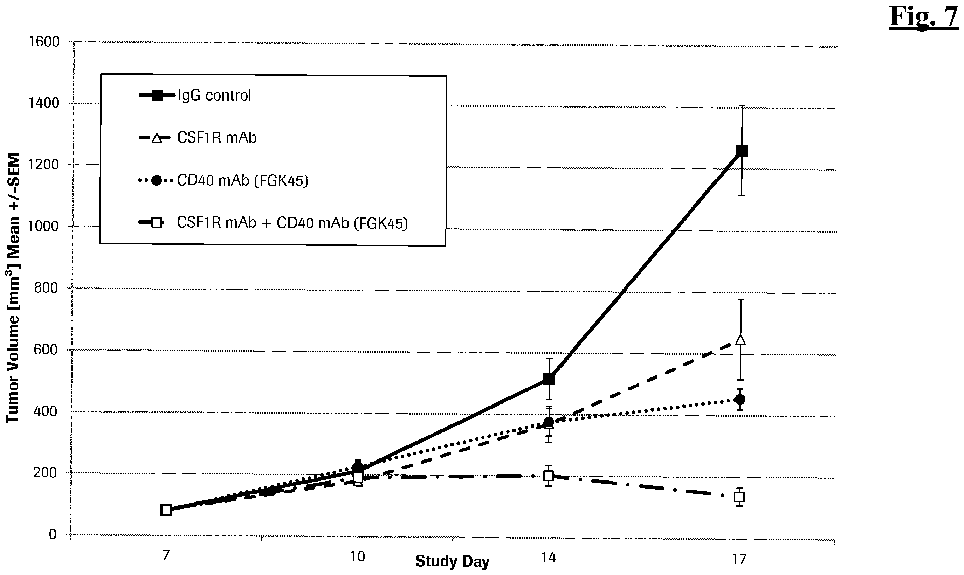

Cells of the murine colorectal adenocarcinoma cell line MC-38 (obtained from Beckman Research Institute of the City of Hope, California, USA) were cultured in Dulbecco's Modified Eagle Medium (DMEM, PAN Biotech) supplemented with 10% FCS and 2mM L-glutamine at 37°C in a water saturated atmosphere at 5% CO2. At the day of inoculation, MC38 tumor cells were harvested with PBS from culture flasks and transferred into culture medium, centrifuged, washed once and re-suspended in PBS. For injection of cells, the final titer is adjusted to 1×107 cells/ml. Subsequently 100 µl of this suspension (1×106 cells) were inoculated subcutaneously into 6-10 weeks old female C57BL/6N mice. Groups of animals were treated with control antibodies (MOPC-21 (30 mg/kg i.p. once weekly) and 2A3 (100 µg i.p. once); Bio X Cell, West Lebanon), anti-murine CSF-1R mAb <mouse CSF1R> antibody (30 mg/kg i.p. once weekly) alone or in combination with anti-CD40 monoclonal antibody FGK45 (agonist CD40 rat anti-mouse IgG2a mAb FGK45 (S. P. Schoenberger, et al, Nature, 393, 480 (1998), availaible from BioXcell) CD40 (FGK45)) (100 µg, i.p., 1x). Treatment started after tumors were established and had reached an average size of 50 mm3. Tumor volume was measured twice a week and animal weights were monitored in parallel. Results are shown in Figure 7. Combination of CSF1R mAb + CD40 mAb FGK45 shows improved anti-tumor efficacy over monotherapies in syngenic MC38 mouse colon cancer model

### Example 17

### Inhibition of tumor growth under treatment with anti-CSF-1R monoclonal antibody in combination with anti-Ang2/VEGF monoclonal antibody and/or FOLFIRI in subcutaneous syngeneic MC38 colon carcinoma model

Cells of the murine colorectal adenocarcinoma cell line MC-38 (obtained from Beckman Research Institute of the City of Hope, California, USA) are cultured in Dulbecco's Modified Eagle Medium (DMEM, PAN Biotech) supplemented with 10% FCS and 2mM L-glutamine at 37°C in a water saturated atmosphere at 5% CO2. At the day of inoculation, MC38 tumor cells are harvested with PBS from culture flasks and transferred into culture medium, centrifuged, washed once and re-suspended in PBS. For injection of cells, the final titer is adjusted to 1×107 cells/ml. Subsequently 100 µl of this suspension (1×106 cells) are inoculated subcutaneously into 7 weeks old female C57BL/6N mice. Treatment with control antibody (MOPC-21; Bio X Cell, West Lebanon), anti-murine CSF-1R mAb <mouse CSF1R> antibody at a weekly dose of 30 mg/kg i.p. alone or in combination with FOLFIRI (5-Fluorouracil, Medac, 100 mg/kg, i.p., 1x / Leucovorin, Pfizer, 40 mg/kg, i.p., 1x / Irinotecan, HEXAL, 20 mg/kg, i.p., 1x) or anti-Ang2/VEGF monoclonal antibody (the bispecific ANG-2-VEGF antibody XMab1 as described in WO2011/117329) (10 mg/kg, i.p., 1x weekly) starts after tumors are established and have reached an average size of 50 mm³. Triple combination treatment is performed as described in Table 13. Tumor volume is measured twice a week and animal weights are monitored in parallel.

Monotherapy with <mouse CSF1R> antibody, anti-Ang2/VEGF antibody or FOLFIRI minimally inhibited primary tumor growth when compared to control antibody treatment (TGI: 28%, 35% or 11%, respectively). Combination of <mouse CSF1R> antibody with either anti-Ang2/VEGF antibody or FOLFIRI led to more pronounced and statistically significant anti-tumor efficacy compared to the control antibody (TGI: 64% or 67%) Triple combination treatment of <mouse CSF-1R> antibody with FOLFIRI followed by the treatment with the anti-Ang2/VEGF antibody 2 days or 9 days thereafter showed the best anti-tumor activity (TGI: 68% or 70%). Concurrent treatment of the 3 compounds or combination of the anti-Ang2/VEGF antibody with FOLFIRI followed by the treatment with of <mouse CSF-1R> antibody 9 days thereafter just yielded an anti-tumor activity of 61% or 56%, respectively. When looking at the progression of individual tumors above a size of 700 mm3, the median time to progression of animals treated with the combination of <mouse CSF-1R> antibody with FOLFIRI followed by the treatment with the anti-Ang2/VEGF antibody 2 days or 9 days thereafter was also superior to the median time to progression of all other treatments in this model (see table 12).

**Table 13:**

| **Anti tumor Efficacy of <mouse CSF1R> antibody in combination with anti-Ang2/VEGF monoclonal antibody and/or FOLFIRI in the MC38 mouse CRC in vivo model** | | | |
|---|---|---|---|
| **Group** | **TGI** (day 20) | **TCR** (day 20) | **Median time to progression** TV > 700 mm3 |
| Control (Mouse IgG1) | --- | --- | 20 |
| <mouse CSF1R> antibody | 28% | 0.72 | 22 |
| anti-Ang2/VEGF antibody | 35% | 0.65 | 23 |
| FOLFIRI | 11% | 0.84 | 20 |
| <mouse CSF1R> antibody/FOLFIRI | 67% | 0.34 | 26 |
| anti-Ang2/VEGF antibody /FOLFIRI | 43% | 0.52 | 24 |

| **Group** | **TGI** (day 20) | **TCR** (day 20) | **Median time to progression** TV > 700 mm3 |
|---|---|---|---|
| <mouse CSF1R> antibody / anti-Ang2/VEGF antibody | 64% | 0.35 | 27 |
| <mouse CSF1R> antibody/FOLFIRI/ anti-Ang2/VEGF antibody; concurrent treatment | 61% | 0.39 | 26 |
| <mouse CSF1R> antibody (day 7)/FOLFIRI/ anti-Ang2/VEGF antibody (day 9) | 68 | 0.27 | 28 |
| <mouse CSF1R> antibody (day 7)//FOLFIRI/ anti-Ang2/VEGF antibody (day 16) | 70% | 0.22 | 28 |
| <mouse CSF1R> antibody (day 16)/FOLFIRI/ anti-Ang2/VEGF antibody (day 7) | 56 | 0.43 | 26 |

## Claims

1. An antibody binding to human CSF-1R for use in the treatment of cancer wherein the anti-CSF-1R antibody is administered in combination with an agonistic CD40 antibody.

2. The antibody binding to human CSF-1R for use according to claim 1
i) wherein the anti-CSF-1R antibody comprises (a) a heavy chain variable domain amino acid sequence of SEQ ID NO:39 and (b) a light chain variable domain amino acid sequence of SEQ ID NO:40; and
ii) wherein the agonistic CD40 antibody comprises (a) a heavy chain variable domain amino acid sequence of SEQ ID NO: 88 and (b) a light chain variable domain amino acid sequence of SEQ ID NO: 89.

## Patentansprüche

1. An humanen CSF-1R bindender Antikörper zur Verwendung bei der Behandlung von Krebs, wobei der Anti-CSF-1R-Antikörper in Kombination mit einem CD40-Agonistenantikörper verabreicht wird.

2. An humanen CSF-1R bindender Antikörper zur Verwendung nach Anspruch 1
i) wobei der Anti-CSF-1R-Antikörper (a) eine variable Domäne der schweren Kette mit der Aminosäuresequenz von SEQ ID NO:39 und (b) eine variable Domäne der leichten Kette mit der Aminosäuresequenz von SEQ ID NO:40 umfasst; und
ii) wobei der CD40-Agonistenantikörper (a) eine variable Domäne der schweren Kette mit der Aminosäuresequenz von SEQ ID NO:88 und (b) eine variable Domäne der leichten Kette mit der Aminosäuresequenz von SEQ ID NO:89 umfasst.

## Revendications

1. Anticorps se liant au CSF-1R humain pour une utilisation dans le traitement d'un cancer, dans lequel l'anticorps anti-CSF-1R est administré en association avec un anticorps CD40 agoniste.

2. Anticorps se liant au CSF-1R humain pour une utilisation selon la revendication 1
i) dans lequel l'anticorps anti-CSF-1R comprend (a) une séquence d'acides aminés de domaine variable de chaîne lourde de SEQ ID NO: 39 et (b) une séquence d'acides aminés de domaine variable de chaîne légère de SEQ ID NO: 40 ; et
ii) dans lequel l'anticorps CD40 agoniste comprend (a) une séquence d'acides aminés de domaine variable de chaîne lourde de SEQ ID NO: 88 et (b) une séquence d'acides aminés de domaine variable de chaîne légère de SEQ ID NO: 89.


### SEQUENCE LISTING

<110> F. Hoffmann-La Roche AG
<120> Combination therapy of antibodies against human CSF-1R and uses thereof
<130> 30886 WO
<150> EP12158519.4
   <151> 2012-03-08
<160> 91
<170> PatentIn version 3.5
<210> 1
   <211> 8
   <212> PRT
   <213> Mus musculus
<400> 1
   Asp Gln Arg Leu Tyr Phe Asp Val
   1               5
<210> 2
   <211> 16
   <212> PRT
   <213> Mus musculus
<400> 2
   Val Ile Trp Thr Asp Gly Gly Thr Asn Tyr Asn Ser Pro Phe Met Ser
   1               5                   10                  15
<210> 3
   <211> 5
   <212> PRT
   <213> Mus musculus
<400> 3
   Thr Tyr Asp Ile Ser
   1               5
<210> 4
   <211> 8
   <212> PRT
   <213> Mus musculus
<400> 4
   Gly Gln Ser Phe Ser Tyr Pro Thr
   1               5
<210> 5
   <211> 7
   <212> PRT
   <213> Mus musculus
<400> 5
   Gly Ala Ser Asn Arg Tyr Thr
   1               5
<210> 6
   <211> 11
   <212> PRT
   <213> Mus musculus
<400> 6
   Lys Ala Ser Glu Asp Val Asn Thr Tyr Val Ser
   1               5                   10
<210> 7
   <211> 116
   <212> PRT
   <213> Mus musculus
<400> 7
   Gln Val Gln Leu Lys Glu Ser Gly Pro Gly Leu Val Ala Pro Ser Gln
   1               5                   10                  15
   Ser Leu Ser Ile Thr Cys Thr Val Ser Gly Phe Ser Leu Thr Thr Tyr
               20                  25                  30
   Asp Ile Ser Trp Ile Arg Gln Ser Pro Gly Lys Gly Leu Glu Trp Leu
           35                  40                  45
   Gly Val Ile Trp Thr Asp Gly Gly Thr Asn Tyr Asn Ser Pro Phe Met
       50                  55                  60
   Ser Arg Leu Ser Ile Arg Lys Asp Asn Ser Lys Ser Gln Val Phe Leu
   65                  70                  75                  80
   Lys Met Asn Arg Leu Gln Thr Asp Asp Thr Ala Ile Tyr Tyr Cys Val
                   85                  90                  95
   Arg Asp Gln Arg Leu Tyr Phe Asp Val Trp Gly Ala Gly Thr Thr Val
               100                 105                 110
   Thr Val Ser Ser
           115
<210> 8
   <211> 106
   <212> PRT
   <213> Mus musculus
<400> 8
   Asn Ile Val Met Thr Gln Ser Pro Lys Ser Met Ser Met Ser Val Gly
   1               5                   10                  15
   Glu Arg Val Thr Leu Asn Cys Lys Ala Ser Glu Asp Val Asn Thr Tyr
               20                  25                  30
   Val Ser Trp Tyr Gln Gln Gln Pro Glu Gln Ser Pro Lys Leu Leu Ile
           35                  40                  45
   Tyr Gly Ala Ser Asn Arg Tyr Thr Gly Val Pro Asp Arg Phe Thr Gly
       50                  55                  60
   Gly Gly Ser Thr Thr Asp Phe Thr Leu Thr Ile Ser Ser Val Gln Ala
   65                  70                  75                  80
   Glu Asp Leu Ala Asp Tyr Phe Cys Gly Gln Ser Phe Ser Tyr Pro Thr
                   85                  90                  95
   Phe Gly Thr Gly Thr Lys Leu Glu Ile Lys
               100                 105
<210> 9
   <211> 7
   <212> PRT
   <213> Mus musculus
<400> 9
   Asp Pro Arg Leu Tyr Phe Asp
   1               5
<210> 10
   <211> 16
   <212> PRT
   <213> Mus musculus
<400> 10
   Val Ile Trp Thr Gly Gly Gly Thr Asn Tyr Asn Ser Gly Phe Met Ser
   1               5                   10                  15
<210> 11
   <211> 5
   <212> PRT
   <213> Mus musculus
<400> 11
   Ser Phe Asp Ile Ser
   1               5
<210> 12
   <211> 8
   <212> PRT
   <213> Mus musculus
<400> 12
   Gly Gln Thr Phe Ser Tyr Pro Thr
   1 5
<210> 13
   <211> 7
   <212> PRT
   <213> Mus musculus
<400> 13
   Gly Ala Ser Asn Arg Tyr Thr
   1 5
<210> 14
   <211> 11
   <212> PRT
   <213> Mus musculus
<400> 14
   Lys Ala Ser Glu Asp Val Val Thr Tyr Val Ser
   1 5 10
<210> 15
   <211> 116
   <212> PRT
   <213> Mus musculus
<400> 15
   Gln Val Gln Leu Lys Glu Ser Gly Pro Gly Leu Val Ala Pro Ser Lys
   1 5 10 15
   Ser Leu Ser Ile Thr Cys Thr Val Ser Gly Ser Ser Leu Asp Ser Phe
   20 25 30
   Asp Ile Ser Trp Ile Arg Gln Ser Pro Gly Lys Gly Leu Glu Trp Leu
   35 40 45
   Gly Val Ile Trp Thr Gly Gly Gly Thr Asn Tyr Asn Ser Gly Phe Met
   50 55 60
   Ser Arg Leu Arg Ile Thr Lys Asp Asn Ser Lys Ser Gln Val Leu Leu
   65 70 75 80
   Lys Met Asn Ser Leu Gln Ser Asp Asp Thr Ala Ile Tyr Tyr Cys Val
   85 90 95
   Arg Asp Pro Arg Leu Tyr Phe Asp Val Trp Gly Ala Gly Thr Thr Val
   100 105 110
   Thr Val Ser Ser
   115
<210> 16
   <211> 106
   <212> PRT
   <213> Mus musculus
<400> 16
   Asn Ile Val Met Thr Gln Ser Pro Lys Ser Met Ser Met Ser Val Gly
   1 5 10 15
   Glu Arg Val Thr Leu Ser Cys Lys Ala Ser Glu Asp Val Val Thr Tyr
   20 25 30
   Val Ser Trp Tyr Gln Gln Lys Pro Asp Gln Ser Pro Lys Leu Leu Ile
   35 40 45
   Tyr Gly Ala Ser Asn Arg Tyr Thr Gly Val Pro Asp Arg Phe Thr Gly
   50 55 60
   Ser Gly Ser Ala Thr Asp Phe Thr Leu Thr Ile Ser Ser Val Gln Ala
   65 70 75 80
   Glu Asp Leu Ala Asp Tyr Tyr Cys Gly Gln Thr Phe Ser Tyr Pro Thr
   85 90 95
   Phe Gly Thr Gly Thr Lys Leu Glu Ile Lys
   100 105
<210> 17
   <211> 8
   <212> PRT
   <213> Artificial
<220>
   <223> heavy chain CDR3, hMab 2F11-c11
<400> 17
   Asp Gln Arg Leu Tyr Phe Asp Val
   1 5
<210> 18
   <211> 16
   <212> PRT
   <213> Artificial
<220>
   <223> heavy chain CDR2, hMab 2F11-c11
<400> 18
   Val Ile Trp Thr Asp Gly Gly Thr Asn Tyr Asn Ser Pro Phe Met Ser
   1 5 10 15
<210> 19
   <211> 5
   <212> PRT
   <213> Artificial
<220>
   <223> heavy chain CDR1, hMab 2F11-c11
<400> 19
   Thr Tyr Asp Ile Ser
   1 5
<210> 20
   <211> 8
   <212> PRT
   <213> Artificial
<220>
   <223> light chain CDR3, hMab 2F11-c11
<400> 20
   Gly Gln Ser Phe Ser Tyr Pro Thr
   1 5
<210> 21
   <211> 7
   <212> PRT
   <213> Artificial
<220>
   <223> light chain CDR2, hMab 2F11-c11
<400> 21
   Gly Ala Ser Asn Arg Tyr Thr
   1 5
<210> 22
   <211> 11
   <212> PRT
   <213> Artificial
<220>
   <223> light chain CDR1, hMab 2F11-c11
<400> 22
   Arg Ala Ser Glu Asp Val Asn Thr Tyr Val Ser
   1 5 10
<210> 23
   <211> 116
   <212> PRT
   <213> Artificial
<220>
   <223> heavy chain variable domain, hMab 2F11-c11
<400> 23
   Gln Val Gln Leu Val Gln Ser Gly Ala Glu Val Lys Lys Pro Gly Ser
   1 5 10 15
   Ser Val Lys Val Ser Cys Lys Ala Ser Gly Phe Ser Leu Thr Thr Tyr
   20 25 30
   Asp Ile Ser Trp Ile Arg Gln Ala Pro Gly Gln Gly Leu Glu Trp Met
   35 40 45
   Gly Val Ile Trp Thr Asp Gly Gly Thr Asn Tyr Asn Ser Pro Phe Met
   50 55 60
   Ser Arg Val Thr Ile Thr Lys Asp Glu Ser Thr Ser Thr Ala Tyr Met
   65 70 75 80
   Glu Leu Ser Ser Leu Arg Ser Glu Asp Thr Ala Val Tyr Tyr Cys Val
   85 90 95
   Arg Asp Gln Arg Leu Tyr Phe Asp Val Trp Gly Gln Gly Thr Thr Val
   100 105 110
   Thr Val Ser Ser
   115
<210> 24
   <211> 106
   <212> PRT
   <213> Artificial
<220>
   <223> light chain variable domain, hMab 2F11-c11
<400> 24
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Glu Asp Val Asn Thr Tyr
               20                  25                  30
   Val Ser Trp Tyr Gln Gln Lys Pro Gly Lys Ala Pro Lys Leu Leu Ile
           35                  40                  45
   Tyr Gly Ala Ser Asn Arg Tyr Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gly Gln Ser Phe Ser Tyr Pro Thr
                   85                  90                  95
   Phe Gly Gln Gly Thr Lys Leu Glu Ile Lys
               100                 105
<210> 25
   <211> 8
   <212> PRT
   <213> Artificial
<220>
   <223> heavy chain CDR3, hMab 2F11-d8
<400> 25
   Asp Gln Arg Leu Tyr Phe Asp Val
   1               5
<210> 26
   <211> 16
   <212> PRT
   <213> Artificial
<220>
   <223> heavy chain CDR2, hMab 2F11-d8
<400> 26
   Val Ile Trp Thr Asp Gly Gly Ala Asn Tyr Ala Gln Lys Phe Gln Gly
   1               5                   10                  15
<210> 27
   <211> 5
   <212> PRT
   <213> Artificial
<220>
   <223> heavy chain CDR1, hMab 2F11-d8
<400> 27
   Thr Tyr Asp Ile Ser
   1               5
<210> 28
   <211> 8
   <212> PRT
   <213> Artificial
<220>
   <223> light chain CDR3, hMab 2F11-d8
<400> 28
   Gly Gln Ser Phe Ser Tyr Pro Thr
   1               5
<210> 29
   <211> 7
   <212> PRT
   <213> Artificial
<220>
   <223> light chain CDR2, hMab 2F11-d8
<400> 29
   Gly Ala Ser Asn Arg Tyr Thr
   1               5
<210> 30
   <211> 11
   <212> PRT
   <213> Artificial
<220>
   <223> light chain CDR1, hMab 2F11-d8
<400> 30
   Lys Ala Ser Glu Asp Val Asn Thr Tyr Val Ser
   1               5                   10
<210> 31
   <211> 116
   <212> PRT
   <213> Artificial
<220>
   <223> heavy chain variable domain, hMab 2F11-d8
<400> 31
   Gln Val Gln Leu Val Gln Ser Gly Ala Glu Val Lys Lys Pro Gly Ser
   1               5                   10                  15
   Ser Val Lys Val Ser Cys Lys Ala Ser Gly Phe Ser Leu Thr Thr Tyr
               20                  25                  30
   Asp Ile Ser Trp Val Arg Gln Ala Pro Gly Gln Gly Leu Glu Trp Met
           35                  40                  45
   Gly Val Ile Trp Thr Asp Gly Gly Ala Asn Tyr Ala Gln Lys Phe Gln
       50                  55                  60
   Gly Arg Val Thr Ile Thr Ala Asp Glu Ser Thr Ser Thr Ala Tyr Met
   65                  70                  75                  80
   Glu Leu Ser Ser Leu Arg Ser Glu Asp Thr Ala Val Tyr Tyr Cys Ala
                   85                  90                  95
   Arg Asp Gln Arg Leu Tyr Phe Asp Val Trp Gly Gln Gly Thr Thr Val
               100                 105                 110
   Thr Val Ser Ser
           115
<210> 32
   <211> 106
   <212> PRT
   <213> Artificial
<220>
   <223> light chain variable domain, hMab 2F11-d8
<400> 32
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Glu Asp Val Asn Thr Tyr
               20                  25                  30
   Val Ser Trp Tyr Gln Gln Lys Pro Gly Lys Ala Pro Lys Leu Leu Ile
           35                  40                  45
   Tyr Gly Ala Ser Asn Arg Tyr Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gly Gln Ser Phe Ser Tyr Pro Thr
                   85                  90                  95
   Phe Gly Gln Gly Thr Lys Leu Glu Ile Lys
               100                 105
<210> 33
   <211> 8
   <212> PRT
   <213> Artificial
<220>
   <223> heavy chain CDR3, hMab 2F11-e7
<400> 33
   Asp Gln Arg Leu Tyr Phe Asp Val
   1               5
<210> 34
   <211> 16
   <212> PRT
   <213> Artificial
<220>
   <223> heavy chain CDR2, hMab 2F11-e7
<400> 34
   Val Ile Trp Thr Asp Gly Gly Thr Asn Tyr Ala Gln Lys Leu Gln Gly
   1               5                   10                  15
<210> 35
   <211> 5
   <212> PRT
   <213> Artificial
<220>
   <223> heavy chain CDR1, hMab 2F11-e7
<400> 35
   Ser Tyr Asp Ile Ser
   1 5
<210> 36
   <211> 8
   <212> PRT
   <213> Artificial
<220>
   <223> light chain CDR3, hMab 2F11-e7
<400> 36
   Gln Gln Ser Phe Ser Tyr Pro Thr
   1 5
<210> 37
   <211> 7
   <212> PRT
   <213> Artificial
<220>
   <223> light chain CDR2, hMab 2F11-e7
<400> 37
   Ala Ala Ser Asn Arg Tyr Thr
   1 5
<210> 38
   <211> 11
   <212> PRT
   <213> Artificial
<220>
   <223> light chain CDR1, hMab 2F11-e7
<400> 38
   Arg Ala Ser Glu Asp Val Asn Thr Tyr Val Ser
   1 5 10
<210> 39
   <211> 116
   <212> PRT
   <213> Artificial
<220>
   <223> heavy chain variable domain, hMab 2F11-e7
<400> 39
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Ser Tyr
   20 25 30
   Asp Ile Ser Trp Val Arg Gln Ala Pro Gly Gln Gly Leu Glu Trp Met
   35 40 45
   Gly Val Ile Trp Thr Asp Gly Gly Thr Asn Tyr Ala Gln Lys Leu Gln
   50 55 60
   Gly Arg Val Thr Met Thr Thr Asp Thr Ser Thr Ser Thr Ala Tyr Met
   65 70 75 80
   Glu Leu Arg Ser Leu Arg Ser Asp Asp Thr Ala Val Tyr Tyr Cys Ala
   85 90 95
   Arg Asp Gln Arg Leu Tyr Phe Asp Val Trp Gly Gln Gly Thr Thr Val
   100 105 110
   Thr Val Ser Ser
   115
<210> 40
   <211> 106
   <212> PRT
   <213> Artificial
<220>
   <223> light chain variable domain, hMab 2F11-e7
<400> 40
   Asp Ile Gln Met Thr Gln Ser Pro Ser Ser Leu Ser Ala Ser Val Gly
   1 5 10 15
   Asp Arg Val Thr Ile Thr Cys Arg Ala Ser Glu Asp Val Asn Thr Tyr
   20 25 30
   Val Ser Trp Tyr Gln Gln Lys Pro Gly Lys Ala Pro Lys Leu Leu Ile
   35 40 45
   Tyr Ala Ala Ser Asn Arg Tyr Thr Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Phe Ser Tyr Pro Thr
   85 90 95
   Phe Gly Gln Gly Thr Lys Leu Glu Ile Lys
   100 105
<210> 41
   <211> 8
   <212> PRT
   <213> Artificial
<220>
   <223> heavy chain CDR3, hMab 2F11-f12
<400> 41
   Asp Gln Arg Leu Tyr Phe Asp Val
   1 5
<210> 42
   <211> 16
   <212> PRT
   <213> Artificial
<220>
   <223> heavy chain CDR2, hMab 2F11-f12
<400> 42
   Val Ile Trp Thr Asp Gly Gly Thr Asn Tyr Asn Ser Pro Phe Met Ser
   1 5 10 15
<210> 43
   <211> 5
   <212> PRT
   <213> Artificial
<220>
   <223> heavy chain CDR1, hMab 2F11-f12
<400> 43
   Thr Tyr Asp Ile Ser
   1 5
<210> 44
   <211> 8
   <212> PRT
   <213> Artificial
<220>
   <223> light chain CDR3, hMab 2F11-f12
<400> 44
   Gly Gln Ser Phe Ser Tyr Pro Thr
   1 5
<210> 45
   <211> 7
   <212> PRT
   <213> Artificial
<220>
   <223> light chain CDR2, hMab 2F11-f12
<400> 45
   Gly Ala Ser Ser Leu Gln Ser
   1 5
<210> 46
   <211> 11
   <212> PRT
   <213> Artificial
<220>
   <223> light chain CDR1, hMab 2F11-f12
<400> 46
   Arg Ala Ser Glu Asp Val Asn Thr Tyr Val Ser
   1 5 10
<210> 47
   <211> 116
   <212> PRT
   <213> Artificial
<220>
   <223> heavy chain variable domain, hMab 2F11-f12
<400> 47
   Gln Val Gln Leu Val Gln Ser Gly Ala Glu Val Lys Lys Pro Gly Ser
   1               5                   10                  15
   Ser Val Lys Val Ser Cys Lys Ala Ser Gly Phe Ser Leu Thr Thr Tyr
               20                  25                  30
   Asp Ile Ser Trp Val Arg Gln Ala Pro Gly Gln Gly Leu Glu Trp Met
           35                  40                  45
   Gly Val Ile Trp Thr Asp Gly Gly Thr Asn Tyr Asn Ser Pro Phe Met
       50                  55                  60
   Ser Arg Val Thr Ile Thr Lys Asp Glu Ser Thr Ser Thr Ala Tyr Met
   65                  70                  75                  80
   Glu Leu Ser Ser Leu Arg Ser Glu Asp Thr Ala Val Tyr Tyr Cys Val
                   85                  90                  95
   Arg Asp Gln Arg Leu Tyr Phe Asp Val Trp Gly Gln Gly Thr Thr Val
               100                 105                 110
   Thr Val Ser Ser
           115
<210> 48
   <211> 106
   <212> PRT
   <213> Artificial
<220>
   <223> light chain variable domain, hMab 2F11-f12
<400> 48
   Asp Ile Gln Met Thr Gln Ser Pro Ser Ser Leu Ser Ala Ser Val Gly
   1 5 10 15
   Asp Arg Val Thr Ile Thr Cys Arg Ala Ser Glu Asp Val Asn Thr Tyr
   20 25 30
   Val Ser Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gly Gln Ser Phe Ser Tyr Pro Thr
   85 90 95
   Phe Gly Gln Gly Thr Lys Leu Glu Ile Lys
   100 105
<210> 49
   <211> 8
   <212> PRT
   <213> Artificial
<220>
   <223> heavy chain CDR3, hMab 2F11-g1
<400> 49
   Asp Gln Arg Leu Tyr Phe Asp Val
   1 5
<210> 50
   <211> 16
   <212> PRT
   <213> Artificial
<220>
   <223> heavy chain CDR2, hMab 2F11-g1
<400> 50
   Val Ile Trp Thr Asp Gly Gly Thr Asn Tyr Asn Ser Pro Leu Lys Ser
   1 5 10 15
<210> 51
   <211> 5
   <212> PRT
   <213> Artificial
<220>
   <223> heavy chain CDR1, hMab 2F11-g1
<400> 51
   Thr Tyr Asp Ile Ser
   1 5
<210> 52
   <211> 8
   <212> PRT
   <213> Artificial
<220>
   <223> light chain CDR3, hMab 2F11-g1
<400> 52
   Gly Gln Ser Phe Ser Tyr Pro Thr
   1 5
<210> 53
   <211> 7
   <212> PRT
   <213> Artificial
<220>
   <223> light chain CDR2, hMab 2F11-g1
<400> 53
   Gly Ala Ser Ser Arg Ala Thr
   1 5
<210> 54
   <211> 11
   <212> PRT
   <213> Artificial
<220>
   <223> light chain CDR1, hMab 2F11-g1
<400> 54
   Arg Ala Ser Glu Asp Val Asn Thr Tyr Leu Ala
   1 5 10
<210> 55
   <211> 116
   <212> PRT
   <213> Artificial
<220>
   <223> heavy chain variable domain, hMab 2F11-g1
<400> 55
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Phe Ser Leu Thr Thr Tyr
               20                  25                  30
   Asp Ile Ser Trp Ile Arg Gln Pro Pro Gly Lys Gly Leu Glu Trp Ile
           35                  40                  45
   Gly Val Ile Trp Thr Asp Gly Gly Thr Asn Tyr Asn Ser Pro Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Asp Gln Arg Leu Tyr Phe Asp Val Trp Gly Gln Gly Thr Thr Val
                   100                 105                 110
   Thr Val Ser Ser
           115
<210> 56
   <211> 106
   <212> PRT
   <213> Artificial
<220>
   <223> light chain variable domain, hMab 2F11-g1
<400> 56
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Glu Asp Val Asn Thr Tyr
               20                  25                  30
   Leu Ala Trp Tyr Gln Gln Lys Pro Gly Gln Ala Pro Arg Leu Leu Ile
           35                  40                  45
   Tyr Gly Ala Ser Ser Arg Ala Thr Gly Ile Pro Asp Arg Phe Ser Gly
       50                  55                  60
   Ser Gly Ser Gly Thr Asp Phe Thr Leu Thr Ile Ser Arg Leu Glu Pro
   65                  70                  75                  80
   Glu Asp Phe Ala Val Tyr Tyr Cys Gly Gln Ser Phe Ser Tyr Pro Thr
                   85                  90                  95
   Phe Gly Gln Gly Thr Lys Leu Glu Ile Lys
               100                 105
<210> 57
   <211> 107
   <212> PRT
   <213> Homo sapiens
<400> 57
<210> 58
   <211> 330
   <212> PRT
   <213> Homo sapiens
<400> 58
<210> 59
   <211> 330
   <212> PRT
   <213> Artificial
<220>
   <223> human heavy chain constant region derived from IgG1 mutated on L234A and L235A
<400> 59
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 60
   <211> 327
   <212> PRT
   <213> Homo sapiens
<400> 60
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 61
   <211> 327
   <212> PRT
   <213> Artificial
<220>
   <223> human heavy chain constant region derived from IgG4 mutated onS228P
<400> 61
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
               290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 62
   <211> 972
   <212> PRT
   <213> Homo sapiens
<400> 62
<210> 63
   <211> 972
   <212> PRT
   <213> Artificial
<220>
   <223> mutant CSF-1R L301S Y969F
<400> 63
   Met Gly Pro Gly Val Leu Leu Leu Leu Leu Val Ala Thr Ala Trp His
   1 5 10 15
   Gly Gln Gly Ile Pro Val Ile Glu Pro Ser Val Pro Glu Leu Val Val
   20 25 30
   Lys Pro Gly Ala Thr Val Thr Leu Arg Cys Val Gly Asn Gly Ser Val
   35 40 45
   Glu Trp Asp Gly Pro Pro Ser Pro His Trp Thr Leu Tyr Ser Asp Gly
   50 55 60
   Ser Ser Ser Ile Leu Ser Thr Asn Asn Ala Thr Phe Gln Asn Thr Gly
   65 70 75 80
   Thr Tyr Arg Cys Thr Glu Pro Gly Asp Pro Leu Gly Gly Ser Ala Ala
   85 90 95
   Ile His Leu Tyr Val Lys Asp Pro Ala Arg Pro Trp Asn Val Leu Ala
   100 105 110
   Gln Glu Val Val Val Phe Glu Asp Gln Asp Ala Leu Leu Pro Cys Leu
   115 120 125
   Leu Thr Asp Pro Val Leu Glu Ala Gly Val Ser Leu Val Arg Val Arg
   130 135 140
   Gly Arg Pro Leu Met Arg His Thr Asn Tyr Ser Phe Ser Pro Trp His
   145 150 155 160
   Gly Phe Thr Ile His Arg Ala Lys Phe Ile Gln Ser Gln Asp Tyr Gln
   165 170 175
   Cys Ser Ala Leu Met Gly Gly Arg Lys Val Met Ser Ile Ser Ile Arg
   180 185 190
   Leu Lys Val Gln Lys Val Ile Pro Gly Pro Pro Ala Leu Thr Leu Val
   195 200 205
   Pro Ala Glu Leu Val Arg Ile Arg Gly Glu Ala Ala Gln Ile Val Cys
   210 215 220
   Ser Ala Ser Ser Val Asp Val Asn Phe Asp Val Phe Leu Gln His Asn
   225 230 235 240
   Asn Thr Lys Leu Ala Ile Pro Gln Gln Ser Asp Phe His Asn Asn Arg
   245 250 255
   Tyr Gln Lys Val Leu Thr Leu Asn Leu Asp Gln Val Asp Phe Gln His
   260 265 270
   Ala Gly Asn Tyr Ser Cys Val Ala Ser Asn Val Gln Gly Lys His Ser
   275 280 285
   Thr Ser Met Phe Phe Arg Val Val Glu Ser Ala Tyr Ser Asn Leu Ser
   290 295 300
   Ser Glu Gln Asn Leu Ile Gln Glu Val Thr Val Gly Glu Gly Leu Asn
   305 310 315 320
   Leu Lys Val Met Val Glu Ala Tyr Pro Gly Leu Gln Gly Phe Asn Trp
   325 330 335
   Thr Tyr Leu Gly Pro Phe Ser Asp His Gln Pro Glu Pro Lys Leu Ala
   340 345 350
   Asn Ala Thr Thr Lys Asp Thr Tyr Arg His Thr Phe Thr Leu Ser Leu
   355 360 365
   Pro Arg Leu Lys Pro Ser Glu Ala Gly Arg Tyr Ser Phe Leu Ala Arg
   370 375 380
   Asn Pro Gly Gly Trp Arg Ala Leu Thr Phe Glu Leu Thr Leu Arg Tyr
   385 390 395 400
   Pro Pro Glu Val Ser Val Ile Trp Thr Phe Ile Asn Gly Ser Gly Thr
   405 410 415
   Leu Leu Cys Ala Ala Ser Gly Tyr Pro Gln Pro Asn Val Thr Trp Leu
   420 425 430
   Gln Cys Ser Gly His Thr Asp Arg Cys Asp Glu Ala Gln Val Leu Gln
   435 440 445
   Val Trp Asp Asp Pro Tyr Pro Glu Val Leu Ser Gln Glu Pro Phe His
   450 455 460
   Lys Val Thr Val Gln Ser Leu Leu Thr Val Glu Thr Leu Glu His Asn
   465 470 475 480
   Gln Thr Tyr Glu Cys Arg Ala His Asn Ser Val Gly Ser Gly Ser Trp
   485 490 495
   Ala Phe Ile Pro Ile Ser Ala Gly Ala His Thr His Pro Pro Asp Glu
   500 505 510
   Phe Leu Phe Thr Pro Val Val Val Ala Cys Met Ser Ile Met Ala Leu
   515 520 525
   Leu Leu Leu Leu Leu Leu Leu Leu Leu Tyr Lys Tyr Lys Gln Lys Pro
   530 535 540
   Lys Tyr Gln Val Arg Trp Lys Ile Ile Glu Ser Tyr Glu Gly Asn Ser
   545 550 555 560
   Tyr Thr Phe Ile Asp Pro Thr Gln Leu Pro Tyr Asn Glu Lys Trp Glu
   565 570 575
   Phe Pro Arg Asn Asn Leu Gln Phe Gly Lys Thr Leu Gly Ala Gly Ala
   580 585 590
   Phe Gly Lys Val Val Glu Ala Thr Ala Phe Gly Leu Gly Lys Glu Asp
   595 600 605
   Ala Val Leu Lys Val Ala Val Lys Met Leu Lys Ser Thr Ala His Ala
   610 615 620
   Asp Glu Lys Glu Ala Leu Met Ser Glu Leu Lys Ile Met Ser His Leu
   625 630 635 640
   Gly Gln His Glu Asn Ile Val Asn Leu Leu Gly Ala Cys Thr His Gly
   645 650 655
   Gly Pro Val Leu Val Ile Thr Glu Tyr Cys Cys Tyr Gly Asp Leu Leu
   660 665 670
   Asn Phe Leu Arg Arg Lys Ala Glu Ala Met Leu Gly Pro Ser Leu Ser
           675                 680                 685
   Pro Gly Gln Asp Pro Glu Gly Gly Val Asp Tyr Lys Asn Ile His Leu
       690                 695                 700
   Glu Lys Lys Tyr Val Arg Arg Asp Ser Gly Phe Ser Ser Gln Gly Val
   705                 710                 715                 720
   Asp Thr Tyr Val Glu Met Arg Pro Val Ser Thr Ser Ser Asn Asp Ser
                   725                 730                 735
   Phe Ser Glu Gln Asp Leu Asp Lys Glu Asp Gly Arg Pro Leu Glu Leu
               740                 745                 750
   Arg Asp Leu Leu His Phe Ser Ser Gln Val Ala Gln Gly Met Ala Phe
           755                 760                 765
   Leu Ala Ser Lys Asn Cys Ile His Arg Asp Val Ala Ala Arg Asn Val
       770                 775                 780
   Leu Leu Thr Asn Gly His Val Ala Lys Ile Gly Asp Phe Gly Leu Ala
   785                 790                 795                 800
   Arg Asp Ile Met Asn Asp Ser Asn Tyr Ile Val Lys Gly Asn Ala Arg
                   805                 810                 815
   Leu Pro Val Lys Trp Met Ala Pro Glu Ser Ile Phe Asp Cys Val Tyr
               820                 825                 830
   Thr Val Gln Ser Asp Val Trp Ser Tyr Gly Ile Leu Leu Trp Glu Ile
           835                 840                 845
   Phe Ser Leu Gly Leu Asn Pro Tyr Pro Gly Ile Leu Val Asn Ser Lys
       850                 855                 860
   Phe Tyr Lys Leu Val Lys Asp Gly Tyr Gln Met Ala Gln Pro Ala Phe
   865                 870                 875                 880
   Ala Pro Lys Asn Ile Tyr Ser Ile Met Gln Ala Cys Trp Ala Leu Glu
                   885                 890                 895
   Pro Thr His Arg Pro Thr Phe Gln Gln Ile Cys Ser Phe Leu Gln Glu
               900                 905                 910
   Gln Ala Gln Glu Asp Arg Arg Glu Arg Asp Tyr Thr Asn Leu Pro Ser
           915                 920                 925
   Ser Ser Arg Ser Gly Gly Ser Gly Ser Ser Ser Ser Glu Leu Glu Glu
       930                 935                 940
   Glu Ser Ser Ser Glu His Leu Thr Cys Cys Glu Gln Gly Asp Ile Ala
   945                 950                 955                 960
   Gln Pro Leu Leu Gln Pro Asn Asn Phe Gln Phe Cys
                   965                 970
<210> 64
   <211> 493
   <212> PRT
   <213> Artificial
<220>
   <223> human CSF-1R Extracellular Domain
<400> 64
   Ile Pro Val Ile Glu Pro Ser Val Pro Glu Leu Val Val Lys Pro Gly
   1               5                   10                  15
   Ala Thr Val Thr Leu Arg Cys Val Gly Asn Gly Ser Val Glu Trp Asp
               20                  25                  30
   Gly Pro Pro Ser Pro His Trp Thr Leu Tyr Ser Asp Gly Ser Ser Ser
           35                  40                  45
   Ile Leu Ser Thr Asn Asn Ala Thr Phe Gln Asn Thr Gly Thr Tyr Arg
       50                  55                  60
   Cys Thr Glu Pro Gly Asp Pro Leu Gly Gly Ser Ala Ala Ile His Leu
   65                  70                  75                  80
   Tyr Val Lys Asp Pro Ala Arg Pro Trp Asn Val Leu Ala Gln Glu Val
                   85                  90                  95
   Val Val Phe Glu Asp Gln Asp Ala Leu Leu Pro Cys Leu Leu Thr Asp
               100                 105                 110
   Pro Val Leu Glu Ala Gly Val Ser Leu Val Arg Val Arg Gly Arg Pro
           115                 120                 125
   Leu Met Arg His Thr Asn Tyr Ser Phe Ser Pro Trp His Gly Phe Thr
       130                 135                 140
   Ile His Arg Ala Lys Phe Ile Gln Ser Gln Asp Tyr Gln Cys Ser Ala
   145                 150                 155                 160
   Leu Met Gly Gly Arg Lys Val Met Ser Ile Ser Ile Arg Leu Lys Val
   165 170 175
   Gln Lys Val Ile Pro Gly Pro Pro Ala Leu Thr Leu Val Pro Ala Glu
   180 185 190
   Leu Val Arg Ile Arg Gly Glu Ala Ala Gln Ile Val Cys Ser Ala Ser
   195 200 205
   Ser Val Asp Val Asn Phe Asp Val Phe Leu Gln His Asn Asn Thr Lys
   210 215 220
   Leu Ala Ile Pro Gln Gln Ser Asp Phe His Asn Asn Arg Tyr Gln Lys
   225 230 235 240
   Val Leu Thr Leu Asn Leu Asp Gln Val Asp Phe Gln His Ala Gly Asn
   245 250 255
   Tyr Ser Cys Val Ala Ser Asn Val Gln Gly Lys His Ser Thr Ser Met
   260 265 270
   Phe Phe Arg Val Val Glu Ser Ala Tyr Leu Asn Leu Ser Ser Glu Gln
   275 280 285
   Asn Leu Ile Gln Glu Val Thr Val Gly Glu Gly Leu Asn Leu Lys Val
   290 295 300
   Met Val Glu Ala Tyr Pro Gly Leu Gln Gly Phe Asn Trp Thr Tyr Leu
   305 310 315 320
   Gly Pro Phe Ser Asp His Gln Pro Glu Pro Lys Leu Ala Asn Ala Thr
   325 330 335
   Thr Lys Asp Thr Tyr Arg His Thr Phe Thr Leu Ser Leu Pro Arg Leu
   340 345 350
   Lys Pro Ser Glu Ala Gly Arg Tyr Ser Phe Leu Ala Arg Asn Pro Gly
   355 360 365
   Gly Trp Arg Ala Leu Thr Phe Glu Leu Thr Leu Arg Tyr Pro Pro Glu
   370 375 380
   Val Ser Val Ile Trp Thr Phe Ile Asn Gly Ser Gly Thr Leu Leu Cys
   385 390 395 400
   Ala Ala Ser Gly Tyr Pro Gln Pro Asn Val Thr Trp Leu Gln Cys Ser
   405 410 415
   Gly His Thr Asp Arg Cys Asp Glu Ala Gln Val Leu Gln Val Trp Asp
   420 425 430
   Asp Pro Tyr Pro Glu Val Leu Ser Gln Glu Pro Phe His Lys Val Thr
   435 440 445
   Val Gln Ser Leu Leu Thr Val Glu Thr Leu Glu His Asn Gln Thr Tyr
   450 455 460
   Glu Cys Arg Ala His Asn Ser Val Gly Ser Gly Ser Trp Ala Phe Ile
   465 470 475 480
   Pro Ile Ser Ala Gly Ala His Thr His Pro Pro Asp Glu
   485 490
<210> 65
   <211> 388
   <212> PRT
   <213> Artificial
<220>
   <223> human CSF-1R fragment delD4
<400> 65
   Ile Pro Val Ile Glu Pro Ser Val Pro Glu Leu Val Val Lys Pro Gly
   1               5                   10                  15
   Ala Thr Val Thr Leu Arg Cys Val Gly Asn Gly Ser Val Glu Trp Asp
               20                  25                  30
   Gly Pro Pro Ser Pro His Trp Thr Leu Tyr Ser Asp Gly Ser Ser Ser
           35                  40                  45
   Ile Leu Ser Thr Asn Asn Ala Thr Phe Gln Asn Thr Gly Thr Tyr Arg
       50                  55                  60
   Cys Thr Glu Pro Gly Asp Pro Leu Gly Gly Ser Ala Ala Ile His Leu
   65                  70                  75                  80
   Tyr Val Lys Asp Pro Ala Arg Pro Trp Asn Val Leu Ala Gln Glu Val
                   85                  90                  95
   Val Val Phe Glu Asp Gln Asp Ala Leu Leu Pro Cys Leu Leu Thr Asp
               100                 105                 110
   Pro Val Leu Glu Ala Gly Val Ser Leu Val Arg Val Arg Gly Arg Pro
           115                 120                 125
   Leu Met Arg His Thr Asn Tyr Ser Phe Ser Pro Trp His Gly Phe Thr
   130 135 140
   Ile His Arg Ala Lys Phe Ile Gln Ser Gln Asp Tyr Gln Cys Ser Ala
   145 150 155 160
   Leu Met Gly Gly Arg Lys Val Met Ser Ile Ser Ile Arg Leu Lys Val
   165 170 175
   Gln Lys Val Ile Pro Gly Pro Pro Ala Leu Thr Leu Val Pro Ala Glu
   180 185 190
   Leu Val Arg Ile Arg Gly Glu Ala Ala Gln Ile Val Cys Ser Ala Ser
   195 200 205
   Ser Val Asp Val Asn Phe Asp Val Phe Leu Gln His Asn Asn Thr Lys
   210 215 220
   Leu Ala Ile Pro Gln Gln Ser Asp Phe His Asn Asn Arg Tyr Gln Lys
   225 230 235 240
   Val Leu Thr Leu Asn Leu Asp Gln Val Asp Phe Gln His Ala Gly Asn
   245 250 255
   Tyr Ser Cys Val Ala Ser Asn Val Gln Gly Lys His Ser Thr Ser Met
   260 265 270
   Phe Phe Arg Tyr Pro Pro Glu Val Ser Val Ile Trp Thr Phe Ile Asn
   275 280 285
   Gly Ser Gly Thr Leu Leu Cys Ala Ala Ser Gly Tyr Pro Gln Pro Asn
   290 295 300
   Val Thr Trp Leu Gln Cys Ser Gly His Thr Asp Arg Cys Asp Glu Ala
   305 310 315 320
   Gln Val Leu Gln Val Trp Asp Asp Pro Tyr Pro Glu Val Leu Ser Gln
   325 330 335
   Glu Pro Phe His Lys Val Thr Val Gln Ser Leu Leu Thr Val Glu Thr
   340 345 350
   Leu Glu His Asn Gln Thr Tyr Glu Cys Arg Ala His Asn Ser Val Gly
   355 360 365
   Ser Gly Ser Trp Ala Phe Ile Pro Ile Ser Ala Gly Ala His Thr His
   370 375 380
   Pro Pro Asp Glu
   385
<210> 66
   <211> 292
   <212> PRT
   <213> Artificial
<220>
   <223> human CSF-1R fragment D1-D3
<400> 66
   Ile Pro Val Ile Glu Pro Ser Val Pro Glu Leu Val Val Lys Pro Gly
   1               5                   10                  15
   Ala Thr Val Thr Leu Arg Cys Val Gly Asn Gly Ser Val Glu Trp Asp
               20                  25                  30
   Gly Pro Pro Ser Pro His Trp Thr Leu Tyr Ser Asp Gly Ser Ser Ser
           35                  40                  45
   Ile Leu Ser Thr Asn Asn Ala Thr Phe Gln Asn Thr Gly Thr Tyr Arg
       50                  55                  60
   Cys Thr Glu Pro Gly Asp Pro Leu Gly Gly Ser Ala Ala Ile His Leu
   65                  70                  75                  80
   Tyr Val Lys Asp Pro Ala Arg Pro Trp Asn Val Leu Ala Gln Glu Val
                   85                  90                  95
   Val Val Phe Glu Asp Gln Asp Ala Leu Leu Pro Cys Leu Leu Thr Asp
               100                 105                 110
   Pro Val Leu Glu Ala Gly Val Ser Leu Val Arg Val Arg Gly Arg Pro
           115                 120                 125
   Leu Met Arg His Thr Asn Tyr Ser Phe Ser Pro Trp His Gly Phe Thr
       130                 135                 140
   Ile His Arg Ala Lys Phe Ile Gln Ser Gln Asp Tyr Gln Cys Ser Ala
   145                 150                 155                 160
   Leu Met Gly Gly Arg Lys Val Met Ser Ile Ser Ile Arg Leu Lys Val
                   165                 170                 175
   Gln Lys Val Ile Pro Gly Pro Pro Ala Leu Thr Leu Val Pro Ala Glu
               180                 185                 190
   Leu Val Arg Ile Arg Gly Glu Ala Ala Gln Ile Val Cys Ser Ala Ser
   195 200 205
   Ser Val Asp Val Asn Phe Asp Val Phe Leu Gln His Asn Asn Thr Lys
   210 215 220
   Leu Ala Ile Pro Gln Gln Ser Asp Phe His Asn Asn Arg Tyr Gln Lys
   225 230 235 240
   Val Leu Thr Leu Asn Leu Asp Gln Val Asp Phe Gln His Ala Gly Asn
   245 250 255
   Tyr Ser Cys Val Ala Ser Asn Val Gln Gly Lys His Ser Thr Ser Met
   260 265 270
   Phe Phe Arg Val Val Glu Ser Ala Tyr Leu Asn Leu Ser Ser Glu Gln
   275 280 285
   Asn Leu Ile Gln
   290
<210> 67
   <211> 21
   <212> PRT
   <213> Artificial
<220>
   <223> signal peptide
<400> 67
   Met Gly Ser Gly Pro Gly Val Leu Leu Leu Leu Leu Val Ala Thr Ala
   1 5 10 15
   Trp His Gly Gln Gly
   20
<210> 68
   <211> 36
   <212> DNA
   <213> Artificial
<220>
   <223> Primer
<400> 68
   cacctccatg ttcttccggt accccccaga ggtaag 36
<210> 69
   <211> 8
   <212> PRT
   <213> Mus musculus
<400> 69
   Asp Leu Arg Leu Tyr Phe Asp Val
   1 5
<210> 70
   <211> 16
   <212> PRT
   <213> Mus musculus
<400> 70
   Val Ile Trp Ser Gly Gly Gly Thr Asn Tyr Asn Ser Pro Phe Met Ser
   1 5 10 15
<210> 71
   <211> 10
   <212> PRT
   <213> Mus musculus
<400> 71
   Gly Phe Ser Leu Thr Ser Tyr Asp Ile Ser
   1 5 10
<210> 72
   <211> 8
   <212> PRT
   <213> Mus musculus
<400> 72
   Gly Gln Ser Phe Thr Tyr Pro Thr
   1 5
<210> 73
   <211> 7
   <212> PRT
   <213> Mus musculus
<400> 73
   Gly Ser Ser Asn Arg Tyr Thr
   1 5
<210> 74
   <211> 11
   <212> PRT
   <213> Mus musculus
<400> 74
   Lys Ala Ser Glu Asp Val Gly Thr Tyr Val Ser
   1 5 10
<210> 75
   <211> 116
   <212> PRT
   <213> Mus musculus
<400> 75
   Arg Val Gln Leu Lys Glu Ser Gly Pro Gly Leu Val Ala Pro Ser Gln
   1               5                   10                  15
   Ser Leu Ser Ile Thr Cys Thr Val Ser Gly Phe Ser Leu Thr Ser Tyr
               20                  25                  30
   Asp Ile Ser Trp Ile Arg Gln Ser Pro Gly Lys Gly Leu Glu Trp Leu
           35                  40                  45
   Gly Val Ile Trp Ser Gly Gly Gly Thr Asn Tyr Asn Ser Pro Phe Met
       50                  55                  60
   Ser Arg Leu Arg Ile Ser Lys Asp Asp Ser Arg Ser Gln Val Phe Leu
   65                  70                  75                  80
   Lys Val Asn Arg Leu Gln Thr Asp Asp Thr Ala Ile Tyr Tyr Cys Val
                   85                  90                  95
   Arg Asp Leu Arg Leu Tyr Phe Asp Val Trp Gly Ala Gly Thr Thr Val
               100                 105                 110
   Thr Val Ser Ser
           115
<210> 76
   <211> 106
   <212> PRT
   <213> Mus musculus
<400> 76
   Lys Ile Val Met Thr Gln Ser Pro Lys Ser Met Ser Val Ser Val Gly
   1               5                   10                  15
   Glu Arg Val Ser Leu Ser Cys Lys Ala Ser Glu Asp Val Gly Thr Tyr
               20                  25                  30
   Val Ser Trp Tyr Gln Gln Lys Pro Glu Gln Ser Pro Lys Leu Leu Ile
           35                  40                  45
   Tyr Gly Ser Ser Asn Arg Tyr Thr Gly Val Pro Asp Arg Phe Thr Gly
       50                  55                  60
   Ser Gly Ser Ala Thr Asp Phe Thr Leu Thr Ile Ser Ser Val Gln Ala
   65                  70                  75                  80
   Glu Asp Leu Ala Asp Tyr Ser Cys Gly Gln Ser Phe Thr Tyr Pro Thr
   85 90 95
   Phe Gly Thr Gly Thr Lys Leu Glu Ile Lys
   100 105
<210> 77
   <211> 8
   <212> PRT
   <213> Mus musculus
<400> 77
   Asp Pro Arg Leu Tyr Phe Asp Val
   1 5
<210> 78
   <211> 16
   <212> PRT
   <213> Mus musculus
<400> 78
   Val Ile Trp Thr Gly Gly Gly Thr Asn Tyr Asn Ser Gly Phe Met Ser
   1 5 10 15
<210> 79
   <211> 10
   <212> PRT
   <213> Mus musculus
<400> 79
   Gly Ser Ser Leu Asp Ser Phe Asp Ile Ser
   1 5 10
<210> 80
   <211> 8
   <212> PRT
   <213> Mus musculus
<400> 80
   Gly Gln Thr Phe Ser Tyr Pro Thr
   1 5
<210> 81
   <211> 7
   <212> PRT
   <213> Mus musculus
<400> 81
   Gly Ala Ser Asn Arg Tyr Thr
   1 5
<210> 82
   <211> 11
   <212> PRT
   <213> Mus musculus
<400> 82
   Lys Ala Ser Glu Asp Val Val Thr Tyr Val Ser
   1               5                   10
<210> 83
   <211> 116
   <212> PRT
   <213> Mus musculus
<400> 83
   Gln Val Gln Leu Lys Glu Ser Gly Pro Gly Leu Val Ala Pro Ser Lys
   1               5                   10                  15
   Ser Leu Ser Ile Thr Cys Thr Val Ser Gly Ser Ser Leu Asp Ser Phe
               20                  25                  30
   Asp Ile Ser Trp Ile Arg Gln Pro Pro Gly Lys Gly Leu Glu Trp Leu
           35                  40                  45
   Gly Val Ile Trp Thr Gly Gly Gly Thr Asn Tyr Asn Ser Gly Phe Met
       50                  55                  60
   Ser Arg Leu Arg Ile Ser Lys Asp Asn Ser Lys Ser Gln Val Phe Leu
   65                  70                  75                  80
   Lys Met Ser Ser Leu Gln Ser Asp Asp Thr Ala Ile Tyr Tyr Cys Val
                   85                  90                  95
   Arg Asp Pro Arg Leu Tyr Phe Asp Val Trp Gly Ala Gly Thr Thr Val
               100                 105                 110
   Thr Val Ser Ser
           115
<210> 84
   <211> 106
   <212> PRT
   <213> Mus musculus
<400> 84
   Asn Ile Val Met Thr Gln Ser Pro Lys Ser Met Ser Met Ser Val Gly
   1               5                   10                  15
   Glu Arg Val Thr Leu Ser Cys Lys Ala Ser Glu Asp Val Val Thr Tyr
               20                  25                  30
   Val Ser Trp Tyr Gln Gln Lys Pro Glu Gln Ser Pro Lys Leu Leu Ile
           35                  40                  45
   Tyr Gly Ala Ser Asn Arg Tyr Thr Gly Val Pro Asp Arg Phe Thr Gly
       50                  55                  60
   Ser Gly Ser Ala Thr Asp Phe Thr Leu Thr Ile Ser Ser Ile Gln Ala
   65                  70                  75                  80
   Glu Asp Leu Ala Asp Tyr Tyr Cys Gly Gln Thr Phe Ser Tyr Pro Thr
                   85                  90                  95
   Phe Gly Thr Gly Thr Lys Leu Glu Ile Lys
               100                 105
<210> 85
   <211> 218
   <212> PRT
   <213> Artificial
<220>
   <223> human CSF-1R fragment domains D4-D5
<400> 85
   Val Val Glu Ser Ala Tyr Leu Asn Leu Ser Ser Glu Gln Asn Leu Ile
   1               5                   10                  15
   Gln Glu Val Thr Val Gly Glu Gly Leu Asn Leu Lys Val Met Val Glu
               20                  25                  30
   Ala Tyr Pro Gly Leu Gln Gly Phe Asn Trp Thr Tyr Leu Gly Pro Phe
           35                  40                  45
   Ser Asp His Gln Pro Glu Pro Lys Leu Ala Asn Ala Thr Thr Lys Asp
       50                  55                  60
   Thr Tyr Arg His Thr Phe Thr Leu Ser Leu Pro Arg Leu Lys Pro Ser
   65                  70                  75                  80
   Glu Ala Gly Arg Tyr Ser Phe Leu Ala Arg Asn Pro Gly Gly Trp Arg
                   85                  90                  95
   Ala Leu Thr Phe Glu Leu Thr Leu Arg Tyr Pro Pro Glu Val Ser Val
               100                 105                 110
   Ile Trp Thr Phe Ile Asn Gly Ser Gly Thr Leu Leu Cys Ala Ala Ser
           115                 120                 125
   Gly Tyr Pro Gln Pro Asn Val Thr Trp Leu Gln Cys Ser Gly His Thr
       130                 135                 140
   Asp Arg Cys Asp Glu Ala Gln Val Leu Gln Val Trp Asp Asp Pro Tyr
   145                 150                 155                 160
   Pro Glu Val Leu Ser Gln Glu Pro Phe His Lys Val Thr Val Gln Ser
                   165                 170                 175
   Leu Leu Thr Val Glu Thr Leu Glu His Asn Gln Thr Tyr Glu Cys Arg
               180                 185                 190
   Ala His Asn Ser Val Gly Ser Gly Ser Trp Ala Phe Ile Pro Ile Ser
           195                 200                 205
   Ala Gly Ala His Thr His Pro Pro Asp Glu
       210                 215
<210> 86
   <211> 554
   <212> PRT
   <213> homo sapiens
<400> 86
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Asp Val Val Thr Lys Pro Asp Cys Asn Cys Leu
               180                 185                 190
   Tyr Pro Lys Ala Ile Pro Ser Ser Asp Pro Ala Ser Val Ser Pro His
           195                 200                 205
   Gln Pro Leu Ala Pro Ser Met Ala Pro Val Ala Gly Leu Thr Trp Glu
       210                 215                 220
   Asp Ser Glu Gly Thr Glu Gly Ser Ser Leu Leu Pro Gly Glu Gln Pro
   225                 230                 235                 240
   Leu His Thr Val Asp Pro Gly Ser Ala Lys Gln Arg Pro Pro Arg Ser
                   245                 250                 255
   Thr Cys Gln Ser Phe Glu Pro Pro Glu Thr Pro Val Val Lys Asp Ser
               260                 265                 270
   Thr Ile Gly Gly Ser Pro Gln Pro Arg Pro Ser Val Gly Ala Phe Asn
           275                 280                 285
   Pro Gly Met Glu Asp Ile Leu Asp Ser Ala Met Gly Thr Asn Trp Val
       290                 295                 300
   Pro Glu Glu Ala Ser Gly Glu Ala Ser Glu Ile Pro Val Pro Gln Gly
   305                 310                 315                 320
   Thr Glu Leu Ser Pro Ser Arg Pro Gly Gly Gly Ser Met Gln Thr Glu
                   325                 330                 335
   Pro Ala Arg Pro Ser Asn Phe Leu Ser Ala Ser Ser Pro Leu Pro Ala
               340                 345                 350
   Ser Ala Lys Gly Gln Gln Pro Ala Asp Val Thr Gly Thr Ala Leu Pro
           355                 360                 365
   Arg Val Gly Pro Val Arg Pro Thr Gly Gln Asp Trp Asn His Thr Pro
       370                 375                 380
   Gln Lys Thr Asp His Pro Ser Ala Leu Leu Arg Asp Pro Pro Glu Pro
   385 390 395 400
   Gly Ser Pro Arg Ile Ser Ser Leu Arg Pro Gln Gly Leu Ser Asn Pro
   405 410 415
   Ser Thr Leu Ser Ala Gln Pro Gln Leu Ser Arg Ser His Ser Ser Gly
   420 425 430
   Ser Val Leu Pro Leu Gly Glu Leu Glu Gly Arg Arg Ser Thr Arg Asp
   435 440 445
   Arg Arg Ser Pro Ala Glu Pro Glu Gly Gly Pro Ala Ser Glu Gly Ala
   450 455 460
   Ala Arg Pro Leu Pro Arg Phe Asn Ser Val Pro Leu Thr Asp Thr Gly
   465 470 475 480
   His Glu Arg Gln Ser Glu Gly Ser Phe Ser Pro Gln Leu Gln Glu Ser
   485 490 495
   Val Phe His Leu Leu Val Pro Ser Val Ile Leu Val Leu Leu Ala Val
   500 505 510
   Gly Gly Leu Leu Phe Tyr Arg Trp Arg Arg Arg Ser His Gln Glu Pro
   515 520 525
   Gln Arg Ala Asp Ser Pro Leu Glu Gln Pro Glu Gly Ser Pro Leu Thr
   530 535 540
   Gln Asp Asp Arg Gln Val Glu Leu Pro Val
   545 550
<210> 87
   <211> 242
   <212> PRT
   <213> homo sapiens
<400> 87
   Met Pro Arg Gly Phe Thr Trp Leu Arg Tyr Leu Gly Ile Phe Leu Gly
   1 5 10 15
   Val Ala Leu Gly Asn Glu Pro Leu Glu Met Trp Pro Leu Thr Gln Asn
   20 25 30
   Glu Glu Cys Thr Val Thr Gly Phe Leu Arg Asp Lys Leu Gln Tyr Arg
   35 40 45
   Ser Arg Leu Gln Tyr Met Lys His Tyr Phe Pro Ile Asn Tyr Lys Ile
       50                  55                  60
   Ser Val Pro Tyr Glu Gly Val Phe Arg Ile Ala Asn Val Thr Arg Leu
   65                  70                  75                  80
   Gln Arg Ala Gln Val Ser Glu Arg Glu Leu Arg Tyr Leu Trp Val Leu
                   85                  90                  95
   Val Ser Leu Ser Ala Thr Glu Ser Val Gln Asp Val Leu Leu Glu Gly
               100                 105                 110
   His Pro Ser Trp Lys Tyr Leu Gln Glu Val Glu Thr Leu Leu Leu Asn
           115                 120                 125
   Val Gln Gln Gly Leu Thr Asp Val Glu Val Ser Pro Lys Val Glu Ser
       130                 135                 140
   Val Leu Ser Leu Leu Asn Ala Pro Gly Pro Asn Leu Lys Leu Val Arg
   145                 150                 155                 160
   Pro Lys Ala Leu Leu Asp Asn Cys Phe Arg Val Met Glu Leu Leu Tyr
                   165                 170                 175
   Cys Ser Cys Cys Lys Gln Ser Ser Val Leu Asn Trp Gln Asp Cys Glu
               180                 185                 190
   Val Pro Ser Pro Gln Ser Cys Ser Pro Glu Pro Ser Leu Gln Tyr Ala
           195                 200                 205
   Ala Thr Gln Leu Tyr Pro Pro Pro Pro Trp Ser Pro Ser Ser Pro Pro
       210                 215                 220
   His Ser Thr Gly Ser Val Arg Pro Val Arg Ala Gln Gly Glu Gly Leu
   225                 230                 235                 240
   Leu Pro
<210> 88
   <211> 126
   <212> PRT
   <213> Homo sapiens
<400> 88
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asp Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Asn Arg Leu Arg Ser Asp Asp Thr Ala Val Tyr Tyr Cys
   85 90 95
   Ala Arg Asp Gln Pro Leu Gly Tyr Cys Thr Asn Gly Val Cys Ser Tyr
   100 105 110
   Phe Asp Tyr Trp Gly Gln Gly Thr Leu Val Thr Val Ser Ser
   115 120 125
<210> 89
   <211> 107
   <212> PRT
   <213> Homo sapiens
<400> 89
   Asp Ile Gln Met Thr Gln Ser Pro Ser Ser Val Ser Ala Ser Val Gly
   1 5 10 15
   Asp Arg Val Thr Ile Thr Cys Arg Ala Ser Gln Gly Ile Tyr Ser Trp
   20 25 30
   Leu Ala Trp Tyr Gln Gln Lys Pro Gly Lys Ala Pro Asn Leu Leu Ile
   35 40 45
   Tyr Thr Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ala Asn Ile Phe Pro Leu
   85 90 95
   Thr Phe Gly Gly Gly Thr Lys Val Glu Ile Lys
   100 105
<210> 90
   <211> 113
   <212> PRT
   <213> Artificial
<220>
   <223> humanized S2C6 heavy chain variabel domain variant
<400> 90
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Tyr Ile His Trp Val Arg Gln Ala Pro Gly Lys Gly Leu Glu Trp Val
           35                  40                  45
   Ala Arg Val Ile Pro Asn Ala Gly Gly Thr Ser Tyr Asn Gln Lys Phe
       50                  55                  60
   Lys Gly Arg Phe Thr Leu Ser Val Asp Asn Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Ile Tyr Trp Trp Gly Gln Gly Thr Leu Val Thr Val
               100                 105                 110
   Ser
<210> 91
   <211> 113
   <212> PRT
   <213> Artificial
<220>
   <223> humanized S2C6 light chain variabel domain variant
<400> 91
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Ser Leu Val His Ser
               20                  25                  30
   Asn Gly Asn Thr Phe Leu His Trp Tyr Gln Gln Lys Pro Gly Lys Ala
                       35                  40                  45
   Pro Lys Leu Leu Ile Tyr Thr Val Ser Asn Arg Phe Ser Gly Val Pro
       50                  55                  60
   Ser Arg Phe Ser Gly Ser Gly Ser Gly Thr Asp Phe Thr Leu Thr Ile
   65                  70                  75                  80
   Ser Ser Leu Gln Pro Glu Asp Phe Ala Thr Tyr Phe Cys Ser Gln Thr
                   85                  90                  95
   Thr His Val Pro Trp Thr Phe Gly Gln Gly Thr Lys Val Glu Ile Lys
               100                 105                 110
   Arg